# DEMANDE DE BREVET EUROPEEN

(11) **EP 3 998 257 A1**
(43) Date de publication de la demande: **18.05.2022**
(21) Numéro de dépôt: 21213303.7
(22) Date de dépôt: 06.07.2015
(51) Int. Cl.: C07D 307/93, A61K 31/343, A61P 35/00

(54) **DÉRIVÉS DE FLAVAGLINES**

(30) Priorité: 04.07.2014 FR 1456474
(62) Demande divisionnaire de: 18187037.9
(71) Demandeur: PIERRE FABRE MEDICAMENT, 81500 Lavaur (FR)
(72) Inventeur: MARION, Frédéric, 31000 TOULOUSE (FR); KALOUN, El Bachir, 31120 ROQUETTES (FR); LIEBY-MULLER, Frédéric, 31120 PORTET-SUR-GARONNE (FR); PEREZ, Michel, 81100 CASTRES (FR); ANNEREAU, Jean-Philippe, 31400 TOULOUSE (FR); CREANCIER, Laurent, 31120 PORTET SUR GARONNE (FR)
(74) Mandataire: Regimbeau

(57) **Abrégé**

La présente invention concerne des composés de formule générale (I) suivante : 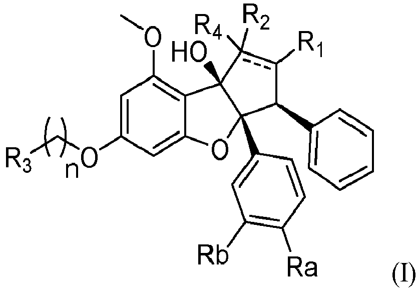 sous forme d'un de leurs énantiomères ou d'un mélange de leurs énantiomères,
et leurs sels et/ou solvates pharmaceutiquement acceptables de ceux-ci,
notamment pour leur utilisation comme médicament, plus particulièrement dans le traitement du cancer.

La présente invention concerne également des compositions pharmaceutiques les contenant et leurs procédés de préparation.

## Description

La présente invention a pour objet de nouveaux dérivés de flavaglines, leurs procédés de fabrication, les compositions pharmaceutiques les contenant et leur utilisation comme médicament, notamment dans le traitement du cancer.

Les flavaglines sont une famille de produits naturels comprenant un squelette unique de type cyclopenta[b]benzofurane et regroupant des membres tels que le silvestrol et le rocaglamide.

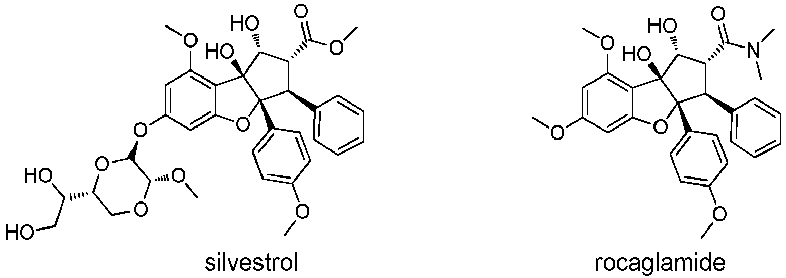

Cette famille possède de nombreuses propriétés biologiques dont notamment des activités anti-prolifératives (Hausott et al. Int. J. Cancer: 109, 933-940 (2004)). Cette capacité à inhiber la croissance de lignées de cellules cancéreuses a été reliée pour certains de ces membres comme le silvestrol à l'inhibiton de la synthèse protéique au travers de l'inactivation de l'hélicase eIF4A (Cencic et al. PloS ONE 2009, 4(4): e5223). Ces propriétés rendent la famille des flavaglines intéressante pour une application potentielle dans le traitement des maladies hyperprolifératives telles que le cancer par exemple. Malgré plusieurs efforts (Ribeiro et al. Bioorg. Med. Chem. 20 (2012) 1857-1864 ; Liu et al. J. Med. Chem. 2012, 55, 8859-8878), à ce jour aucun produit naturel ou dérivé de flavagline n'a démontré le potentiel suffisant pour conduire à une utilisation en tant que médicament.

La présente invention porte donc sur de nouveaux dérivés de flavagline possédant des activités anti-prolifératives intéressantes pour une application en tant que thérapie anti-cancéreuse notamment. Les inventeurs ont ainsi mis en évidence de façon inattendue que des modifications importantes sur le squelette cyclopenta[b]benzofurane ou des ensembles de modifications conduisent à des composés plus puissants que le silvestrol, possédant une activité anti-tumorale plus importante.

La présente invention a ainsi pour objet un composé de formule générale (I) suivante : 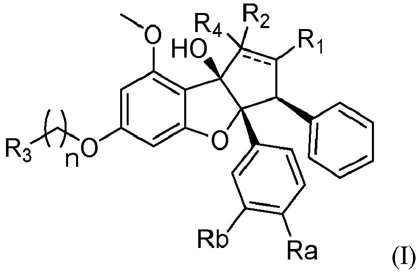 sous forme d'un de ses énantiomères ou d'un mélange de ses énantiomères tel qu'un mélange racémique, ou un sel et/ou solvate pharmaceutiquement acceptable de celui-ci, dans laquelle :
- 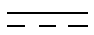 représente une liaison simple ou une liaison double,
- n représente un nombre entier compris entre 1 et 10,
- R₁ représente CO₂R₁₀, CONH₂, NR₁₁R₁₂, NR₁₃COR₁₄, NR₁₅CONR₁₆R₁₇, NR₁₈CSNR₁₉R₂₀, NR₂SO₂R₂₂, NR₂₃CO₂R₂₄ ou un hétéroaryle éventuellement substitué préférentiellement choisi parmi les triazoles et oxadiazoles éventuellement substitués,
- R₂ représente OH, ou
- R₁ et R₂ forment ensemble, avec les atomes de carbone qui les portent, un hétérocycle éventuellement substitué, préférentiellement choisi parmi les cycles pyrimidine, pyrazole, pyrazolone, oxazoline, isoxazoline, oxazalanone, oxazalanethione, morpholinone et oxazepane éventuellement substitués, l'hétérocycle éventuellement substitué ne pouvant être : 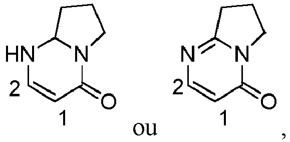 le carbone 1 désignant l'atome de carbone portant le groupe R₁ et le carbone 2 désignant l'atome de carbone portant le groupe R₂,
- R₃ représente H, OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)p)ᵣ(CH₂)qR₃₁, (NR₃₂(CH₂)_{w}NR₃₃(CH₂)ₓ)_{y}(CH₂)_{z}R₃₄, ONR₈₄R₈₅, aryle éventuellement substitué, ou hétéroaryle éventuellement substitué,
- R₄ est absent lorsque 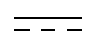 représente une liaison double et R₄ représente H ou OH lorsque 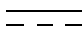 représente une liaison simple,
- R₁₀ à R₃₀, R₃₂, R₃₃, R₃₈ et R₃₉ représentent, indépendamment les uns des autres, H ou un groupe (C₁-C₆)alkyle, aryle, aryl-(C₁-C₆)alkyle ou (C₁-C₆)alkyl-aryle, ledit groupe étant éventuellement substitué par un ou plusieurs groupes choisis parmi (C₁-C₆)alkyle, OR₃₅, et NR₃₆R₃₇, ou
- R₁₁ et R₁₂, ou R₁₆ et R₁₇, ou R₁₉ et R₂₀, ou R₂₆ et R₂₇, ou R₂₉ et R₃₀, forment ensemble, avec l'atome d'azote qui les porte, un hétérocycle azoté éventuellement substitué,
- R₃₁ et R₃₄ représentent, indépendamment l'un de l'autre, H, OR₃₅, NR₃₆R₃₇, ONR₈₆R₈₇ ou un groupe (C₁-C₆)alkyle, aryle, aryl-(C₁-C₆)alkyle ou (C₁-C₆)alkyl-aryle, ledit groupe étant éventuellement substitué par un ou plusieurs groupes choisis parmi (C₁-C₆)alkyle, OR₃₅, et NR₃₆R₃₇,
- R₃₅ à R₃₇ et R₈₄ à R₈₇ représentent, indépendamment les uns des autres, H ou un groupe (C₁-C₆)alkyle, aryle ou aryl-(C₁-C₆)alkyle,
- Ra représente un atome d'halogène (par ex. Br ou C1), CN ou un groupe (C₁-C₆)alcoxy (tel que méthoxy),
- Rb représente H ou un groupe (C₁-C₆)alcoxy (tel que méthoxy), ou
- Ra et Rb forment ensemble une chaîne -OCH₂O-, et
- m, p, r, q, w, x, y, z représentent, indépendamment les uns des autres, un nombre entier compris entre 1 et 4,
à condition que lorsque R₁ représente CO₂R₁₀ ou CONH₂ et que n=1 ou 2 alors R₃ représente OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃(CH₂)ₓ)_{y}(CH₂)_{z}R₃₄, ou ONR₈₄R₈₅ avec R₂₅ ≠ H.

De préférence, lorsque R₁ représente CO₂R₁₀ ou CONH₂, alors R₃ représente OR₂₅, CHOHCH₂OH, CHO, N₃, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃(CH₂)ₓ)_{y}(CH₂)_{z}R₃₄, ou ONR₈₄R₈₅ avec R₂₅ ≠ H.

Dans la présente invention, on entend désigner par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même que pharmaceutique humaine.

On entend désigner par « sel et/ou solvate pharmaceutiquement acceptable » d'un composé, un sel et/ou solvate qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que les acides chlorhydrique, bromhydrique, phosphorique, sulfurique et similaires ; ou formés avec des acides organiques pharmaceutiquement acceptables tels que les acides acétique, trifluoroacétique, propionique, succinique, fumarique, malique, tartarique, citrique, ascorbique, maléique, glutamique, benzoïque, salicylique, toluènesulfonique, méthanesulfonique, stéarique, lactique et similaires, et
(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la lysine, l'arginine et similaires ; ou avec une base inorganique pharmaceutiquement acceptable telle que la soude, la potasse, l'hydroxyde de calcium et similaires.

Ces sels peuvent être préparés à partir des composés selon l'invention contenant une fonction basique ou acide et les acides ou bases correspondants selon les méthodes chimiques conventionnelles.

Des solvates acceptables pour l'utilisation pharmaceutique des composés selon la présente invention incluent des solvates conventionnels tels que ceux formés, durant la dernière étape du procédé de préparation des composés selon l'invention, avec le(s) solvant(s) de réaction. A titre d'exemple, il peut être fait mention des solvates formés avec l'eau (appelés communément hydrates) ou avec l'éthanol.

Par « énantiomères », on entend désigner des composés qui sont des images l'un de l'autre dans un miroir mais non superposables.

Un mélange contenant des quantités égales de deux formes énantiomères individuelles de chiralité opposée est désigné par « mélange racémique ».

Par « (C₁-C₆)alkyle », on entend, au sens de la présente invention, une chaîne hydrocarbonée saturée, linéaire ou ramifiée comprenant 1 à 6 atomes de carbone. A titre d'exemple, on peut citer les groupes méthyle, éthyle, propyle, isopropyle, butyle, isobutyle, *sec*-butyle, *tert*-butyle, pentyle ou encore hexyle.

Par groupement « (C₁-C₆)alcoxy », on entend, au sens de la présente invention, un groupe (C₁-C₆)alkyle tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore *tert*-butoxy. Il s'agira en particulier d'un groupe méthoxy.

Par « aryle », on entend, au sens de la présente invention, un groupe hydrocarboné aromatique comportant de préférence 6 à 10 atomes de carbone et pouvant comprendre un ou deux cycles accolés. A titre d'exemple on peut citer un phényle ou un naphtyle. Avantageusement il s'agit du phényle.

Par « hétéroaryle », on entend, au sens de la présente invention, un groupe aromatique comprenant un ou plusieurs, notamment 1 ou 2, cycles hydrocarbonés accolés, dans lequel un ou plusieurs atomes de carbone, avantageusement 1 à 4 et encore plus avantageusement 1 ou 2, sont chacun remplacés par un hétéroatome choisi parmi les atomes de soufre, d'azote et d'oxygène. Des exemples de groupes hétéroaryle sont les groupes furyle, thiényle, pyrrolyle, pyridinyle, pyrimidinyle, pyrazolyle, imidazolyle, oxazolyle, isoxazolyle, oxadiazolyle, triazolyle, tétrazolyle ou encore indyle.

Par « hétérocycle », on entend, au sens de la présente invention, un groupe hydrocarboné saturé, insaturé ou aromatique comprenant 1 ou 2 cycles accolés et dans lequel un ou plusieurs, avantageusement 1 à 4, encore plus avantageusement 1 ou 2, des atomes de carbone sont remplacés chacun par un hétéroatome choisi parmi l'oxygène, l'azote et le soufre. Avantageusement, l'hétérocycle comprendra 5 à 10 atomes de carbone et hétéroatomes. A titre d'exemple on peut citer les cycles furane, pyrrole, thiophène, thiazole, triazoles, isothiazole, oxadiazole, imidazole, oxazole, isoxazole, pyrazole, pyridine, pyrimidine, pipérazine, pipéridine, pyrazolone, oxazoline, isoxazoline, oxazalanone, oxazalanethione, morpholinone, oxazepane, quinazoline, quinoline, quinoxaline, benzofurane, benzothiophène, indoline, indolizine, benzothiazole, benzothiophène, benzopyranne, benzoxazole, benzo[1,3]dioxole, benzoisoxazole, benzimidazole, chromane, chromène, dihydrobenzofurane, dihydrobenzothiophène, dihydroisoxazole, isoquinoline, dihydrobenzo[1,4]dioxine, imidazo[1,2-a]pyridine, furo[2,3-c]pyridine, 2,3-dihydro-1*H*-indène, [1,3]dioxolo[4,5-c]pyridine, pyrrolo[1,2-c]pyrimidine, pyrrolo[1,2-a]pyrimidine, tétrahydronaphtalène, et benzo[b][1,4]oxazine.

Par « hétérocycle azoté », on entend, au sens de la présente invention, un hétérocycle tel que défini ci-dessus comprenant au moins un atome d'azote, de préférence saturé. Il pourra s'agir en particulier d'un cycle à 5 ou 6 chaînons comprenant éventuellement, en plus de l'atome d'azote, un autre hétéroatome choisi de préférence parmi l'oxygène et l'azote. Il s'agira en particulier d'un groupe pipéridine, pipérazine, morpholine ou pyrolidine.

Par « aryl-(C₁-C₆)alkyle », on entend, au sens de la présente invention un groupe aryle tel que défini ci-dessus lié au reste de la molécule par l'intermédiaire d'un groupe alkyle tel que défini ci-dessus et comprenant 1 à 6, avantageusement 1 à 4, de préférence 1 ou 2, atomes de carbone. Il s'agira notamment d'un groupe benzyle ou phénéthyle.

Par « (C₁-C₆)alkyl-aryle », on entend, au sens de la présente invention, un groupe (C₁-C₆)alkyle tel que défini ci-dessus, lié au reste de la molécule par l'intermédiaire d'un groupe aryle tel que défini ci-dessus. A titre d'exemple, on peut citer le groupe tolyle (CH₃Ph).

Par « éventuellement substitué », on entend, au sens de la présente invention, que le groupe en question est éventuellement substitué par un ou plusieurs substituants qui peuvent être choisis notamment parmi un atome d'halogène, un groupement SO₂, CN, NO₂, OR₉₅, SR₉₆, NR₉₇R₉₈, (C₁-C₆)alkyle, (C₁-C₆)alkyl-aryle, aryl-(C₁-C₆)alkyle, hétérocycle ou aryle, avec R₉₅ à R₉₈ représentant, indépendamment les uns des autres, H ou un groupe (C₁-C₆)alkyle, aryle, aryl-(C₁-C₆)alkyle ou (C₁-C₆)alkyl-aryle, ledit groupe étant éventuellement substitué par un ou plusieurs groupes choisis parmi (C₁-C₆)alkyle, OR₉₉, et NR₁₀₀R₁₀₁, R₉₉ à R₁₀₁ représentant, indépendamment les uns des autres, H ou un groupe (C₁-C₆)alkyle,
ou R₉₇ et R₉₈, formant ensemble, avec l'atome d'azote qui les porte, un hétérocycle azoté éventuellement substitué.

De préférence, R₉₅ à R₉₈ représentent, indépendamment les uns des autres, H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₉₇ et R₉₈ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine.

Par « atome d'halogène », on entend, au sens de la présente invention, les atomes de fluor, de chlore, de brome et d'iode. Il s'agira en particulier d'un atome de brome ou de chlore.

Par « polyamine », on entend, au sens de la présente invention, une chaîne hydrocarbonée linéaire comprenant de 4 à 15 atomes de carbone, et dont au moins deux de ces atomes de carbone sont remplacés par des atomes d'azote, deux atomes d'azote ne pouvant se retrouver sur des positions adjacentes. Ladite polyamine pourra répondre notamment à la formule suivante :

R₉₁-[(CH₂)ₐ-NR₉₂]_{d}-[(CH₂)_{b}-NR₉₃-(CH₂)_{c}-NR₉₄]ₑ-

avec a, b et c représentant, indépendamment les uns des autres, un nombre entier compris entre 1 et 5 et d et e représentant chacun 0, 1 ou 2 mais ne pouvant pas représenter 0 en même temps et d ne pouvant représenter 1 quand e = 0 et avec R₉₁ à R₉₄ représentant H ou un groupe (C₁-C₆)alkyle, aryle, (C₁-C₆)alkyl-aryle ou aryl-(C₁-C₆)alkyle.

De manière avantageuse, n est compris entre 1 et 4.

Selon un mode de réalisation particulier, Ra = (C₁-C₆)alcoxy tel que OMe et Rb = H.

Une classe de composés particulièrement appréciée correspond aux composés de formule (I) dans laquelle :
- 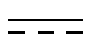 représente une liaison simple,
- R₁ représente CO₂R₁₀ ou CONH₂, notamment CO₂R₁₀,
- R₃ représente OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)qR₃₁, (NR₃₂(CH₂)_{w}NR₃₃(CH₂)ₓ)_{y}(CH₂)_{z}R₃₄, ONR₈₄R₈₅, aryle éventuellement substitué, ou hétéroaryle éventuellement substitué, en particulier OR₂₅, CHOHCH₂OH, CHO, N₃, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃(CH₂)ₓ)_{y}(CH₂)_{z}P₃₄, ONR₈₄R₈₅, aryle éventuellement substitué, ou hétéroaryle éventuellement substitué, et
- R₄ représente H.

Dans ce cas, R₁₀ représente avantageusement H ou un groupe (C₁-C₆)alkyle tel que méthyle.

De préférence, R₃ représentera OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃ (CH₂)ₓ)_{y}(CH₂)_{z}R₃₄ ou ONR₈₄R₈₅ ; notamment OR₂₅, CHOHCH₂OH, CHO, N₃, NR₂₆R₂₇, CO₂R₂₈, CONR₂₉R₃₀, (NR₃₂(CH₂)_{w}NR₃₃(CH₂)ₓ)_{y}(CH₂)_{z}R₃₄ ou ONR₈₄R₈₅; avantageusement NR₂₆R₂₇, CONR₂₉R₃₀, (NR₃₂(CH₂)_{w}NR₃₃(CH₂)ₓ)_{y}(CH₂)_{z}R₃₄ ou ONR₈₄R₈₅ avec R₂₅ à R₃₄, R₃₈, R₃₉, R₈₄ et R₈₅ tels que définis précédemment et notamment avec :
- R₂₅ étant tel que défini précédemment et représentant avantageusement H ou un groupe (C₁-C₆)alkyle, notamment H,
- R₂₆ et R₂₇ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₆ et R₂₇ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine, notamment R₂₆ et R₂₇ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine,
- R₂₈, R₃₂, R₃₃, R₃₈ et R₃₉ représentant, indépendamment les uns des autres, H ou un groupe (C₁-C₆)alkyle, aryle, aryl-(C₁-C₆)alkyle ou (C₁-C₆)alkyl-aryle, de préférence H ou un groupe (C₁-C₆)alkyle,
- R₂₉ et R₃₀ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₉ et R₃₀ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine, et
- R₃₁ représentant H, OR₃₅, NR₃₆R₃₇, ONR₈₆R₈₇. ou un groupe (C₁-C₆)alkyle, aryle, aryl-(C₁-C₆)alkyle ou (C₁-C₆)alkyl-aryle, de préférence H, OR₃₅, ou un groupe (C₁-C₆)alkyle,
- R₃₄ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, NR₃₆R₃₇ ou un groupe (C₁-C₆)alkyle, et
- R₃₅ à R₃₇ et R₈₄ à R₈₇ étant tels que définis précédemment et représentant avantageusement H ou un groupe (C₁-C₆)alkyle.

R₃ pourra représenter également avantageusement OR₂₅, CHOHCH₂OH, CHO, N₃, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃(CH₂)ₓ)_{y}(CH₂)_{z}R₃₄ ou ONR₈₄R₈₅ ; notamment OR₂₅, CHOHCH₂OH, CHO, N₃, CO₂R₂₈, CONR₂₉R₃₀, (NR₃₂(CH₂)_{w}NR₃₃(CH₂)ₓ)_{y}(CH₂)_{z}R₃₄ ou ONR₈₄R₈₅; avantageusement CONR₂₉R₃₀, (NR₃₂(CH₂)_{w}NR₃₃(CH₂)ₓ)_{y}(CH₂)_{z}R₃₄ ou ONR₈₄R₈₅ avec R₂₅ à R₃₄, R₃₈, R₃₉, R₈₄ et R₈₅ tels que définis précédemment et notamment avec :
- R₂₅ étant tel que défini précédemment et représentant avantageusement H ou un groupe (C₁-C₆)alkyle, notamment H,
- R₂₈, R₃₂, R₃₃, R₃₈ et R₃₉ représentant, indépendamment les uns des autres, H ou un groupe (C₁-C₆)alkyle, aryle, aryl-(C₁-C₆)alkyle ou (C₁-C₆)alkyl-aryle, de préférence H ou un groupe (C₁-C₆)alkyle,
- R₂₉ et R₃₀ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₉ et R₃₀ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine, et
- R₃₁ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, OR₃₅, ou un groupe (C₁-C₆)alkyle,
- R₃₄ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, NR₃₆R₃₇ ou un groupe (C₁-C₆)alkyle, et
- R₃₅ à R₃₇ et R₈₄ à R₈₇ étant tels que définis précédemment et représentant avantageusement H ou un groupe (C₁-C₆)alkyle.

Une autre classe de composés particulièrement appréciée correspond aux composés de formule (I) dans laquelle :
- 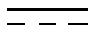 représente une liaison simple,
- R₁ représente NR₁₁R₁₂, NR₁₃COR₁₄, NR₁₅CONR₁₆R₁₇, NR₁₈CSNR₁₉R₂₀ ou NR₂₁SO₂R₂₂, et
- R₄ représente H.

De préférence R₃ est tel que défini ci-dessus, de préférence R₃ représente H.

R₁₁ à R₂₂ sont tels que définis ci-dessus et de préférence :
R₁₁ à R₂₂ représentent, indépendamment les uns des autres, H ou un groupe (C₁-C₆)alkyle, éventuellement substitué par un ou plusieurs groupes choisis parmi (C₁-C₆)alkyle, OR₃₅, et NR₃₆R₃₇, de préférence choisis parmi (OR₃₅, et NR₃₆R₃₇, tels que NR₃₆R₃₇, ou
R₁₁ et R₁₂, ou R₁₆ et R₁₇, ou R₁₉ et R₂₀, forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine.

De préférence, R₁₁ à R₂₂ représentent, indépendamment les uns des autres, H ou un groupe (C₁-C₆)alkyle, éventuellement substitué par un ou plusieurs groupes choisis parmi (C₁-C₆)alkyle, OR₃₅, et NR₃₆R₃₇, de préférence choisis parmi (OR₃₅, et NR₃₆R₃₇, tels que NR₃₆R₃₇.

Une autre classe de composés particulièrement appréciée correspond aux composés de formule (I) dans laquelle :
- 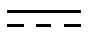 représente une liaison simple,
- R₁ représente 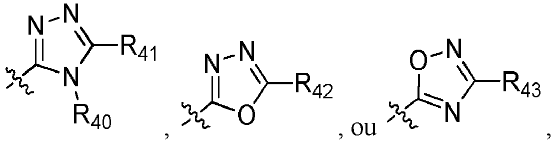
- R₄ représente H,
- R₄₀ représente H ou un groupe (C₁-C₆)alkyle, aryle ou aryl-(C₁-C₆)alkyle, notamment H ou un groupe (C₁-C₆)alkyle, avantageusement H,
- R₄₁ à R₄₃ représentent, indépendamment les uns des autres, H ou un groupe (C₁-C₆)alkyle, aryle, aryl-(C₁-C₆)alkyle,OR₄₄, SR₄₅ ou NR₄₆R₄₇, notamment H ou un groupe (C₁-C₆)alkyle, OR₄₄, SR₄₅ ou NR₄₆R₄₇, en particulier un groupe (C₁-C₆)alkyle, OR₄₄, SR₄₅ ou NR₄₆R₄₇,
- R₄₄ à R₄₇ représentent, indépendamment les uns des autres, H ou un groupe (C₁-C₆)alkyle, aryle ou aryl-(C₁-C₆)alkyle,ledit groupe étant éventuellement substitué par un ou plusieurs groupes choisis parmi (C₁-C₆)alkyle, OR₄₈, NR₄₉R₅₀, et les polyamines, notamment choisis parmi (C₁-C₆)alkyle, OR₄₈ et NR₄₉R₅₀, en particulier choisis parmi OR₄₈ et NR₄₉R₅₀, tels que NR₄₉R₅₀, ou
- R₄₆ et R₄₇ forment ensemble, avec l'atome d'azote qui les porte, un hétérocycle azoté éventuellement substitué par un groupe (C₁-C₆)alkyle, et
- R₄₈ à R₅₀ représentent, indépendamment les uns des autres, H ou un groupe (C₁-C₆)alkyle, aryle ou aryl-(C₁-C₆)alkyle.

L'hétérocycle azoté sera avantageusement un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine.

De préférence, R₄₄ à R₄₇ représentent, indépendamment les uns des autres, H ou un groupe (C₁-C₆)alkyle éventuellement substitué par un ou plusieurs groupes choisis parmi (C₁-C₆)alkyle, OR₄₈, NR₄₉R₅₀, et les polyamines, notamment choisis parmi (C₁-C₆)alkyle, OR₄₈ et NR₄₉R₅₀, en particulier choisis parmi OR₄₈ et NR₄₉R₅₀, tels que NR₄₉R₅₀.

Avantageusement, R₃ représentera H, OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃ (CH₂)ₓ)_{y}(CH₂)_{z}R₃₄ ou ONR₈₄R₈₅ ; notamment H, OR₂₅, CHOHCH₂OH, CHO, N₃, NR₂₆R₂₇, CO₂R₂₈, CONR₂₉R₃₀, ou (NR₃₂(CH₂)_{w}NR₃₃(CH₂)ₓ)_{y}(CH₂)_{z}R₃₄, avec R₂₅ à R₃₄, R₃₈, R₃₉, R₈₄ et R₈₅ tels que définis précédemment et notamment avec :
- R₂₅ représentant H ou un groupe (C₁-C₆)alkyle, notamment H,
- R₂₆ et R₂₇ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₆ et R₂₇ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine,
- R₂₈, R₃₂, R₃₃, R₃₈ et R₃₉ représentant, indépendamment les uns des autres, H ou un groupe (C₁-C₆)alkyle, aryle, aryl-(C₁-C₆)alkyle ou (C₁-C₆)alkyl-aryle, de préférence H ou un groupe (C₁-C₆)alkyle,
- R₂₉ et R₃₀ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₉ et R₃₀ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine, et
- R₃₁ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, OR₃₅, ou un groupe (C₁-C₆)alkyle,
- R₃₄ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, NR₃₆R₃₇ ou un groupe (C₁-C₆)alkyle, et
- R₃₅ à R₃₇ et R₈₄ à R₈₇ étant tels que définis précédemment et représentant avantageusement H ou un groupe (C₁-C₆)alkyle.

Une autre classe de composés particulièrement appréciée correspond aux composés de formule (I) dans laquelle le motif 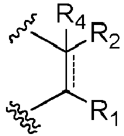 représente : 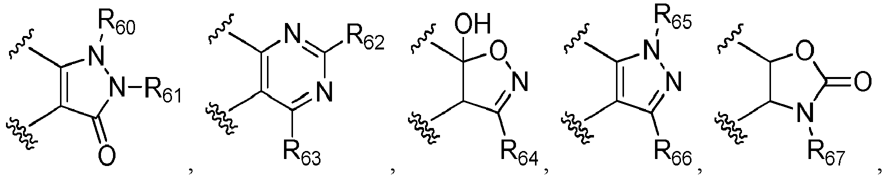 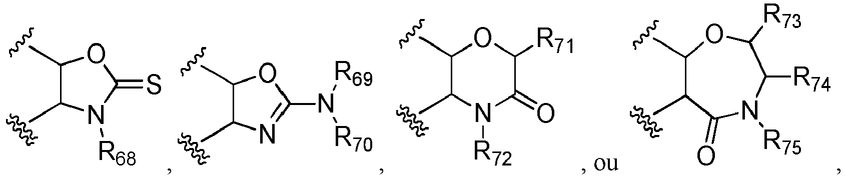 avec :
- R₆₀, R₆₁, R₆₅, R₆₇, R₆₈, R₇₂ et R₇₅ représentant, indépendamment les uns des autres, H ou un groupe (C₁-C₆)alkyle, aryle ou aryl-(C₁-C₆)alkyle,notamment H ou un groupe (C₁-C₆)alkyle, de préférence H,
- R₆₉ et R₇₀ représentant, indépendamment l'un de l'autre, H ou un groupe (C₁-C₆)alkyle, aryle ou aryl-(C₁-C₆)alkyle,ou formant ensemble, avec l'atome d'azote qui les porte, un hétérocycle azoté éventuellement substitué par un groupe (C₁-C₆)alkyle,
- R₆₂, R₆₃, R₆₄, R₆₆, R₇₁, R₇₃ et R₇₄ représentant, indépendamment les uns des autres, H ou un groupe (C₁-C₆)alkyle, aryle, aryl-(C₁-C₆)alkyle,OR₇₆, SR₇₇ ou NR₇₈R₇₉, et
- R₇₆ à R₇₉ représentant, indépendamment les uns des autres, H ou un groupe (C₁-C₆)alkyle, aryle, aryl-(C₁-C₆)alkyle,ou CN, ou
- R₇₈ et R₇₉ formant ensemble, avec l'atome d'azote qui les porte, un hétérocycle azoté éventuellement substitué par un groupe (C₁-C₆)alkyle.

L'hétérocycle azoté sera avantageusement un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine.

De préférence R₆₉ et R₇₀ représentent, indépendamment l'un de l'autre, H ou un groupe (C₁-C₆)alkyle, aryle ou aryl-(C₁-C₆)alkyle,notamment H ou un groupe (C₁-C₆)alkyle, en particulier H.

Avantageusement, R₆₂, R₆₃, R₆₄ et R₆₆ représentent, indépendamment les uns des autres, H ou un groupe (C₁-C₆)alkyle, OR₇₆, SR₇₇ ou NR₇₈R₇₉, notamment un groupe OR₇₆, SR₇₇ ou NR₇₈R₇₉.

Avantageusement, R₇₁, R₇₃ et R₇₄ représentent, indépendamment les uns des autres, H ou un groupe (C₁-C₆)alkyle, aryle, aryl-(C₁-C₆)alkyle,notamment, H ou un groupe (C₁-C₆)alkyle, en particulier H.

R₇₆ à R₇₉ représentent notamment, indépendamment les uns des autres, un groupe (C₁-C₆)alkyle, aryle, aryl-(C₁-C₆)alkyle,ou CN, en particulier un groupe (C₁-C₆)alkyle, aryle, ou aryl-(C₁-C₆)alkyle.

Selon un mode de réalisation particulier, lorsque 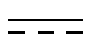 représente une liaison simple, alors R₁ et R₂ sont situés du même côté du noyau cyclopentane auquel ils sont liés, et de préférence du côté opposé aux groupes OH, phényle et *m*-Rb-*p*-Ra-phényle liés également à ce noyau cyclopentane.

Avantageusement, R₃ représentera H, OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, ou (NR₃₂(CH₂)_{w}NR₃₃ (CH₂)ₓ)_{y}(CH₂)_{z}R₃₄, ou ONR₈₄R₈₅ ; notamment H, OR₂₅, CHOHCH₂OH, CHO, N₃, NR₂₆R₂₇, CO₂R₂₈, CONR₂₉R₃₀, ou (NR₃₂(CH₂)_{w}NR₃₃(CH₂)ₓ)_{y}(CH₂)_{z}R₃₄, avec R₂₅ à R₃₄, R₃₈, R₃₉, R₈₄ et R₈₅ tels que définis précédemment et notamment avec :
- R₂₅ représentant H ou un groupe (C₁-C₆)alkyle, notamment H,
- R₂₆ et R₂₇ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₆ et R₂₇ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine,
- R₂₈, R₃₂, R₃₃, R₃₈ et R₃₉ représentant, indépendamment les uns des autres, H ou un groupe (C₁-C₆)alkyle, aryle, aryl-(C₁-C₆)alkyle ou (C₁-C₆)alkyl-aryle, de préférence H ou un groupe (C₁-C₆)alkyle,
- R₂₉ et R₃₀ représentant H ou un groupe (C₁-C₆)alkyle, aryle, aryl-(C₁-C₆)alkyle ou (C₁-C₆)alkyl-aryle, de préférence H ou un groupe (C₁-C₆)alkyle, ou R₂₉ et R₃₀ forment ensemble, avec l'atome d'azote qui les porte, un hétérocycle azoté éventuellement substitué par un groupe (C₁-C₆)alkyle, l'hétérocycle étant notamment un hétérocycle à 5 ou 6 chaînons comprenant éventuellement, en plus de l'atome d'azote, un autre hétéroatome choisi parmi l'oxygène et l'azote, tel qu'un groupe pipéridine, pipérazine, morpholine ou pyrolidine, et
- R₃₁ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, OR₃₅, ou un groupe (C₁-C₆)alkyle,
- R₃₄ représentant H, OR₃₅, NR₃₆R₃₇, ONR₈₆R₈₇, ou un groupe (C₁-C₆)alkyle, aryle, aryl-(C₁-C₆)alkyle ou (C₁-C₆)alkyl-aryle, de préférence H, NR₃₆R₃₇ ou un groupe (C₁-C₆)alkyle, et
- R₃₅ à R₃₇ et R₈₄ à R₈₇ étant tels que définis précédemment et représentant avantageusement H ou un groupe (C₁-C₆)alkyle.

Les composés de la présente invention pourront être choisis parmi les composés 1 à 55 exemplifiés ci-après, sous forme d'un de leurs énantiomères ou d'un mélange de leurs énantiomères tel qu'un mélange racémique, et les sels et/ou les solvates pharmaceutiquement acceptables de ceux-ci.

La présente invention a également pour objet un composé selon l'invention de formule (I) tel que défini ci-dessus, pour son utilisation en tant que médicament, notamment destiné au traitement du cancer.

La présente invention concerne également l'utilisation d'un composé de formule (I) tel que défini ci-dessus, pour la fabrication d'un médicament, notamment destiné au traitement du cancer.

La présente invention concerne également une méthode de traitement du cancer, comprenant l'administration à une personne en ayant besoin d'une dose efficace d'un composé de formule (I) tel que défini ci-dessus.

Le cancer pourra être plus particulièrement dans ce cas un cancer du côlon, un cancer du sein, un cancer du rein, un cancer du foie, un cancer du pancréas, un cancer de la prostate, un glioblastome, un cancer du poumon-non-à-petites-cellules, un neuroblastome, une tumeur myofibroblastique inflammatoire, un lymphome B diffus ou un lymphome anaplasique à grande cellules.

La présente invention concerne également une composition pharmaceutique comprenant au moins un composé de formule (I) tel que défini ci-dessus, et au moins un excipient pharmaceutiquement acceptable.

Les compositions pharmaceutiques selon l'invention peuvent être formulées notamment pour une administration orale ou par injection, lesdites compositions étant destinées aux mammifères, y compris l'homme.

L'ingrédient actif peut être administré sous formes unitaires d'administration, en mélange avec des supports pharmaceutiques classiques, aux animaux ou aux êtres humains. Les composés de l'invention en tant que principes actifs peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

Les compositions pharmaceutiques selon l'invention peuvent comprendre en outre au moins un autre principe actif, tel qu'un agent anticancéreux.

La présente invention a également pour objet une composition pharmaceutique comprenant :
(i) au moins un composé de formule (I) tel que défini ci-dessus, et
(ii) au moins un autre principe actif, tel qu'un agent anticancéreux,
en tant que produit de combinaison, pour une utilisation simultanée, séparée ou étalée dans le temps.

La présente invention concerne également une composition pharmaceutique telle que définie ci-dessus pour son utilisation en tant que médicament, notamment destiné au traitement du cancer.

La présente invention concerne également une méthode de traitement du cancer, comprenant l'administration à une personne en ayant besoin d'une dose efficace d'une composition pharmaceutique telle que définie ci-dessus.

La présente invention a également pour objet des procédés de préparation des composés de formule (I) selon l'invention.

Un premier procédé de préparation d'un composé de formule (I) selon l'invention pour lequel R₃ = H comprend la réaction d'un composé de formule (II) suivante : 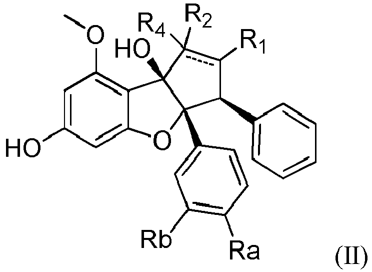 pour lequel Ra, Rb, R₁, R₂ et R₄ sont tels que définis précédemment, avec un alcool de formule H-(CH₂)ₙOH, pour lequel n est tel que défini précédemment, dans des conditions de Mitsunobu.

Une telle réaction peut être effectuée en présence de DEAD (diéthyl azodicarboxylate) ou de DMEAD (bis(2-méthoxyéthyl) azodicarboxylate) et de PPh₃.

Un deuxième procédé de préparation d'un composé de formule (I) selon l'invention pour lequel R₃ = N₃, NR₃₈COR₃₉ ou NR₂₆R₂₇ comprend :
(a1) pour obtenir un composé de formule (I) pour lequel R₃ = N₃, la réaction d'un composé de formule (III) suivante : 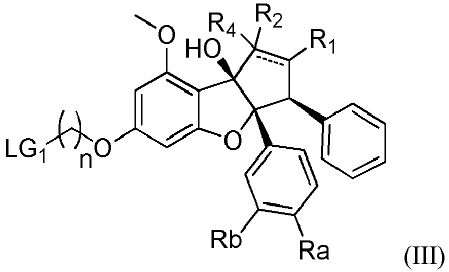 pour lequel Ra, Rb, R₁, R₂, R₄ et n sont tels que définis précédemment et LG₁ représente un groupe partant tel qu'un atome d'halogène ou une fonction hydroxyle activée,
   avec un azoture de formule MN₃, M représentant un métal alcalin ou un groupe SiRR'R" avec R, R′ et Rʺ représentant chacun, indépendamment les uns des autres, un groupe (C₁-C₆)alkyle ou aryle,
(b1) pour obtenir un composé de formule (I) pour lequel R₃ = NH₂, la réduction de la fonction azoture d'un composé de formule (I) pour lequel R₃ = N₃ éventuellement obtenu selon l'étape (al),
(c1) pour obtenir un composé de formule (I) pour lequel R₃ = NR₃₈COR₃₉ ou NR₂₆R₂₇ et au moins l'un de R₂₆ et R₂₇ ne représente pas un atome d'hydrogène, la substitution d'un composé de formule (I) pour lequel R₃ = NH₂ éventuellement obtenu selon l'étape (b1).

### Etape (a1):

Par « groupe partant », on entend, au sens de la présente invention, un groupement chimique qui peut être facilement déplacé par un nucléophile lors d'une réaction de substitution nucléophile, le nucléophile étant dans le cas présent un azoture. Un tel groupe partant peut être plus particulièrement un atome d'halogène tel qu'un atome de chlore ou de brome ou un sulfonate. Le sulfonate peut être en particulier un groupe -OSO₂-R₉₀ avec R₉₀ représentant un groupe (C₁-C₆)alkyle, aryle, aryl-(C₁-C₆)alkyle ou (C₁-C₆)alkyl-aryle, ledit groupe étant éventuellement substitué par un ou plusieurs atomes d'halogène tels que des atomes de fluor. Le sulfonate peut être en particulier un mésylate (-OS(O₂)-CH₃), un triflate (-OS(O)₂-CF₃) ou encore un tosylate (-OS(O)₂-(*p*-Me-C₆H₄)).

Le groupe partant peut également être une fonction alcool (OH) activée en présence par exemple de DPPA (azoture de diphénylphosphine) et de DBU (1,8-diazabicyclo[5.4.0]undéc-7-ène).

Le métal alcalin pourra être notamment Na, K ou Li.

R, R′ et Rʺ représenteront notamment chacun, indépendamment les uns des autres, un groupe méthyle ou phényle, notamment méthyle.

M représentera notamment Na ou SiMe₃.

### Etape (b1) :

L'étape de réduction de l'azoture pourra notamment être réalisée en présence d'hydrogène. Un catalyseur d'hydrogénation tel que le palladium sur charbon pourra être utilisé.

### Etape (c1) :

Lorsque R₃ = NR₂₆R₂₇, la substitution de la fonction amine pourra être réalisée par des méthodes bien connues de l'homme du métier, notamment par une réaction de substitution nucléophile en présence de R₂₆LG₄ et/ou R₂₇LG₅ où LG₄ et LG₅ représentent chacun, indépendamment l'un de l'autre, un groupe partant. Une telle réaction est avantageusement réalisée en présence d'une base. Lorsque R₂₆ et R₂₇ ne représentent pas un atome d'hydrogène, deux réactions successives pourront être réalisées afin d'introduire successivement les groupes R₂₆ et R₂₇.

Lorsque NR₃₈COR₃₉, cette étape pourra être réalisée par des méthodes bien connues de l'homme du métier, par exemple par couplage peptidique ou en utilisant des chlorures d'acyle de formule R₃₉COCl.

Un troisième procédé de préparation d'un composé de formule (I) selon l'invention, pour lequel R₃ = CHOHCH₂OH, CHO, CO₂R₂₈, CONR₂₉R₃₀, OR₂₅, (O(CH₂)ₘO(CH₂)ₚ)ᵣ(CH₂)_{q}R₃₁, NR₂₆R₂₇ ou (NR₃₂(CH₂)_{w}NR₃₃(CH₂)ₓ)_{y}(CH₂)_{z}R₃₄, comprend :
(a2) pour obtenir un composé de formule (I) pour lequel R₃ = CHOHCH₂OH, la réaction de dihydroxylation de la fonction vinyle d'un composé de formule (IV) suivante : 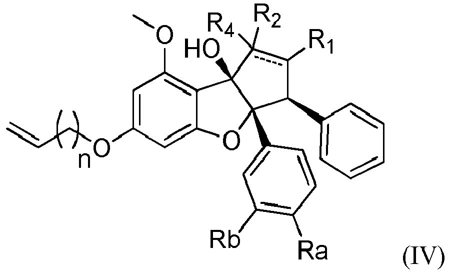 pour lequel Ra, Rb, R₁, R₂, R₄ et n sont tels que définis précédemment,
(b2) pour obtenir un composé de formule (I) pour lequel R₃ = CHO, la coupure oxydante du groupe CHOHCH₂OH d'un composé de formule (I) pour lequel R₃ = CHOHCH₂OH éventuellement obtenu selon l'étape (a2),
(c2) pour obtenir un composé de formule (I) pour lequel R₃ = CO₂H, l'oxydation de la fonction aldéhyde d'un composé de formule (I) pour lequel R₃ = CHO éventuellement obtenu selon l'étape (b2),
(d2) pour obtenir un composé de formule (I) pour lequel R₃ = CO₂R₂₈ et R₂₈ ≠ H, la substitution de la fonction acide carboxylique d'un composé de formule (I) pour lequel R₃ = CO₂H éventuellement obtenu selon l'étape (c2),
(e2) pour obtenir un composé de formule (I) pour lequel R₃ = CONR₂₉R₃₀, la réaction d'un composé de formule (I) pour lequel R₃ = CO₂R₂₈, éventuellement obtenu selon l'étape (b2) ou (c2), avec une amine de formule HNR₂₉R₃₀,
(f2) pour obtenir un composé de formule (I) pour lequel R₃ = OH, la réduction de la fonction aldéhyde d'un composé de formule (I) pour lequel R₃ = CHO éventuellement obtenu selon l'étape (b2),
(g2) pour obtenir un composé de formule (I) pour lequel R₃ = OR₂₅ ou (O(CH₂)ₘO(CH₂)ₚ)ᵣ(CH₂)_{q}R₃₁, NR₂₆R₂₇ avec R₂₅ ≠ H, la substitution de la fonction hydroxyle d'un composé de formule (I) pour lequel R₃ = OH éventuellement obtenu selon l'étape (f2),
(h2) pour obtenir un composé de formule (I) pour lequel R₃ = NR₂₆R₂₇, l'amination réductrice de la fonction aldéhyde d'un composé de formule (I) pour lequel R₃ = CHO, éventuellement obtenu selon l'étape (b2), en présence d'une amine de formule HNR₂₆R₂₇.

### Etape (a2) :

Une telle réaction peut être réalisée en particulier en présence de OsO₄ et de NMO (4-méthylmorpholine N-oxyde).

### Etape (b2) :

Cette réaction peut être réalisée notamment en présence deNaIO₄.

### Etape (c2) :

Les conditions d'oxydation sont bien connues de l'homme du métier.

### Etape (d2) ou (g2) :

Une telle réaction pourra être réalisée dans des conditions de substitution nucléophile bien connues de l'homme du métier. La fonction acide carboxylique pourra être activée au préalable, notamment sous forme de chlorure d'acyle ou d'anhydride. De même, la fonction alcool pourra être transformée en groupe partant tel qu'un atome d'halogène ou un sulfonate par exemple.

### Etape (e2) :

Une telle réaction pourra être réalisée dans des conditions de couplage peptidique ou par substitution nucléophile après activation de la fonction acide carboxylique, notamment sous forme de chlorure d'acyle ou d'anhydride.

### Etape (f2) :

Les conditions de réduction sont bien connues de l'homme du métier. NaBH₄ pourra notamment être utilisé comme agent réducteur.

### Etape (h2) :

Les conditions d'amination réductrice sont bien connues de l'homme du métier. Une telle réaction peut être réalisée en présence de NaBHOAc₃ comme agent réducteur.

Un quatrième procédé de préparation d'un composé de formule (I) selon l'invention, pour lequel R₁ représente 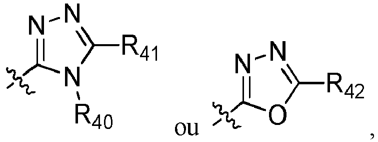 comprend la réaction d'un composé de formule (V) suivante : 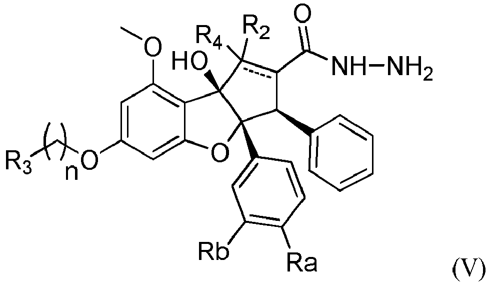 pour lequel Ra, Rb, R₂, R₃, R₄ et n sont tels que définis précédemment, avec :
(1) un cyanogène de formule Hal-CN, pour lequel Hal représente un atome d'halogène tel que Br, ou un isothiocyanate de formule 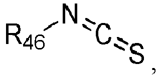 notamment en présence d'une base telle que NaHCO₃, suivie éventuellement d'une ou plusieurs étapes de substitution pour donner un composé de formule (I) pour lequel R₁ représente 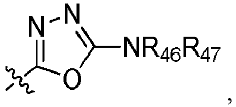 ou
(2) du sulfure de carbone (CS₂) en présence d'une base telle que NaOH ou KOH, suivie éventuellement d'une ou plusieurs étapes de substitution pour donner un composé de formule (I) pour lequel R₁ représente 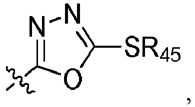 ou
(3) du carbonyldiimidazole en présence d'une base telle que la triéthylamine, suivie éventuellement d'une ou plusieurs étapes de substitution pour donner un composé de formule (I) pour lequel R₁ représente 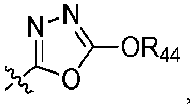 ou
(4) un acide carboxylique de formule R₄₂COOH, pour lequel R₄₂ représente un groupe (C₁-C₆)alkyle, aryle ou aryl-(C₁-C₆)alkyle,en présence de POCl₃ pour donner un composé de formule (I) pour lequel R₁ représente 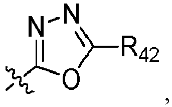 ou
(5) une isothiourée de formule 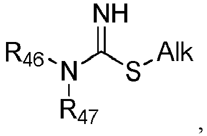 pour laquelle Alk représente un groupe (C₁-C₆)alkyle, en présence d'une base telle que NaOH ou KOH, suivie éventuellement d'une ou plusieurs étapes de substitution pour donner un composé de formule (I) pour lequel R₁ représente 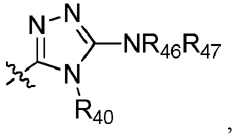 ou
(6) un imidate de formule 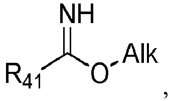 pour lequel R₄₁ représente un groupe (C₁-C₆)alkyle, aryle ou aryl-(C₁-C₆)alkyle et Alk représente un groupe (C₁-C₆)alkyle, en présence d'une base telle que la triéthylamine, suivie éventuellement d'une ou plusieurs étapes de substitution pour donner un composé de formule (I) pour lequel R₁ représente 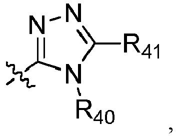 ou
(7) du triéthyl orthoformate suivie éventuellement d'une ou plusieurs étapes de substitution pour donner un composé de formule (I) pour lequel R₁ représente

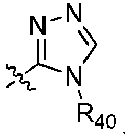

Les conditions réactionnelles de ces différentes réactions sont bien connues de l'homme du métier et sont généralement exemplifiés dans la partie expérimentale ci-après. Il en est de même des réactions de substitution.

Lorsque R₁ représente 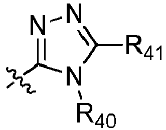 avec R₄₁ = H, le composé de formule (I) selon l'invention pourra être préparé par réaction d'un composé de formule (I) pour lequel R₁ = CONH₂ avec du diméthylformamide diméthylacétate, puis réaction du produit obtenu avec de l'hydrazine.

Lorsque R₁ représente 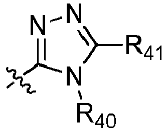 avec R₄₁ = OR₄₄, le composé de formule (I) selon l'invention pourra être préparé par réaction d'un composé de formule (I) pour lequel R₁ 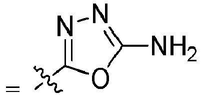 par réarrangement de l'amino-oxadiazole en présence de KOH et d'un alcool R₄₄OH.

Lorsque R₁ représente 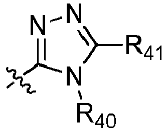 avec R₄₁ = SR₄₅, le composé de formule (I) selon l'invention pourra être préparé par réaction d'un composé de formule (I) pour lequel R₁ = COOH avec un thiosemicarbazide.

Un cinquième procédé de préparation d'un composé de formule (I) selon l'invention, pour lequel R₁ représente 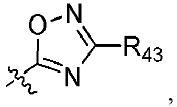 comprend la réaction d'un composé de formule (I) avec R₁ = CO₂R₁₀ et R₁₀ représente un groupe (C₁-C₆)alkyle, avec un hydroxy-imidamide de formule 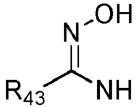 en présence d'une base telle que K₂CO₃.

Les conditions réactionnelles d'une telle réaction sont exemplifiées dans la présente demande de brevet.

Un sixième procédé de préparation d'un composé de formule (I) selon l'invention, pour lequel 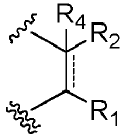 représente 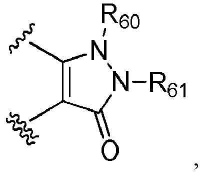 comprend la réaction d'un composé de formule (I), pour lequel 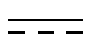 représente une liaison double, R₁ = CO₂R₁₀, R₂ = OH, R₄ = H et R₁₀ = (C₁-C₆)alkyle, avec une hydrazine de formule H₂N-NH₂, suivie éventuellement d'une ou plusieurs étapes de substitution.

Les conditions réactionnelles d'une telle réaction de couplage avec l'hydrazine sont exemplifiées dans la présente demande de brevet. L'homme du métier sait également comment mettre en œuvre une réaction de substitution.

Un septième procédé de préparation d'un composé de formule (I) selon l'invention, pour lequel 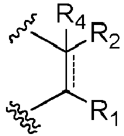 représente 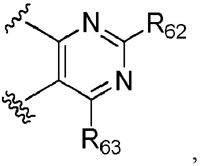 comprend la réaction d'un composé de formule (VI) suivante : 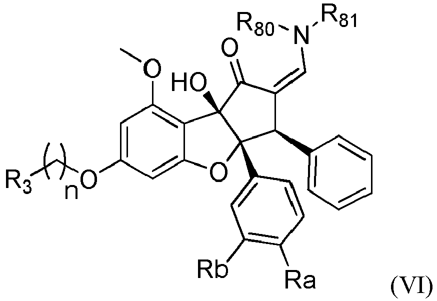 pour lequel Ra, Rb, R₃ et n sont tels que définis précédemment, et R₈₀ et R₈₁ représentent, indépendamment l'un de l'autre, un groupe (C₁-C₆)alkyle, avec un dérivé de guanidine de formule

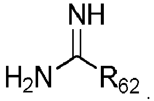

Les conditions réactionnelles d'une telle réaction de couplage avec un dérivé de guanidine sont exemplifiées dans la présente demande de brevet.

Un huitième procédé de préparation d'un composé de formule (I) selon l'invention, pour lequel 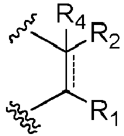 représente 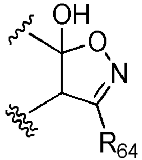 ou 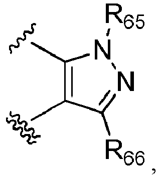 comprend la réaction d'un composé de formule (VII) suivante : 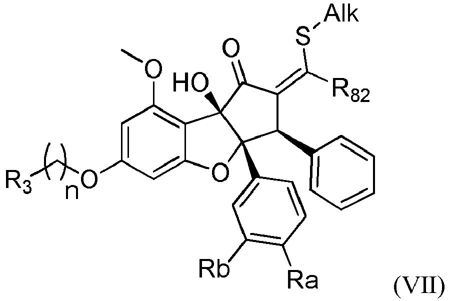 pour lequel Ra, Rb, R₃ et n sont tels que définis précédemment, Alk représente un groupe (C₁-C₆)alkyle, et R₈₂ représente R₆₄ ou R₆₆, avec :
(1) l'hydroxylamine de formule HO-NH₂ en présence d'une base telle que la triéthylamine, pour donner un composé de formule (I) pour lequel 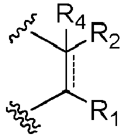 représente 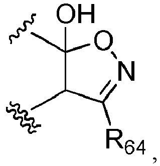 ou
(2) une hydrazine de formule H₂N-NH₂, suivie éventuellement d'une ou plusieurs étapes de substitution pour donner un composé de formule (I) pour lequel 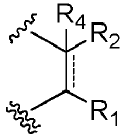 représente

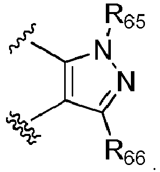

Les conditions réactionnelles de ces réactions de couplage sont exemplifiées dans la présente demande de brevet. L'homme du métier sait également comment mettre en œuvre une réaction de substitution.

Un neuvième procédé de préparation d'un composé de formule (I) selon l'invention, pour lequel 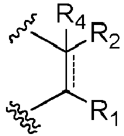 représente 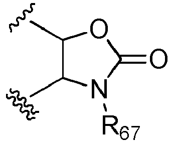 ou 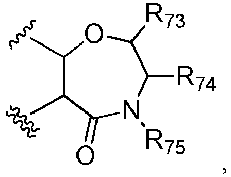 comprend la réaction d'un composé de formule (VIII) suivante : 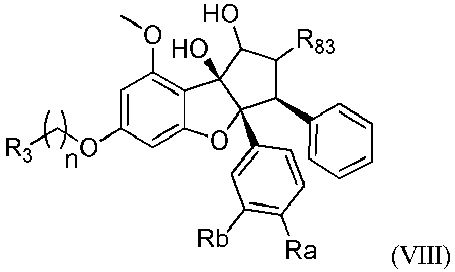 pour lequel Ra, Rb, R₃ et n sont tels que définis précédemment, et R₈₃ représente un groupe CO₂H éventuellement sous une forme activée, avec :
(1) un azoture, dans des conditions de réarrangement de Curtius, suivie éventuellement d'une ou plusieurs étapes de substitution, pour donner un composé de formule (I) pour lequel 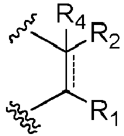 représente 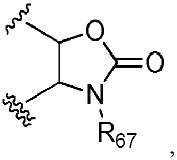 ou
(2) une amine de formule 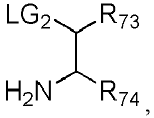 pour laquelle LG₂ représente un groupe partant tel qu'un halogène, en présence d'une base telle que la diisopropyléthylamine (DIEA), suivie éventuellement d'une ou plusieurs étapes de substitution, pour donner un composé de formule (I) pour lequel 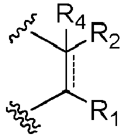 représente

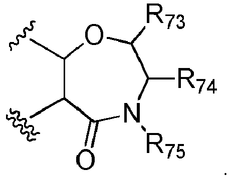

Le réarrangement de Curtius de l'étape (1) est une réaction bien connue de l'homme du métier. Il peut être réalisé notamment en présence d'azoture de diphénylphosphoryle (DPPA) et d'une base telle que la triéthylamine. Le composé (VIII) porte plus particulièrement un groupe R₈₃ = COOH.

Les conditions réactionnelles de l'étape (2) et des éventuelles réactions de substitution ultérieures sont bien connues de l'homme du métier.

Un dixième procédé de préparation d'un composé de formule (I) l'invention, pour lequel 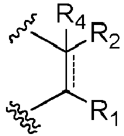 représente 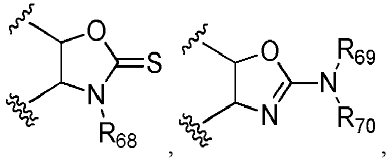 ou 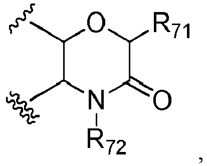 comprend la réaction d'un composé de formule (I), pour lequel 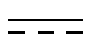 représente une liaison simple, R₁ = NH₂, R₂ = OH et R₄ = H, avec :
(1) du sulfure de carbone (CS₂) en présence d'une base telle que du carbonate de sodium, suivie éventuellement d'une ou plusieurs étapes de substitution, pour donner un composé de formule (I) pour lequel 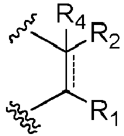 représente 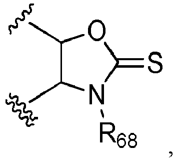 ou
(2) un cyanogène de formule Hal-CN, pour lequel Hal représente un atome d'halogène tel que Br, en présence d'une base telle que NaHCO₃, suivie éventuellement d'une ou plusieurs étapes de substitution, pour donner un composé de formule (I) pour lequel 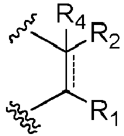 représente 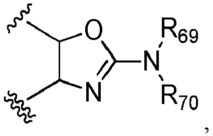 ou
(3) un ester de formule 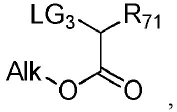 pour lequel Alk représente un groupe (C₁-C₆)alkyle et LG₃ représente un groupe partant tel qu'un atome d'halogène tel que Cl, en présence d'une base telle que NaH, suivie éventuellement d'une ou plusieurs étapes de substitution, pour donner un composé de formule (I) pour lequel 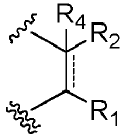 représente

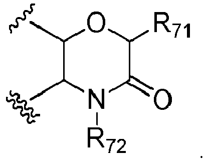

Les conditions réactionnelles de ces étapes et des éventuelles réactions de substitution ultérieures sont connues de l'homme du métier.

Les composés de formules (II) à (VIII) peuvent être préparés par des méthodes décrites dans l'art antérieur ou dans la présente demande de brevet.

Le composé de formule (I) obtenu par l'un des procédés mentionnés ci-dessus pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Les exemples qui suivent illustrent l'invention sans toutefois en limiter la portée.

### EXEMPLES

### 1- Synthèse des composés selon l'invention

Les abréviations suivantes ont été utilisées dans les exemples qui suivent :
- ES :: Electrospray
- HPLC :: Chromatographie liquide haute performance
- HRMS :: Spectre de masse haute résolution
- LCMS :: Chromatographie liquide couplée à un spectromètre de masse
- RMN :: Résonance magnétique nucléaire
- TA :: Température ambiante

Les noms des composés selon la présente invention ont été attribués par Autonom.

### 1.1. Composés avec R₁ = CO₂Me, Co₂H ou CONH₂ et R₂ = OH

Les composés selon l'invention peuvent être synthétisés selon le schéma réactionnel suivant : 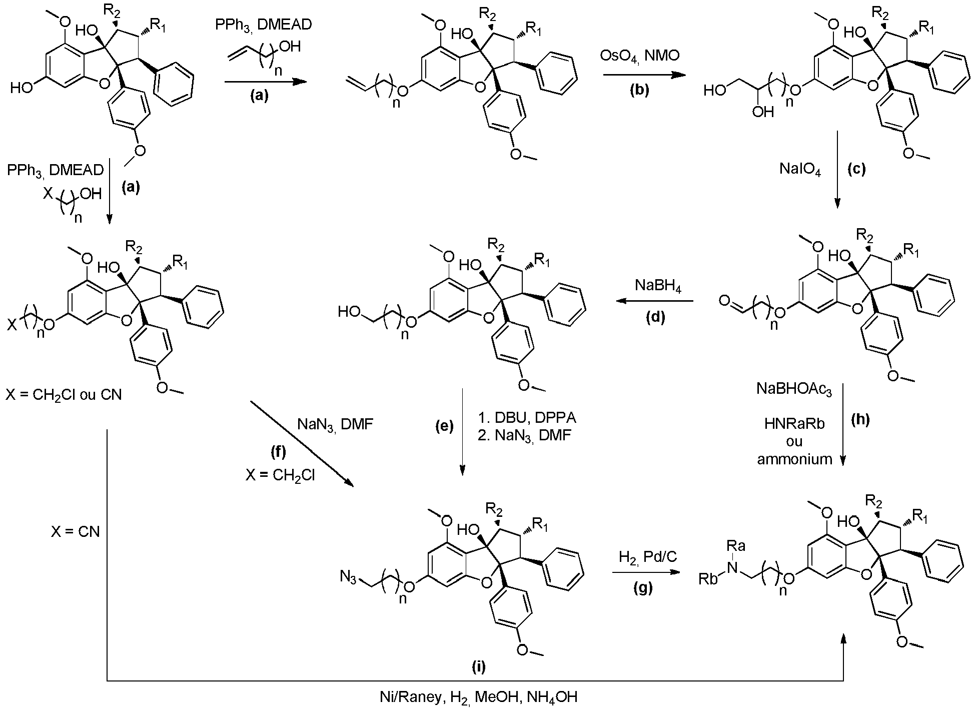
**(a) Procédure pour la réaction de substitution du phénol** (exemple R₁=CO₂Me, R₂=OH, n=3, alcool = 4-chloro-1-butanol) : Dans un ballon d'un litre, dissoudre rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b] benzofuran-2-carboxylate (R₁=CO₂Me, R₂=OH, 10,25 g, 21,42 mmol) dans 200 ml de toluène puis ajouter du 4-chloro-1-butanol (12,57 ml, 107 mmol) et PPh₃ (28,1 g, 107 mmol). A 0°C et sous azote, ajouter goutte à goutte du bis(2-méthoxyéthyl) azodicarboxylate (DMEAD, 8,1 g, 120 mmol) dissous dans 155 ml de toluène. Laisser agiter à 0°C pendant 10 min puis à température ambiante pendant 4h30. Ajouter un peu d'eau puis extraire 2 fois à l'acétate d'éthyle, laver une fois par H₂O/NaCl puis sécher les phases organiques sur MgSO₄, filtrer puis évaporer sous pression réduite. L'huile orange obtenue est reprise dans de l'éther diéthylique, un précipité blanc se forme, le solide blanc est éliminé par filtration et le filtrat est évaporé. Le résidu est purifié sur gel de silice en utilisant comme éluant un mélange cyclohexane / AcOEt 80/20 à 50/50.
**(b) Procédure pour la réaction de dihydroxylation** (exemple R₁=CO₂Me, R₂=OH, n=3) : A une solution de rac-methyl (1R,2R,3S,3aR,8bS)-1,8b-dihydroxy-8-methoxy-3a-(4-methoxyphenyl)-6-(pent-4-en-1-yloxy)-3-phenyl-2,3,3a,8b-tetrahydro-1H-cyclopenta[b]benzofuran-2-carboxylate (1,4 g, 2,56 mmol) dans le tétrahydrofurane (THF, 17 mL), sous atmosphère d'argon, ajouter du 4-méthylmorpholine N-oxyde monohydrate (NMO, 0,535 g, 3,84 mmol) dissous dans l'eau (2,5 mL) puis de l'acide osmique (3,24 ml, 0,512 mmol). Agiter vigoureusement magnétiquement à température ambiante pendant 1 heure. Ajouter une solution saturée de NaHSO₃ saturée, extraire avec de l'acétate d'éthyle deux fois, sécher la phase organique sur Na₂SO₄, filtrer puis évaporer. Le résidu est purifié sur gel de silice en utilisant comme éluant un mélange CH₂Cl₂/MeOH 95/5 à 80/20.
**(c) Procédure pour la réaction de coupure oxydante** (exemple R₁=CO₂Me, R₂=OH, n=3) : A une solution de rac-methyl (1R,2R,3S,3aR,8bS)-6-((4,5-dihydroxypentyl)oxy)-1,8b-dihydroxy-8-methoxy-3a-(4-methoxyphenyl)-3-phenyl-2,3,3a,8b-tetrahydro-1H-cyclopenta[b]benzofuran-2-carboxylate (102 mg, 0,176 mmol) dans le THF (1,5 mL) et l'eau (1,5 mL), ajouter le periodate de sodium (39 mg, 1,05 équiv.) à 0°C, puis laisser revenir le milieu à température ambiante et agiter magnétiquement pendant 3 heures. Additionner de l'eau et du dichlorométhane (DCM), extraire 3 fois avec du dichlorométhane puis de l'acétate d'éthyle. Sécher les phases organiques sur Na₂SO₄, filtrer puis évaporer. Utiliser le produit brut sans plus de purification.
**(d) Procédure pour la réaction de réduction de l'aldéhyde** (exemple R₁=CO₂Me, R₂=OH, n=3) : Dans un ballon, introduire rac-methyl (1R,2R,3S,3aR,8bS)-1,8b-dihydroxy-8-methoxy-3a-(4-methoxyphenyl)-6-(4-oxobutoxy)-3-phenyl-2,3,3a,8b-tetrahydro-1H-cyclopenta[b] benzofuran-2-carboxylate (2,66 g, 4,85 mmol) en solution dans le méthanol (50 mL), ajouter NaBH₄ (0,275 g, 7,27 mmol) et laisser agiter à température ambiante pendant 1h30. Ajouter une solution de NH₄Cl saturé (75 ml), concentrer puis extraire 3 fois avec de l'acétate d'éthyle. Rassembler les phases organiques, faire un lavage avec NaCl puis sécher sur Na₂SO₄, filtrer et concentrer sous pression réduite. Le résidu est purifié sur gel de silice en utilisant comme éluant DCM à DCM/MeOH 80/20.
**(e) Procédure de synthèse de l'azoture à partir de l'alcool** (exemple R₁=CO₂Me, R₂=OH, n=3) : Dans le ballon contenant rac-methyl (1R,2R,3S,3aR,8bS)-1,8b-dihydroxy-6-(4-hydroxybutoxy)-8-methoxy-3a-(4-methoxyphenyl)-3-phenyl-2,3,3a,8b-tetrahydro-1H-cyclopenta[b]benzofuran-2-carboxylate (1 g, 1,816 mmol), ajouter le tétrahydrofurane (8 mL). Abaisser la température à 0°C et additionner l'azoture de diphénylphosphoryle (DPPA, 0,866 ml, 4,00 mmol) et le 1,8-diazabicyclo[5.4.0]undéc-7-ène (DBU, 0,706 ml, 4,72 mmol). Laisser revenir à température ambiante et agiter magnétiquement pendant 1h. Evaporer le brut puis le diluer avec du diméthylformamide (DMF, 20 mL), ajouter l'azoture de sodium (0,354 g, 5,44 mmol) et chauffer le mélange à 110°C pendant 5 heures. Evaporer le DMF sous pression réduite et purifier le résidu sur gel de silice en utilisant comme éluant un mélange CH₂Cl₂/AcOEt 100/0 à 85/15.
**(f) Procédure de synthèse de l'azoture à partir du Chloré** (exemple R₁=CO₂Me, R₂=OH, n=3) : Dans le ballon contenant rac-methyl (1R,2R,3S,3aR,8bS)-6-(4-chlorobutoxy)-1,8b-dihydroxy-8-methoxy-3a-(4-methoxyphenyl)-3-phenyl-2,3,3a,8b-tetrahydro-1H-cyclopenta[b]benzofuran-2-carboxylate (3,6 g, 6,34 mmol) introduire le DMF (35 mL), ajouter l'azoture de sodium (0,825 g, 12.69 mmol) et chauffer le mélange à 110°C pendant 5 heures. Evaporer le DMF sous pression réduite et purifier le résidu sur gel de silice en utilisant comme éluant CH₂Cl₂/AcOEt 100/0 à 85/15.
**(g) Procédure réduction de l'azoture** (exemple R₁=CO₂Me, R₂=OH, n=3) : Dans un ballon de 500 ml, dissoudre rac-(1R,2R,3S,3aR,8bS)-methyl 6-(4-azidobutoxy)-1,8b-dihydroxy-8-methoxy-3a-(4-methoxyphenyl)-3-phenyl-2,3,3a,8b-tetrahydro-1H-cyclopenta[b]benzofuran-2-carboxylate (3,65 g, 6,34 mmol) dans l'éthanol (68 mL) et le tétrahydrofurane (68,0 mL). Purger sous azote puis ajouter le palladium sur charbon (1,350 g, 1,268 mmol) sous azote. Mettre le milieu réactionnel sous atmosphère d'hydrogène et laisser agiter à température ambiante pendant 3 heures. Filtrer le palladium sur gâteau de Dicalite^{®} puis rincer avec de l'éthanol et du THF, évaporer le filtrat sous pression réduite. Le résidu obtenu est purifié sur gel de silice en utilisant comme éluant un mélange cyclohexane/AcOEt 30/70 puis CH₂Cl₂/MeOH/NH₄OH (7 N dans le méthanol) 90/9/1.
**(h) Procédure pour l'amination réductrice** (exemple R₁=CO₂Me, R₂=OH, n=3) : Dans un ballon, introduire rac-methyl (1R,2R,3S,3aR,8bS)-1,8b-dihydroxy-8-methoxy-3a-(4-methoxyphenyl)-6-(4-oxobutoxy)-3-phenyl-2,3,3a,8b-tetrahydro-1H-cyclopenta [b]benzofuran-2-carboxylate (0,050 g, 0,091 mmol) en solution dans du 1,2-dichloroéthane (2 mL) et du méthanol (0,5 mL), ajouter l'acétate d'ammonium (5 équiv.) puis le triacétoxyborohydrure de sodium (2,0 equiv.), laisser agiter à température ambiante pendant 18h. Ajouter une solution saturée de NaHCO₃ puis diluer avec du DCM, extraire 2 fois au DCM puis rassembler les phases organiques et laver par une solution saturée de NaCl, sécher la phase organique sur Na₂SO₄, filtrer et concentrer sous pression réduite. Le résidu est purifié sur gel de silice en utilisant comme éluant un mélange DCM / MeOH 90/10. **(i) Procédure de réduction du nitrile** (exemple R₁ = CO₂Me, R₂ = OH, n = 3) : Dans une bombe à hydrogéner, mélanger le nickel de Raney (100 mg, 0,852 mmol), rac-(1R,2R,3S,3aR,8bS)-methyl-6-(3-cyanopropoxy)-1,8b-dihydroxy-8-methoxy-3a-(4-methoxyphenyl)-3-phenyl-2,3,3a,8b-tetrahydro-1H-cyclopenta[b]benzofuran-2-carboxylate (100 mg, 0,183 mmol) et NH₄OH 7N dans le MeOH (5 mL) et un peu de MeOH (= rinçage du matériel utilisé). Le mélange est placé sous atmosphère d'hydrogène et agité à température ambiante pendant 18 heures. Filtrer sur un acrodisc^{®} 0,45 µm puis bien rincer avec du méthanol, évaporer, un solide blanc est obtenu (m= 101 mg, rendement quantitatif).

Les composés obtenus par ces différents procédés sont caractérisés ci-dessous.

### Composé 1

### rac-(1R,2R,3S,3aR,8bS)-methyl 6-(2,3-dihydroxypropoxy)-1,8b-dihydroxy-8-methoxy-3a-(4-methoxyphenyl)-3-phenyl-2,3,3a,8b-tetrahydro-1H-cyclopenta[b]benzofuran-2-carboxylate

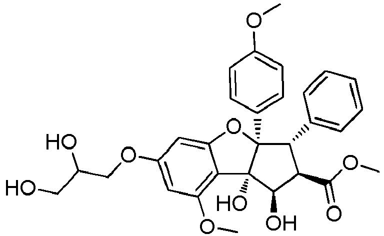

Solide blanc, 30,1 mg (31 %) ; obtenu à partir du rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant la procédure de substitution du phénol avec le prop-2-èn-1-ol comme alcool, puis la procédure de dihydroxylation.

RMN ¹H (CDCl₃, 400MHz, δ, ppm) : 7,10 (d, 2H, J = 8,6 Hz), 7,06 (m, 3H), 6,87 (m, 2H), 6,67 (d, 2H, J = 8,6 Hz), 6,29 (s, 1H), 6,15 (s, 1H), 5,02 (d, 1H, J = 6,5 Hz), 4,30 (d, 1H, J = 14,1 Hz), 4,11 (m, 1H), 4,05 (m, 2H), 3,90 (dd, 1H, J = 6,6 Hz, 14,1 Hz), 3,84dd, 1H, J = 3,6 Hz, 11,7 Hz), 3,78 (s, 3H), 3,73 (m, 1H), 3,70 (s, 3H), 3,64 (s, 3H) ; LCMS (ES+, m/z) : 534,93 [M-OH]⁺ ; LCMS (ES-, m/z) : 596,83 [M+HCOO⁻]⁻.

### Composé 2

### rac-(1R,2R,3S,3aR,8bS)-methyl 6-(2-aminoethoxy)-1,8b-dihydroxy-8-methoxy-3a-(4-methoxyphenyl)-3-phenyl-2,3,3a,8b-tetrahydro-1H-cyclopenta[b]benzofuran-2-carboxylate

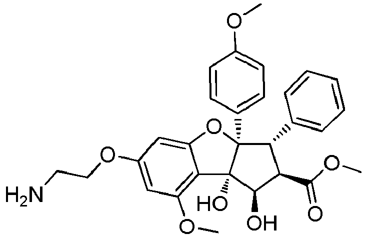

Solide blanc, 16 mg (66 %) ; obtenu à partir du rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant la procédure de substitution du phénol avec le 2-chloroéthan-1-ol comme alcool, puis les procédures de synthèse et de réduction de l'azoture.

RMN ¹H (CDCl₃, 400MHz, δ, ppm) : 7,12 (d, 2H, J = 8,7 Hz), 7,05 (m, 3H), 6,83 (m, 2H), 6,67 (d, 2H, J = 8,7 Hz), 6,20 (d, 1H, J = 1,6 Hz), 6,10 (d, 1H, J = 1,6 Hz), 5,02 (d, 1H, J = 6,7 Hz), 4,26 (d, 1H, J = 14,1 Hz), 3,84 (m, 6H), 3,71 (s, 3H), 3,63 (s, 3H), 2,87 (m, 2H) ; LCMS (ES+, m/z) : 503,97 [M-OH]⁺ ; LCMS (ES-, m/z) : 565,87 [M+HCOO⁻]⁻.

### Composé 3

### rac-(1R,2R,3S,3aR,8bS)-methyl 6-((4,5-dihydroxypentyl)oxy)-1,8b-dihydroxy-8-methoxy-3a-(4-methoxyphenyl)-3-phenyl-2,3,3a,8b-tetrahydro-1H-cyclopenta[b]benzofuran-2-carboxylate

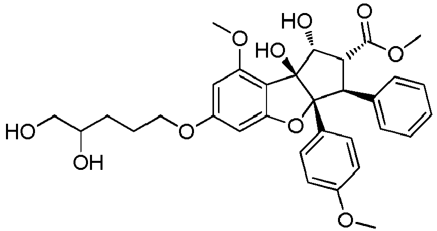

Solide blanc, obtenu à partir du rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b] benzofuran-2-carboxylate en suivant la procédure de substitution du phénol avec le pent-4-èn-1-ol comme alcool, puis la procédure de dihydroxylation.

RMN ¹H (CDCl₃, 400MHz, δ, ppm) : 7,05 (m, 5H), 6,84 (m, 2H), 6,63 (m, 2H), 6,23 (d, 1H, J = 1,4 Hz), 6,09 (d, 1H, J = 1,4 Hz), 4,99 (d, 1H, J = 6,7 Hz), 4,25 (d, 1H, J = 14,1 Hz), 3,96 (m, 2H), 3,86 (m, 1H), 3,82 (s, 3H), 3,65 (s, 3H), 3,62 (s, 3H), 3,51 (dd, 1H, J = 2,8 Hz, 11,2 Hz), 3,32 (dd, 1H, J = 7,6 Hz, 11,2 Hz), 1,84 (m, 2H), 1,51 (m, 2H) ; LCMS (ES+, m/z) : 563,0 [M-OH]⁺ ; LCMS (ES-, m/z) : 625,0 [M+HCOO⁻]⁻.

### Composé 4

### rac-(1R,2R,3S,3aR,8bS)-methyl 1,8b-dihydroxy-8-methoxy-3a-(4-methoxyphenyl)-6-(4-oxobutoxy)-3-phenyl-2,3,3a,8b-tetrahydro-1H-cyclopenta[b]benzofuran-2-carboxylate

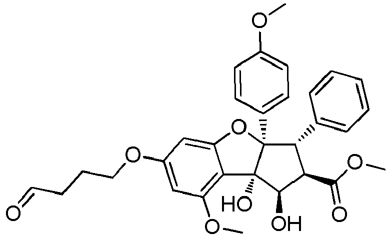

Solide blanc ; obtenu à partir du composé 3 en suivant la procédure de coupure oxydante.

RMN ¹H (CDCl₃, 400MHz, δ, ppm) : 9,80 (s, 1H), 7,06 (m, 5H), 6,85 (m, 2H), 6,64 (m, 2H), 6,23 (d, 1H, J = 1,7 Hz), 6,08 (d, 1H, J = 1,7 Hz), 5,00 (d, 1H, J = 6,7 Hz), 4,27 (d, 1H, J = 14,1 Hz), 3,99 (t, 2H, J = 6,1 Hz), 3,87 (dd, 1H, J = 6,7 Hz, 14,1 Hz), 3,84 (s, 3H), 3,67 (m, 1H), 3,66 (s, 3H), 3,62 (s, 3H), 2,64 (dd, 2H, J = 6,7 Hz, 7,2 Hz), 2,10 (m, 2H) ; LCMS (ES+, m/z) : 530,98 [M-OH]⁺ ; LCMS (ES-, m/z) : 592,97 [M+HCOO⁻]⁻.

### Composé 5

### rac-(1R,2R,3S,3aR,8bS)-methyl 1,8b-dihydroxy-6-(4-hydroxybutoxy)-8-methoxy-3a-(4-methoxyphenyl)-3-phenyl-2,3,3a,8b-tetrahydro-1H-cyclopenta[b]benzofuran-2-carboxylate

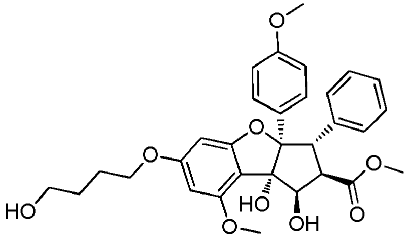

Mousse blanche, 1,72 g (64 %) ; obtenue à partir du **composé 4** en suivant la procédure de réduction.

RMN ¹H (DMSO-D₆, 400MHz, δ, ppm) : 7,02 (m, 5H), 6,87 (d, 1H, J = 7,4 Hz), 6,58 (d, 2H, J = 8,7 Hz), 6,26 (s, 1H), 6,09 (s, 1H), 5,07 (s, 1H), 5,02 (d, 1H, J = 5,0 Hz), 4,68 (t, 1H, J = 5,0 Hz), 4,47 (t, 1H, J = 5,0 Hz), 4,13 (d, 1H, J = 14,0 Hz), 4,00 (t, 2H, J = 6,5 Hz), 3,91 (dd, 1H, J = 5,0 Hz, 14,0 Hz), 3,73 (s, 3H), 3,60 (s, 3H), 3,54 (s, 3H), 3,47 (m, 2H), 1,75 (m, 2H), 1,58 (m, 2H) ; LCMS (ES+, m/z) : 532,97 [M-OH]⁺ ; LCMS (ES-, m/z) : 594,99 [M+HCOO⁻]⁻.

### Composé 6

### rac-(1R,2R,3S,3aR,8bS)-methyl 6-(4-((3-((4-((3-aminopropyl)amino)butyl)amino)propyl)amino)butoxy)-1,8b-dihydroxy-8-methoxy-3a-(4-methoxyphenyl)-3-phenyl-2,3,3a,8b-tetrahydro-1H-cyclopenta[b]benzofuran-2-carboxylate, chlorhydric acid salt

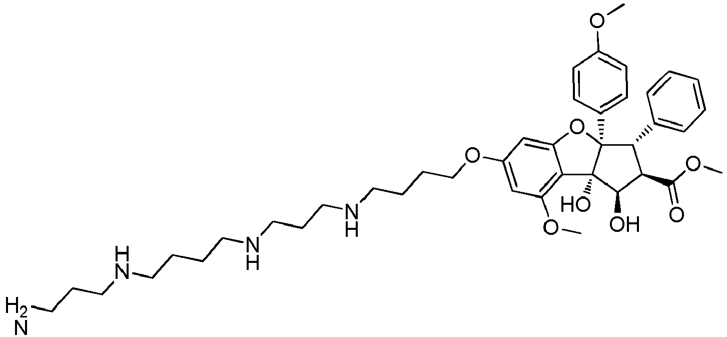

Solide blanc, 20 mg (66 %) ; obtenu à partir du **composé 4** en suivant la procédure d'amination réductrice en utilisant le tert-butyl (4-((3-aminopropyl)(tert-butoxycarbonyl) amino)butyl)(3-((tert-butoxycarbonyl)amino)propyl)carbamate comme amine. La déprotection finale est réalisée selon les conditions décrites dans [J. Org. Chem. 2006, 71, 9045-9050].

RMN ¹H (DMSO-D₆, 400MHz, δ, ppm) : 7.03 (m, 5H), 6.87 (m, 2H), 6.59 (m, 2H), 6.29 (s, 1H), 6.09 (s, 1H), 5,11 (m, 1H), 5.09 (s, 1H), 4,70 (m, 1H), 4.12 (d, 1H, J = 14.4 Hz), 4.05 (m, 2H), 3.94 (dd, 1H, J = 14.4 Hz, 5.8 Hz), 3.73 (s, 3H), 3.61 (s, 3H), 3.55 (s, 3H), 3.16 (m, 5H), 2.94 (m, 7H), 2.03-1.58 (m, 11H) ; HRMS : C41H59N408 [M+H]⁺ calc. 735,4327 found 735,4335.

### Composé 7

### rac-(1R,2R,3S,3aR,8bS)-methyl1,8b-dihydroxy-8-methoxy-6-(4-methoxy-4-oxobutoxy)-3a-(4-methoxyphenyl)-3-phenyl-2,3,3a,8b-tetrahydro-1H-cyclopenta[b]benzofuran-2-carboxylate

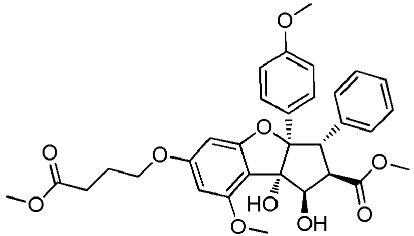

Solide blanc, 3,8 mg (7 %) ; obtenu à partir du **composé 4** en réalisant une oxydation comme suit : A un mélange du **composé 4** (1 équiv.) et de 2-méthyl-2-butène (4,5 équiv.) dans l'acétone (0,1 M) sont ajoutés une solution de NaH₂PO₄ (3 équiv, 0,5 M) dans l'eau puis du chlorite de sodium (3 équiv.), le milieu réactionnel est agité vigoureusement pendant 18 heures. Le mélange est dilué avec de l'eau et de l'acétate d'éthyle, le pH de la phase aqueuse est ajusté à 6 par addition de HCl (1N), la phase organique est alors extraite puis séchée sur sulfate de sodium, filtrée et concentrée pour donner l'acide carboxylique sous forme d'une mousse blanche. Ce composé est dissous dans un mélange de méthanol et de toluène 1/1 (0,05 M) puis du triméthylsilyldiazométhane est ajouté (4 équiv.), le milieu réactionnel est agité pendant 18 heures à température ambiante. Les solvants sont alors évaporés et le résidu est purifié sur gel de silice.

RMN ¹H (CDCl₃, 400MHz, δ, ppm) : 7,12 (d, 2H, J = 8,7 Hz), 7,08 (m, 3H), 6,89 (m, 2H), 6,70 (d, 2H, J = 8,7 Hz), 6,28 (d, 1H, J = 1,9 Hz), 6,14 (d, 1H, J = 1,9 Hz), 5,04 (d, 1H, J = 6,7 Hz), 4,32 (d, 1H, J = 14,1 Hz), 4,06 (t, 2H, J = 6,0 Hz), 3,92 (dd, 1H, J = 6,7 Hz, 14,1 Hz), 3,89 (s, 3H), 3,74 (s, 3H), 3,73 (s, 3H), 3,68 (m, 1H), 3,67 (s, 3H), 2,57 (m, 2H), 2,16 (m, 2H) ; LCMS (ES+, m/z) : 561,0 [M-OH]⁺ ; LCMS (ES-, m/z): 622,9

[M+HCOO⁻]⁻.

### Composé 8

### rac-(1R,2R,3S,3aR,8bS)-methyl6-(4-aminobutoxy)-1,8b-dihydroxy-8-methoxy-3a-(4-methoxyphenyl)-3-phenyl-2,3,3a,8b-tetrahydro-1H-cyclopenta[b]benzofuran-2-carboxylate

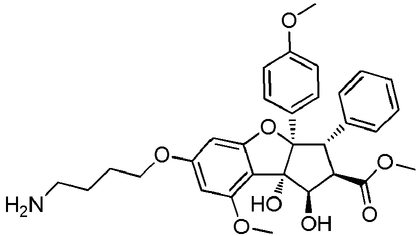

Solide blanc, 45 mg (7%) ; obtenu à partir du **composé 5** en suivant la procédure de synthèse de l'azoture puis de réduction de l'azoture, ce composé peut également être obtenu à partir du rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant la procédure de substitution du phénol avec le 4-chlorobutan-1-ol comme alcool, puis les procédures de synthèse et de réduction de l'azoture. Ce composé peut également être obtenu à partir du rac-(1R,2R,3S,3aR,8bS)-methyl 1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant la procédure de substitution du phénol avec le 4-hydroxybutanenitrile puis la procédure de réduction du nitrile.

RMN ¹H (DMSO-D₆, 400MHz, δ, ppm) : 7,02 (m, 5H), 6,87 (m, 2H), 6,59 (d, 2H, J = 8,9 Hz), 6,26 (d, 1H, J = 1,9 Hz), 6,10 (d, 1H, J = 1,9 Hz), 5,07 (bs, 1H), 5,01 (d, 1H, J = 4,4 Hz), 4,68 (m, 1H), 4,14 (d, 1H, J = 14,1 Hz), 3,99 (t, 2H, J = 6,5 Hz), 3,91 (dd, 5,7 Hz, 14,1 Hz), 3,73 (s, 3H), 3,60 (s, 3H), 3,54 (s, 3H), 2,60 (t, 2H, J = 6,5 Hz), 1,74 (m, 2H), 1,50 (m, 2H) ; LCMS (ES+, m/z) : 550,0 [M+H]⁺ ; LCMS (ES-, m/z) : 593,98

[M+HCOO⁻]⁻.

### Composé 50

### methyl (1R,2R,3S,3aR,8bS)-6-(4-aminobutoxy)-1,8b-dihydroxy-8-methoxy-3a-(4-methoxyphenyl)-3-phenyl-2,3,3a,8b-tetrahydro-1H-cyclopenta[b]benzofuran-2-carboxylate

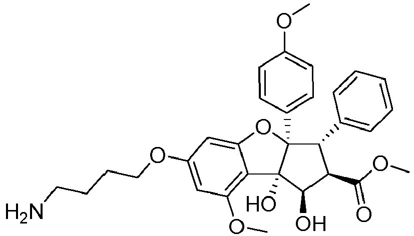

Solide beige, obtenu à partir du **composé 8** par séparation chirale par HPLC en utilisant une colonne Chiralpak^{®} IC 4.6^{∗}250mm DAICEL en utilisant comme éluant un mélange heptane / éthanol / BUA (butylamine) 50/50/0.05.

RMN et masse identique au **composé 8** ; e.e. 93 % ; [α]_{D}²⁰ =-54.0° (c 0.43 MeOH).

### Composé 51

### methyl (1S,2S,3R,3aS,8bR)-6-(4-aminobutoxy)-1,8b-dihydroxy-8-methoxy-3a-(4-methoxyphenyl)-3-phenyl-2,3,3a,8b-tetrahydro1H-cyclopenta[b]benzofuran-2-carboxylate

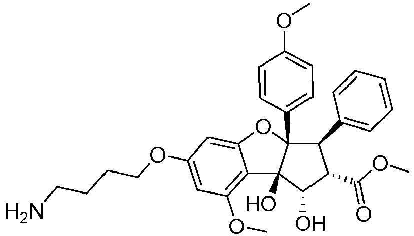

Solide beige, obtenu à partir du **composé 8** par séparation chirale par HPLC en utilisant une colonne Chiralpak^{®}IC 4.6^{∗}250mm DAICEL en utilisant comme éluent un mélange heptane / éthanol / BUA 50/50/0.05.

RMN et masse identique au **composé 8** ; e.e. 99 % ; [α]_{D}²⁰ =+61.5° (c 0.47 MeOH).

### Composé 52

### rac-methyl (1R,2R,3S,3aR,8bS)-6-(4-(dimethylamino)butoxy)-1,8b-dihydroxy-8-methoxy-3a-(4-methoxyphenyl)-3-phenyl-2,3,3a,8b-tetrahydro-1H-cyclopenta[b]benzofuran-2-carboxylate

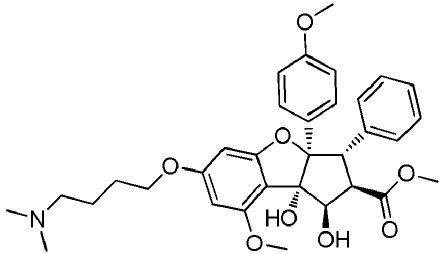

Solide blanc, m= 40,5 mg (38 %) ; obtenu à partir du composé chloré selon la procédure suivante dans un pilulier, mélanger rac-(1R,2R,3S,3aR,8bS)-methyl 6-(4-chlorobutoxy)-l,8b-dihydroxy-8-methoxy-3a-(4-methoxyphenyl)-3-phenyl-2,3,3a,8b-tetrahydro-1H-cyclopenta[b]benzofuran-2-carboxylate (100 mg, 0,176 mmol) dans le DMF (1,5 mL) puis y ajouter de la diméthylamine à 40 % dans l'eau (501 µl, 3,95 mmol). Chauffer à 90°C pendant 1 heure. Evaporer à sec. Une huile jaune est obtenue. Purifier sur gel de silice en utilisant comme éluant un mélange CH₂Cl₂/MeOH 90/10.

RMN ¹H (DMSO-D₆, 400MHz, δ, ppm) : 7.01 (m, 5H), 6.86 (m, 2H), 6.58 (m, 2H), 6.28 (m, 1H), 6.10 (m, 1H), 5.07 (s, 1H), 5.045 (m, 1H), 4.68 (m, 1H), 4.13 (m, 1H), 4.02 (m, 2H), 3.91 (m, 1H), 3.73 (s, 3H), 3.59 (s, 3H), 3.53 (s, 3H), 2.96 (m, 2H), 2.64 (s, 6H), 1.76 (m, 4H) ; LCMS (ES+, m/z) : 578,05 [M+H]⁺ ; LCMS (ES-, m/z) : 622,01

[M+HCOO⁻]⁻.

### Composé 53

### rac-(1R,2R,3S,3aR,8bS)-6-(4-aminobutoxy)-1,8b-dihydroxy-8-methoxy-3a-(4-methoxyphenyl)-3-phenyl-2,3,3a,8b-tetrahydro-1H-cyclopenta[b]benzofuran-2-carboxylic acid

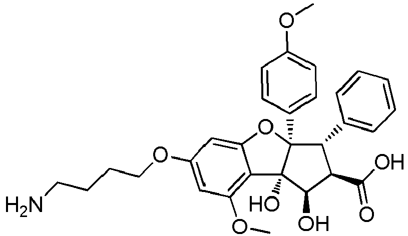

Solide blanc, 366 mg (88%) ; obtenu à partir du **composé 8** : dans un flacon de 20 ml, dissoudre le **composé 8** (500 mg, 0.774 mmol) et K₂CO₃ (535 mg, 3.87 mmol) dans du méthanol (5.00 mL) et de l'eau (5 mL). Laisser agiter à 70°C pendant 4 h 30, après retour à température ambiante, le brut est évaporé puis le résidu est purifié sur silice en utilisant comme éluant un mélange CH₂Cl₂/MeOH/Eau : 50/50/4.

RMN ¹H (DMSO-D₆, 400MHz, δ, ppm) : 7.02 (m, 5H), 6.89 (m, 2H), 6.58 (m, 2H), 6.28 (m, 1H), 6.07 (m, 1H), 4.98 (m, 2H), 4.65 (m, 1H), 4.09 (m, 1H), 4.02 (m, 2H), 3.73 (s, 3H), 3.59 (s, 3H), 2.88 (m, 2H), 1.74 (m, 4H) ; LCMS (ES+, m/z) : 535,97 [M+H]⁺ ; LCMS (ES-, m/z) : 533,87 [M-H]⁻.

### Composé 54

### rac-(1R,2R,3S,3aR,8bS)-1,8b-dihydroxy-6-(4-hydroxybutoxy)-8-methoxy-3a-(4-methoxyphenyl)-3-phenyl-2,3,3a,8b-tetrahydro-1H-cyclopenta[b]benzofuran-2-carboxamide

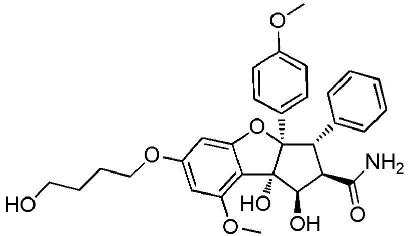

Solide blanc, 475 mg (99 %) ; obtenu à partir du **composé 5** en suivant la procédure de synthèse de l'hydrazide (*vide infra*) puis l'hydrazide obtenu (490 mg, 0,890 mmol) est réduit par addition à une suspension de nickel de Raney (313 mg, 2,67 mmol) dans le DMF (3 mL) et l'eau (3 mL). Le mélange obtenu est chauffé à 100°C pendant 1 heure 30. Après retour à température ambiante, le nickel est éliminé par filtration (AcOEt, CH₂Cl₂, MeOH) et le filtrat est concentré sous pression réduite. Le résidu est purifié sur gel de silice en utilisant comme éluant un mélange CH₂Cl₂/MeOH 90/10.

RMN ¹H (DMSO-D₆, 400MHz, δ, ppm) : 7,66 (s, 1H), 6,99 (m, 8H), 6,58 (m, 2H), 6,24 (s, 1H), 6,08 (s, 1H), 4,95 (s, 1H), 4,64 (d, 1H, J = 3,2 Hz), 4,54 (m, 1H), 4,69 (t, 1H, J = 5,3 Hz), 4,14 (d, 1H, J = 14,0 Hz), 3,99 (m, 1H), 3,78 (dd, 1H, J = 5,3 Hz, 14,0 Hz), 3,72 (s, 3H), 3,59 (s, 3H), 3,46 (m, 2H), 1,75 (m, 2H), 1,57 (m, 2H) ; LCMS (ES+, m/z) : 536,03 [M+H]⁺.

### Composé 55

### rac-(1R,2R,3S,3aR,8bS)-6-(4-aminobutoxy)-1,8b-dihydroxy-8-methoxy-3a-(4-methoxyphenyl)-3-phenyl-2,3,3a,8b-tetrahydro-1H-cyclopenta[b]benzofuran-2-carboxamide formate

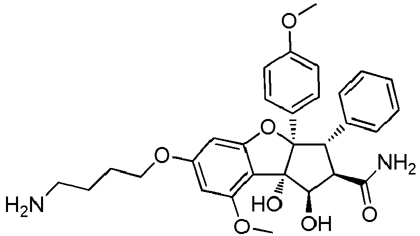

Solide blanc, 105 mg (42 %) : obtenu à partir du **composé 54** en suivant la procédure de synthèse de l'azoture à partir de l'alcool puis la procédure de réduction de l'azoture.

RMN ¹H (DMSO-D⁶, 400MHz, δ, ppm) : 8,42 (s, 1H), 7,69 (s, 1H), 7,04 (m, 3H), 6,97 (m, 3H), 6,59 (d, 2H, J = 8,9 Hz), 6,27 (d, 1H, J = 1,9 Hz), 6,09 (d, 1H, J = 1,9 Hz), 4,92 (bs, 1H), 4,66 (bs, 1H), 4,56 (d, 1H, J = 5,5 Hz), 4,15 (d, 1h, J = 14,3 Hz), 4,01 (t, 2H, J = 6,0 Hz), 3,80 (dd, 1H, J = 6,0 Hz, 14,3 Hz), 3,73 (s, 3H), 3,61 (s, 3H), 2,82 (t, 2H, J = 7,5 Hz), 1,77 (m, 2H), 1,68 (m, 2H) ; LCMS (ES+, m/z) : 535,02 [M+H]⁺ ; LCMS (ES-, m/z) : 579,02 [M+HCOO⁻]⁻.

### 1.2. Composés avec R₁ = NR'R" etR₂ = OH

Les composés selon l'invention peuvent être synthétisés selon le schéma réactionnel suivant : 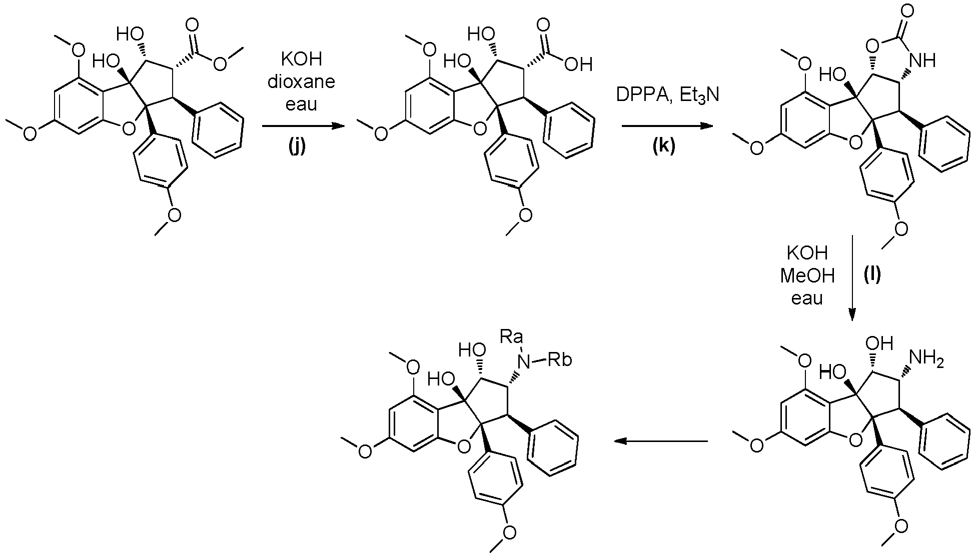
**(j) Procédure de saponification** : Dans un ballon de 250 mL, introduire rac-(1R,2R,3S,3aR,8bS)-methyl 1,8b-dihydroxy-6,8-dimethoxy-3a-(4-methoxyphenyl)-3-phenyl-2,3,3a,8b-tetrahydro-1H-cyclopenta[b]benzofuran-2-carboxylate (1,93 g, 3,92 mmol) dans 24 mL de 1,4-dioxane. Préparer une solution d'hydroxyde de potassium (0,930 g, 16,58 mmol) dans l'eau (3 mL) et l'additionner à 0°C au milieu réactionnel. Laisser revenir à température ambiante et agiter à 60°C durant 4 heures. Abaisser la température du milieu à 0°C puis ajouter de l'eau (5 mL) et acidifier le milieu avec une solution HCL 1N jusqu'à atteindre un pH de 5. Extraire le produit à l'acétate d'éthyle (deux fois). Sécher la phase organique sur Na₂SO₄, filtrer et concentrer le milieu. L'acide est obtenu avec un rendement quantitatif.
**(k) Procédure pour le réarrangement de Curtius** : Dans un pilulier et sous azote, introduire l'acide carboxylique (200 mg, 0,418 mmol) dans 10 mL de toluène. Ajouter, à TA, l'azoture de diphénylphosphoryle (162 µl, 0,752 mmol) puis la triéthylamine (58,1 µl, 0,418 mmol). Agiter à 80°C durant 7h puis agiter à TA la nuit. Concentrer le toluène. Reprendre le solide dans de l'eau puis rincer à l'acétate d'éthyle. Le solide n'est pas pur. Reprendre le solide dans du méthanol, le passer aux ultrasons et filtrer. Un solide blanc est récupéré. Dans le cas où le produit ne précipite pas, le résidu est purifié sur gel de silice.
**(1) Procédure d'hydrolyse de l'oxazolidinone** : Dans un ballon, introduire rac-(3aR,4R,4aR,9bS,9cR)-9b-hydroxy-7,9-dimethoxy-4a-(4-methoxyphenyl)-4-phenyl-3, 3a,4,4a,9b,9c-hexahydro-2H-benzofuro[3',2':3,4]cyclopenta[1,2-d]oxazol-2-one (1,45g, 3,05 mmol) dans 6 mL de méthanol. Ajouter KOH à température ambiante (1,027 g, 18,30 mmol) dissous dans l'eau (3 mL). Le milieu est hétérogène (solide blanc en suspension). Le milieu réactionnel est agité à 75°C pendant 72 heures. Evaporer le méthanol puis ajuster le pH à 8 avec une solution de NH₄Cl saturée. Un solide marron est toujours en suspension. Le filtrer et le rincer à l'eau puis à l'éther diéthylique, enfin le sécher sous vide pour obtenir l'amine avec un rendement de 93 %.

Les composés obtenus par ces différents procédés sont caractérisés ci-dessous.

### Composé 9

### rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol

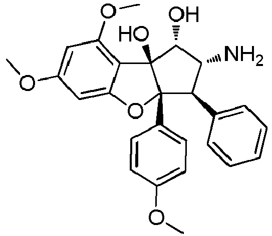

Solide blanc, 44 mg (93 %) ; obtenu à partir du **composé 40** en suivant la procédure d'hydrolyse de l'oxazolidinone.

RMN ¹H (DMSO-D₆, 400MHz, δ, ppm) : 7,04 (m, 7H), 6,58 (d, 2H, J = 8,8 Hz), 6,21 (d, 1H, J = 1,8Hz), 6,08 (d, 1H, J = 1,8Hz), 4,82 (s, 1H), 4,16 (d, 1H J = 4,7 Hz), 4,02 (dd, 1H, J = 4,7 Hz, 13,0 Hz), 3,76 (s, 3H), 3,74 (s, 3H), 3,60 (s, 3H), 3,54 (d, 1H J= 13,0 Hz) ; LCMS (ES+, m/z): 450,0 [M+H]⁺ ; LCMS (ES-, m/z) : 493,9 [M+HCOO⁻]⁻.

### Composé 10

### rac-N-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)formamide

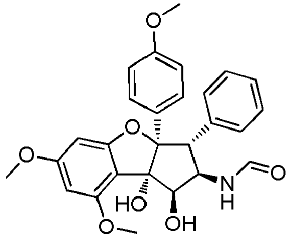

Solide jaune pâle, 134 mg (84 %) ; obtenu à partir du **composé 9** comme suit : Dans un ballon et sous azote, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (50 mg, 0,111 mmol) dans 1 mL de THF, ajouter Ethyl formate (135 µl, 1,669 mmol) et une goutte d'acide acétique. Agiter à reflux durant 18 heures. Les solvants sont évaporés, le solide obtenu est repris dans de l'éthanol. Un solide précipite, le filtrer et le rincer à l'éthanol. Après séchage sous vide un solide jaune clair est récupéré.

RMN ¹H (DMSO-D₆, 400MHz, δ, ppm) : 8,00 (d, 1H, J = 9,4 Hz), 7,96 (d, 1H, J = 1,4 Hz), 7,01 (m, 7H), 6,60 (d, 2H, J = 6,6 Hz), 6,25 (d, 1H, J = 1,9 Hz), 6,10 (d, 1H, J = 1,9 Hz), 5,19 (m, 1H), 5,10 (d, 1H, J = 4,9 Hz), 5,04 (s, 1H), 4,27 (t, 1H, J = 4,9 Hz), 3,93 (d, 1H, J = 13,9 Hz), 3,77 (s, 3H), 3,73 (s, 3H), 3,60 (s, 3H) ; LCMS (ES+, m/z) : 460,0 [M-OH]⁺ ; LCMS (ES-, m/z) : 522,1 [M+HCOO⁻]⁻.

### Composé 11

### rac-1-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)urea

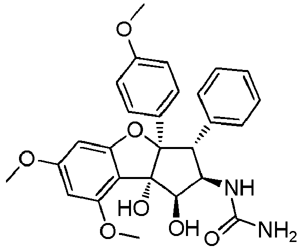

Solide blanc, 22 mg (50 %) ; obtenu à partir du **composé 9** comme suit : Dans un ballon et sous azote, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (40 mg, 0,089 mmol) dans un mélange de dichlorométhane et propan-2-ol. Ajouter, à température ambiante, Trimethylsilyl isocyanate (9,04 µl, 0,067 mmol). Le milieu est limpide. Agiter à température ambiante pendant 4 heures. Concentrer à sec, reprendre le solide dans un minimum de volume d'éthanol : filtrer le solide en suspension. Après séchage, un solide blanc est récupéré.

RMN ¹H (DMSO-D₆, 400MHz, δ, ppm) : 7,03 (m, 7H), 6,59 (d, 2H, J = 8,8 Hz), 6,24 (d, 1H, J = 1,7 Hz), 6,09 (d, 1H, J = 1,7 Hz), 5,83 (d, 1H, J = 9,5 Hz), 5,53 (s, 2H), 5,04 (m, 2H), 4,93 (s, 1H), 4,18 (m, 1H), 3,81 (m, 1H), 3,77 (s, 3H), 3,73 (s, 3H), 3,59 (s, 3H) ; LCMS (ES+, m/z): 492,9 [M+H]⁺ ; LCMS (ES-, m/z) : 536,9 [M+HCOO⁻]⁻.

### Composé 12

### rac-N-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)methanesulfonamide

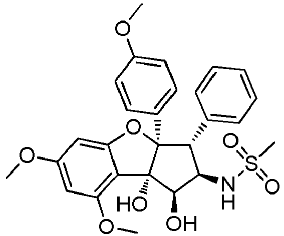

Solide jaune pâle, 1,8 mg (3 %) ; obtenu à partir du **composé 9** comme suit : Dans un ballon et sous azote, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (50 mg, 0,111 mmol) dans 1,05 mL de dichloromethane, ajouter, à 0°C, un mélange de *N*,*N*-diisopropyléthylamine (DIEA, 29,1 µl, 0,167 mmol) et Methanesulfonyl chloride (10,33 µl, 0,133 mmol). Agiter à température ambiante durant 2h. Ajouter 3 mL de HCl 1N. Extraire la phase organique puis la sécher sur MgSO₄, filtrer et concentrer le milieu. Purifier le résidu sur silice en utilisant comme éluant un mélange DCM/AcOEt 100/0 à 90/10.

RMN ¹H (DMSO-D₆, 400MHz, δ, ppm) : 7.04 (m, 8H), 6.62 (m, 2H), 6.25 (m, 1H), 6.12 (m, 1H), 5.14 (m, 1H), 5.08 (s, 1H), 4.56 (m, 1H), 4.45 (m, 1H), 3.80 (m, 1H), 3.78 (s, 3H), 3.76 (s, 3H), 3.61 (s, 3H), 3.00 (s, 3H) ; LCMS (ES+, m/z) : 509,9 [M-OH]⁺ ; LCMS (ES-, m/z): 525,9 [M-H]⁻.

### Composé 13

### rac-1-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)-3-methylthiourea

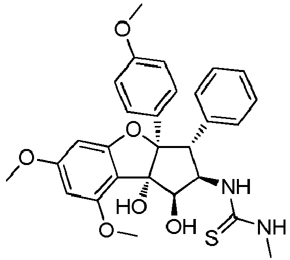

Solide beige, 33 mg (58 %) ; obtenu à partir du **composé 9** comme suit : Dans un ballon, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (50 mg, 0,111 mmol) en solution dans le tétrahydrofurane (2 mL). Ajouter l'isothiocyanatométhane (9,76 mg, 0,133 mmol) et laisser agiter à température ambiante pendant 2 heures. Concentrer le milieu réactionnel puis ajouter de l'éther diéthylique. Une précipitation est observée. Filtrer le solide et le rincer à l'éther diéthylique.

RMN ¹H (DMSO-D₆, 400MHz, δ, ppm) : 7.06 (m, 9H), 6.25 (m, 1H), 6.09 (m, 1H), 5.04 (m, 1H), 4.92 (s, 1H), 4,34 (m, 1H), 3.77 (s, 3H), 3.72 (s, 3H), 3.60 (s, 3H), 2.53 (s, 3H) ; LCMS (ES+, m/z) : 522,97 [M+H]⁺ ; LCMS (ES-, m/z) : 521,0 [M-H]⁻.

### Composé 14

### rac-1-(3-(diethylamino)propyl)-3-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)thiourea

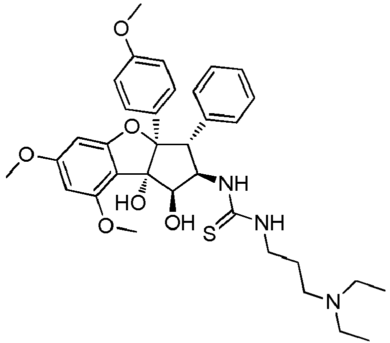

Solide brun, 3,9 mg (5 %) ; obtenu à partir du **composé 9** en suivant un mode opératoire identique à celui permettant de synthétiser le **composé 13** en utilisant la N,N-diéthyl-3-isothiocyanatopropan-1-amine à la place de l'isothiocyanatométhane.

LCMS (ES+, m/z): 622,1 [M+H]⁺ ; LCMS (ES-, m/z): 620,0 [M-H]⁻.

### Composé 15

### rac-(1R,2R,3R,3aR,8bS)-6,8-dimethoxy-3a-(4-methoxyphenyl)-2-(methylamino)-3-phenyl-1,2,3,3a-tetrahydro-8bH-cyclopenta[b]benzofuran-1,8b-diol

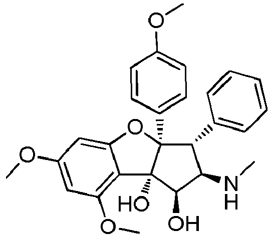

Solide brun, 3 mg (7 %) ; obtenu à partir du **composé 40** comme suit : dans un ballon et sous azote, introduire rac-(3aR,4R,4aR,9bS,9cR)-9b-hydroxy-7,9-dimethoxy-4a-(4-methoxyphenyl)-4-phenyl-3,3a,4,4a,9b,9c-hexahydro-2H-benzofuro[3',2' :3,4] cyclopenta[1,2-d]oxazol-2-one (43,4 mg, 0,091 mmol) dans 1 mL de THF, ajouter, à 0°C, Lithium aluminum hydride (15,31 µl, 0,365 mmol) et chauffer à 80°C pendant 3 heures. Ajouter à 0°C 30 µL d'eau. Agiter à température ambiante 5 minutes. Ajouter ensuite 30 µL NaOH à 10 % puis 100 µL d'eau. Agiter à température ambiante 5 min. Filtrer sur Dicalite^{®} et rincer au THF et à l'acétate d'éthyle, concentrer le filtrat. Le résidu est purifié sur gel de silice en utilisant comme éluant un mélange DCM/MeOH 100/0 à 80/20.

RMN ¹H (CD₃OD, 400MHz, δ, ppm) : 7.14 (m, 2H), 7.05 (m, 3H), 6.93 (m, 2H), 6.63 (m, 2H), 6.23 (m, 1h), 6.16 (m, 1H), 4.65 (d, 1H), 3.85 (s, 3H), 3.83 (m, 1H), 3.81 (s, 3H), 3.67 (s, 3H), 3.63 (m, 1H), 2.46 (s, 3H) ; LCMS (ES+, m/z) : 464,0 [M+H]⁺ ; LCMS (ES-, m/z) : 507,9 [M+HCOO⁻]⁻.

### Composé 16

### rac-N-((1R,2R,3R,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-yl)acetamide

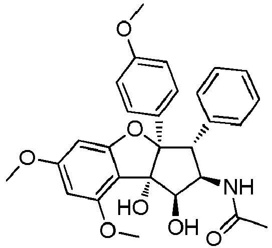

Solide beige, 12 mg (23 %) ; obtenu à partir du **composé 9** comme suit : Dans un ballon, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (50 mg, 0,111 mmol) en solution dans le tétrahydrofurane (500 µl). Refroidir à 0°C et ajouter DIEA (38,9 µl, 0,222 mmol) et acetyl chloride (8,73 mg, 0,111 mmol), puis laisser remonter à température ambiante et agiter pendant 4 heures. Ajouter de l'eau et de l'acétate d'éthyle, rassembler les phases organiques et laver par une solution saturée de NaCl, puis à l'eau, sécher la phase organique sur Na₂SO₄ et concentrer. Triturer le brut dans l'éther diéthylique et filtrer le solide.

LCMS (ES+, m/z) : 491,9 [M+H]⁺ ; LCMS (ES-, m/z) : 535,9 [M+HCOO⁻]⁻.

### Composé 17

### rac-(1R,2R,3R,3aR,8bS)-2-amino-6-(4-aminobutoxy)-8-methoxy-3a-(4-methoxyphenyl)-3-phenyl-2,3,3a,8b-tetrahydro-1H-cyclopenta[b]benzofuran-1,8b-diol, di-formic acid salt

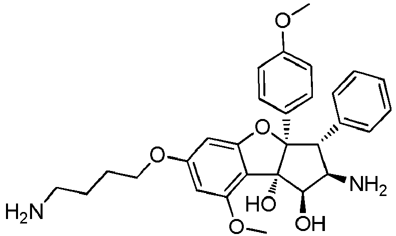

Solide blanc, 1,2 mg (3 %) ; obtenu à partir du **composé 43** en suivant les procédures de réduction de l'azoture puis d'hydrolyse de l'oxazolidinone.

RMN ¹H (CD₃OD, 400MHz, δ, ppm) : 8,51 (bs, 2H), 7,12 (m, 5H), 6,90 (m, 2H), 6,68 (m, 2H, J = 8,7 Hz), 6,28 (s, 1H), 6,20 (s, 1H), 4,74 (d, 1H, J = 5,9 Hz), 4,39 (dd, 1H, J = 5,9 Hz, 13,7 Hz), 4,06 (m, 2H), 3,89 (d, 1H, J = 13,7 Hz), 3,87 (s, 3H), 3,69 (s, 3H), 3,02 (m, 2H), 1,88 (m, 4H) ; LCMS (ES+, m/z) : 506,89 [M+H]⁺ ; LCMS (ES-, m/z) : 550,84 [M+HCOO⁻]⁻.

### 1.3. Composés avec R₁ = Hétérocycle et R₂ = OH

Les composés selon l'invention peuvent être synthétisés selon le schéma réactionnel suivant : 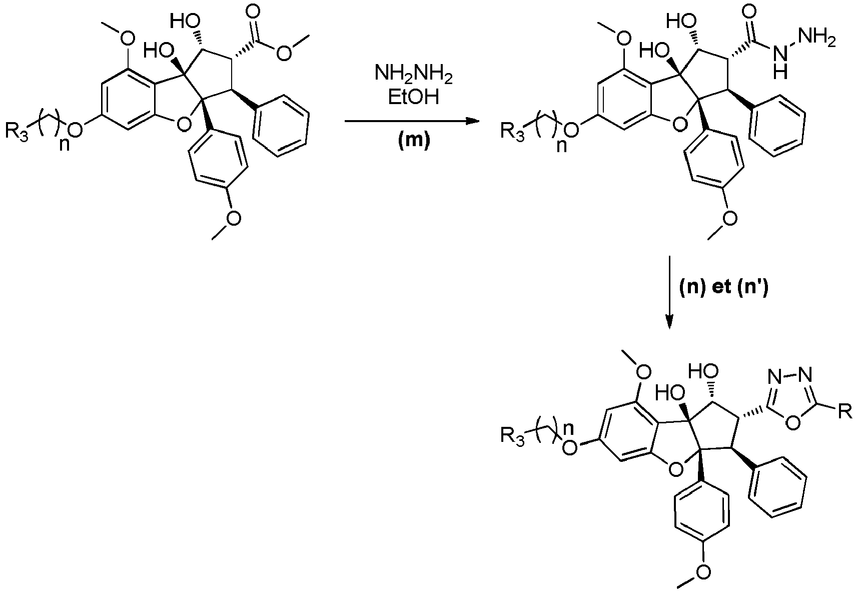
**(m) Procédure de synthèse de l'hydrazide** (exemple n = 1, R₃ = H) : Dans un tube scellé, introduire rac-(1R,2R,3S,3aR,8bS)-methyl 1,8b-dihydroxy-6,8-dimethoxy-3a-(4-methoxyphenyl)-3-phenyl-2,3,3a,8b-tetrahydro-1H-cyclopenta[b]benzofuran-2-carboxylate (2g, 4,06 mmol) dans 7 mL d'éthanol. Ajouter, à 0°C, l'hydrazine hydrate (1,275 ml, 40,6 mmol) puis agiter à 80°C pendant 24 heures. Concentrer le milieu à sec puis reprendre le solide dans de l'éthanol. Filtrer le solide, le rincer avec de l'éthanol froid puis le sécher.
**(n) Procédure de synthèse de l'oxadiazole** (exemple n = 1, R₃ = H, R = NH₂) : Dans un ballon, introduire rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2 carbohydrazide (200 mg, 0,406 mmol) dans 700 µl de 1,4-dioxane. Ajouter, à température ambiante, NaHCO₃ (34,1 mg, 0,406 mmol) et de l'eau (70 µl). A 0°C, ajouter du bromure de cyanogène (162 µl, 0,487 mmol) puis à température ambiante durant 5 heures. Après avoir concentré le milieu, ajouter de l'eau, passer aux ultrasons, un solide est en suspension. Solubiliser le solide dans l'acétate d'éthyle. Extraire la phase organique et la laver avec une solution de NaHCO₃ saturée. Sécher la phase organique sur Na₂SO₄, filtrer puis concentrer. Purifier le résidu sur gel de silice en utilisant comme éluant DCM/MeOH 100/0 à 95/5.
**(n') Procédure de synthèse des amino-oxadiazole subtitués** (exemple n = 1, R₃ = H, R = NHMe) : A une solution de rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b] benzofuran-2carbohydrazide (40 mg, 0,081 mmol) dans le tétrahydrofurane (1,5 mL) est ajouté l'isothiocyanatométhane (7,54 mg, 0,103 mmol) puis le chlorhydrate de 1-(3-diméthylaminopropyl)-3-éthylcarbodiimide (15,57 mg, 0,081 mmol) à 0°C. Le mélange est agité à 80°C pendant 18 heures. Le milieu réactionnel est concentré et le résidu obtenu est purifié sur gel de silice en utilisant comme éluant un mélange DCM/MeOH 100/0 à 97/3.

### Composé 18

### rac-(lR,2R,3S,3aR,8bS)-2-(5-amino-1,3,4-oxadiazol-2-yl)-6,8-dimethoxy-3a-(4-methoxyphenyl)-3-phenyl-2,3,3a,8b-tetrahydro-1H-cyclopenta[b]benzofuran-1,8b-diol

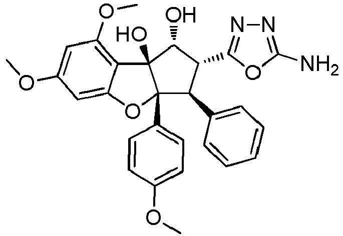

Solide blanc, 99 mg (47 %) ; obtenu à partir du composé rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-carbohydrazide en suivant la procédure de synthèse de l'oxadiazole.

RMN ¹H (DMSO-D₆, 400MHz, δ, ppm) : 7,02 (m, 5H), 6,86 (m, 2H), 6,73 (s, 2H), 6,60 (d, 2H, J = 8,9 Hz), 6,28 (d, 1H, J = 1,8 Hz), 6,11 (d, 1H, J = 1,8 Hz), 5,16 (s, 1H), 4,99 (d, 1H, J = 4,4 Hz), 4,61 (m, 1H), 4,26 (m, 2H), 3,78 (s, 3H), 3,72 (s, 3H), 3,60 (s, 3H) ; LCMS (ES+, m/z) : 518,0 [M+H]⁺ ; LCMS (ES-, m/z) : 516,0 [M-H]⁻.

### Composé 19

### (1R,2R,3S,3aR,8bS)-2-(5-amino-1,3,4-oxadiazol-2-yl)-6,8-dimethoxy-3a-(4-methoxyphenyl)-3-phenyl-2,3,3a,8b-tetrahydro-1H-cyclopenta[b]benzofuran-1,8b-diol

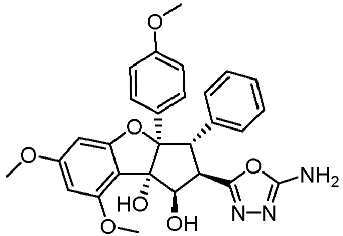

Solide blanc, 205 mg( 41 %) ; obtenu à partir du **composé 18** par séparation chirale sur HPLC en utilisant une colonne Chiralpak^{®} IC 4.6^{∗}250mm DAICEL et comme éluant un mélange Heptane/Ethanol/Methanol/Diéthylamine 80/10/10/0.05

LCMS (ES+, m/z) : 518,0 [M+H]⁺ ; LCMS (ES-, m/z) : 516,0 [M-H]⁻ e.e. 96,7 % ; [α]_{D}²⁰ = -27,7 (c 0,59 MeOH)

### Composé 20

### rac-(1R,2R,3S,3aR,8bS)-2-(5-amino-1,3,4-oxadiazol-2-yl)-6-(2-hydroxyethoxy)-8-methoxy-3a-(4-methoxyphenyl)-3-phenyl-2,3,3a,8b-tetrahydro-1H-cyclopenta[b] benzofuran-1,8b-diol

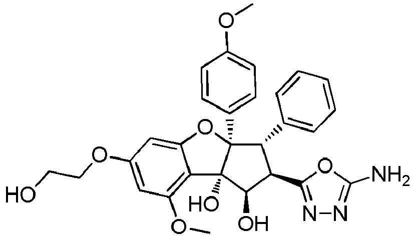

Solide blanc, 51 mg (51 %) ; obtenu à partir du composé rac-methyl (1R,2R,3S,3aR,8bS)-1,6,8b-trihydroxy-8-methoxy-3a-(4-methoxyphenyl)-3-phenyl-2,3, 3a,8b-tetrahydro-1H-cyclopenta[b]benzofuran-2-carboxylate en suivant les procédures de substitution du phénol avec du prop-2-èn-1-ol, de synthèse de l'hydrazide, de formation de l'oxadiazole, de dihydroxylation, de coupure oxydante et enfin de réduction de l'aldéhyde.

RMN ¹H (DMSO-D₆, 400MHz, δ, ppm) : 7,03 (m, 5H), 6,87 (m, 2H), 6,74 (s, 2H), 6,61 (d, 2H, J = 8,8 Hz), 6,28 (m, 1H), 6,12 (m, 1H), 5,17 (s, 1H), 5,01 (d, 1H, J = 4,4 Hz), 4,88 (t, 1H, J = 5,5 Hz), 4,61 (m, 1H), 4,27 (m, 2H), 4,01 (m, 2H), 3,73 (m, 2H+3H), 3,61 (s, 3H) ; LCMS (ES+, m/z) : 548 [M+H]⁺ ; LCMS (ES-, m/z) : 592 [M+HCOO⁻]⁻.

### Composé 21

### rac-(1R,2R,3S,3aR,8bS)-2-(5-amino-1,3,4-oxadiazol-2-yl)-6-(2-(dimethylamino)ethoxy)-8-methoxy-3a-(4-methoxyphenyl)-3-phenyl-2,3,3a,8b-tetrahydro-1H-cyclopenta[b]benzofuran-1,8b-diol

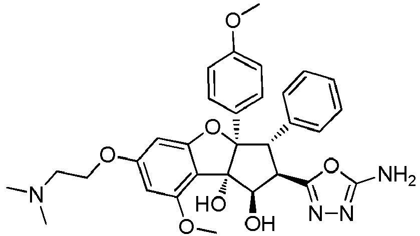

Solide blanc ; obtenu à partir du composé rac-methyl (1R,2R,3S,3aR,8bS)-1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant les procédures de substitution du phénol avec du prop-2-èn-1-ol, de synthèse de l'hydrazide, de formation de l'oxadiazole, de dihydroxylation, de coupure oxydante et enfin d'amination réductrice en utilisant la diméthylamine.

RMN ¹H (DMSO-D₆, 400MHz, δ, ppm) : 7,03 (m, 5H), 6,87 (m, 2H), 6,74 (s, 2H), 6,61 (d, 2H, J = 8,9 Hz), 6,29 (m, 1H), 6,11 (m, 1H), 5,17 (s, 1H), 5,00 (d, 1H, J = 4,3 Hz), 4,61 (m, 1H), 4,27 (s, 2H), 3,73 (s, 3H), 3,61 (s, 3H), 2,63 (m, 2H), 2,23 (s, 6H) ; LCMS (ES+, m/z) : 575,01 [M+H]⁺ ; LCMS (ES-, m/z) : 572,94 [M-H]⁻.

### Composé 22

### rac-(1R,2R,3S,3aR,8bS)-2-(5-amino-1,3,4-oxadiazol-2-yl)-8-methoxy-3a-(4-methoxyphenyl)-6-(2-(4-methylpiperazin-1-yl)ethoxy)-3-phenyl-2,3,3a,8b-tetrahydro-1H-cyclopenta[b]benzofuran-1,8b-diol

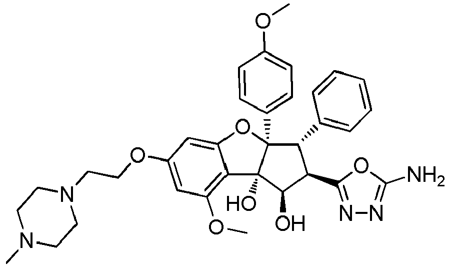

Solide blanc, 56 mg (24 %) ; obtenu à partir du composé rac-methyl (1R,2R, 3S,3aR,8bS)-1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant les procédures de substitution du phénol avec du prop-2-èn-1-ol, de synthèse de l'hydrazide, de formation de l'oxadiazole, de dihydroxylation, de coupure oxydante et enfin d'amination réductrice en utilisant la 1-méthylpipérazine.

RMN ¹H (DMSO-D₆, 400MHz, δ, ppm) : 7.03 (m, 5H), 6.87 (m, 2H), 6.74 (s, 2H), 6.61 (m, 2H), 6.29 (s, 1H), 6.12 (s, 1H), 5.17 (s, 1H), 5.01 (m, 1H), 4.61 (m, 1H), 4.26 (s, 2H), 4.10 (m, 2H), 3.73 (s, 3H), 3.61 (s, 3H), 2.50 (m, 8H), 2.16 (s, 3H); LCMS (ES+, m/z) : 630,07 [M+H]⁺ ; LCMS (ES-, m/z) : 674,00 [M+HCOO⁻]⁻.

### Composé 23

### rac-(1R,2R,3S,3aR,8bS)-2-(5-amino-1,3,4-oxadiazol-2-yl)-8-methoxy-3a-(4-methoxyphenyl)-6-(2-morpholinoethoxy)-3-phenyl-2,3,3a,8b-tetrahydro-1H-cyclopenta[b]benzofuran-1,8bdiol

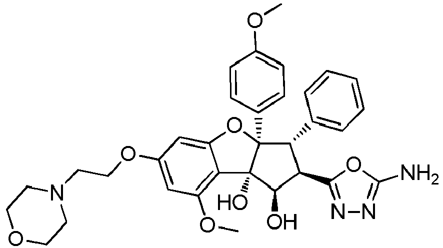

Solide blanc, 60 mg (26 %) ; obtenu à partir du composé rac-methyl (1R,2R, 3S,3aR,8bS)-1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant les procédures de substitution du phénol avec du prop-2-èn-1-ol, de synthèse de l'hydrazide, de formation de l'oxadiazole, de dihydroxylation, de coupure oxydante et enfin d'amination réductrice en utilisant la morpholine.

RMN ¹H (DMSO-D₆, 400MHz, δ, ppm) : 7.03 (m, 5H), 6.87 (m, 2H), 6.74 (s, 2H), 6.61 (m, 2H), 6.29 (s, 1H), 6.12 (s, 1H), 5.17 (s, 1H), 5.01 (m, 1H), 4.61 (m, 1H), 4.26 (s, 2H), 4.12 (m, 2H), 3.73 (s, 3H), 3.60 (m, 7H), 2.70 (m, 4H), 2.50 (m, 4H); LCMS (ES+, m/z) : 616,98 [M+H]⁺ ; LCMS (ES-, m/z) : 660,87 [M+HCOO⁻]⁻.

### Composé 24

### rac-(1R,2R,3S,3aR,8bS)-6,8-dimethoxy-3a-(4-methoxyphenyl)-2-(5-(methylamino)-1,3,4-oxadiazol-2-yl)-3-phenyl-2,3,3a,8b-tetrahydro-1H-cyclopenta[b]benzofuran-1,8b-diol

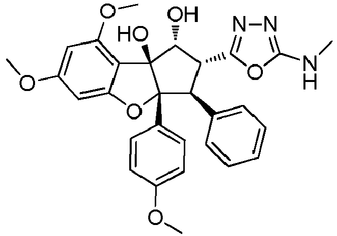

Solide blanc, 100 mg (30 %) ; obtenu à partir de rac-(1R,2R,3S,3aR,8bS)-1,8b-dihydroxy-6,8-dimethoxy-3a-(4-methoxyphenyl)-3-phenyl-2,3,3a, 8b-tetrahydro-1H-cyclopenta[b]benzofuran-2carbohydrazide précédemment décrit et en appliquant la procédure de synthèse des amino-oxadiazoles substitués en utilisant du isothiocyanatomethane.

RMN ¹H (DMSO-D₆, 400MHz, δ, ppm) : 7,14 (q, 1H, J = 5,0 Hz), 7,03 (m, 5H), 6,87 (m, 2H), 6,61 (d, 2H, J = 8,9 Hz), 6,29 (d, 1H, J = 1,8 Hz), 6,12 (d, 1H, J = 1,8 Hz), 5,17 (s, 1H), 5,01 (d, 1H, J = 4,3 Hz), 4,61 (m, 1H), 4,27 (m, 2H), 3,78 (s, 3H), 3,73 (s, 3H), 3,61 (s, 3H), 2,66 (d, 3H, J = 5,0 Hz) ; LCMS (ES+, m/z) : 532 [M+H]⁺ ; LCMS (ES-, m/z) : 576 [M+HCOO⁻]⁻.

### Composé 25

### rac-(1R,2R,3S,3aR,8bS)-6,8-dimethoxy-3a-(4-methoxyphenyl)-3-phenyl-2-(5-(propylamino)-1,3,4-oxadiazol-2-yl)-2,3,3a,8b-tetrahydro-1H-cyclopenta[b] benzofuran-1,8b-diol

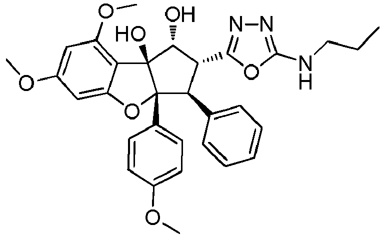

Solide blanc, 63 mg (37 %) ; obtenu à partir de rac-(1R,2R,3S,3aR,8bS)-1,8b-dihydroxy-6,8-dimethoxy-3a-(4-methoxyphenyl)-3-phenyl-2,3,3a, 8b-tetrahydro-1H-cyclopenta[b]benzofuran-2carbohydrazide précédemment décrit et en appliquant la procédure de synthèse des amino-oxadiazoles substitués en utilisant du 1-isothiocyanatopropane.

RMN ¹H (DMSO-D₆, 400MHz, δ, ppm) : 7,25 (t, 1H, J = 5,8 Hz), 7,03 (m, 5H), 6,87 (m, 2H), 6,61 (d, 2H, J = 8,9 Hz), 6,29 (d, 1H, J = 1,9 Hz), 6,12 (d, 1H, J = 1,9 Hz), 5,17 (bs, 1H), 5,01 (bs, 1H), 4,62 (bs, 1H), 4,27 (m, 2H), 3,10 (s, 3H), 3,73 (s, 3H), 3,61 (s, 3H), 2,98 (m, 2H), 1,43 (hex., 2H, J = 7,2 Hz), 0,8 (t, 3H, J = 7,2 Hz) ; LCMS (ES+, m/z) : 560,1 [M+H]⁺ ; LCMS (ES-, m/z) : 604,1 [M+HCOO⁻]⁻.

### Composé 26

### rac-(1R,2R,3S,3aR,8bS)-2-(5-((3-(diethytamino)propyt)amino)-1,3,4-oxadiazot-2-yl)-6,8-dimethoxy-3a-(4-methoxyphenyl)-3-phenyl-2,3,3a,8b-tetrahydro-1H-cyclopenta[b] benzofuran-1,8b-diol

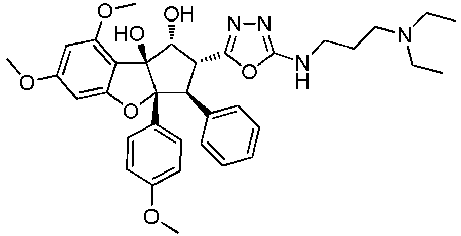

Solide beige, 2,8 mg (5 %) ; obtenu à partir de rac-(1R,2R,3S,3aR,8bS)-1,8b-dihydroxy-6, 8-dimethoxy-3 a-(4-methoxyphenyl)-3-phenyl-2,3,3 a, 8b-tetrahydro-1H-cyclopenta[b]benzofuran-2carbohydrazide précédemment décrit et en appliquant la procédure de synthèse des amino-oxadiazoles substitués en utilisant de la N,N-diéthyl-3-isothiocyanatopropan-1-amine

LCMS (ES+, m/z): 631,1 [M+H]⁺ ; LCMS (ES-, m/z): 663,1 [M+HCOO⁻]⁻

### Composé 27

### rac-(1R,2R,3S,3aR,8bS)-2-(5-hydroxy-1,3,4-oxadiazol-2-yl)-6,8-dimethoxy-3a-(4-methoxyphenyl)-3-phenyl-2,3,3a,8b-tetrahydro-1H-cyclopenta[b|benzofuran-1,8b-diol

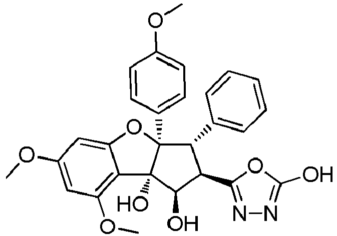

Solide blanc, 82 mg (65 %) ; obtenu à partir du composé rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2carbohydrazide comme suit : Dans un ballon et sous azote, introduire rac-(1R,2R,3S,3aR,8bS)-1,8b-dihydroxy-6,8-dimethoxy-3a-(4-methoxyphenyl)-3-phenyl-2,3,3a,8b-tetrahydro-1Hcyclopenta[b] benzofuran -2-carbohydrazide (40 mg, 0,081 mmol) dans 0,4 mL de tétrahydrofurane puis Et₃N (23,66 µl, 0,170 mmol). Ajouter, à température ambiante, 1,1'-Carbonyldiimidazole (16,33 mg, 0,101 mmol). Agiter à température ambiante durant 18 heures. Diluer le milieu avec de l'acétate d'éthyle. Laver avec une solution de HCL 1N puis avec une solution de NaCl saturée. Sécher la phase organique sur MgSO₄, filtrer puis concentrer. Un solide blanc est récupéré.

RMN ¹H (DMSO-D₆, 400MHz, δ, ppm) : 11,92 (s, 1H), 7,00 (m, 7H), 6,59 (d, 2H, J = 8,9 Hz), 6,28 (d, 1H, J = 1,9 Hz), 6,11 (d, 1H, J = 1,9 Hz), 5,17 (m, 2H), 4,63 (m, 1H), 4,20 (m, 2H), 3,78 (s, 3H), 3,73 (s, 3H), 3,60 (s, 3H) ; LCMS (ES+, m/z) : 501,0 [M-OH]⁺ ; LCMS (ES-, m/z) : 517,0 [M-H]⁻.

### Composé 28

### rac-(1R,2R,3S,3aR,8bS)-2-(5-mercapto-1,3,4-oxadiazol-2-yl)-6,8-dimethoxy-3a-(4-methoxyphenyl)-3-phenyl-2,3,3a,8b-tetrahydro-1H-cyclopenta[b]benzofuran-1,8b-diol

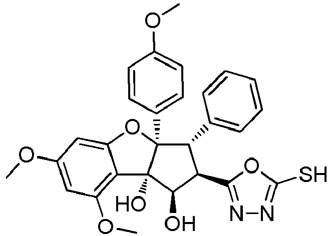

Solide blanc, 26 mg (49 %) ; obtenu à patir du composé rac-(1R,2R,3S,3aR,8bS)-1, 8b-dihydroxy-6, 8-dimethoxy-3 a-(4-methoxyphenyl)-3-phenyl-2,3,3 a, 8b-tetrahydro-1H-cyclopenta[b]benzofuran-2-carbohydrazide comme suit : Dans un ballon, introduire rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-carbohydrazide (50 mg, 0,102 mmol) dans l'éthanol (1 mL). Ajouter KOH (17,09 mg, 0,305 mmol) et CS₂ (12,24 µl, 0,203 mmol) et chauffer à 90°C pendant 1 heure. Concentrer le milieu réactionnel et reprendre à l'acétate d'éthyle et à l'eau. Ajouter une solution saturée de NaHCO₃ à la phase aqueuse et extraire à nouveau à l'acétate d'éthyle. Rassembler les phases organiques et les laver avec une solution saturée de NaCl puis les sécher sur Na₂SO₄ et concentrer. Le résidu est purifié sur gel de silice en utilisant comme éluant un mélange DCM/MeOH98/2.

RMN ¹H (DMSO-D₆, 400MHz, δ, ppm) : 14,19 (s, 1H), 7,01 (m, 7H), 6,59 (d, 2H, J = 9,0 Hz), 6,28 (d, 1H, J = 1,9 Hz), 6,11 (d, 1H, J = 1,9 Hz), 5,26 (d, 1H, J = 4,6 Hz), 5,22 (s, 1H), 4,67 (t, 1H, J = 4,6 Hz), 4,43 (dd, 1H, J = 4,6 Hz, 14,1 Hz), 4,25 (d, 1H, J = 14,1 Hz), 3,77 (s, 3H), 3,72 (s, 3H), 3,60 (s, 3H) ; LCMS (ES+, m/z) : 535,0 [M+H]⁺ ; LCMS (ES-, m/z) : 532,9 [M-H]⁻

### Composé 29

### rac-(1R,2S,3S,3aR,8bS)-2-(5-amino-4H-1,2,4-triazol-3-yl)-6,8-dimethoxy-3a-(4-methoxyphenyl)-3-phenyl-2,3,3a,8b-tetrahydro-1H-cyclopenta[b]benzofuran-1,8b-diol

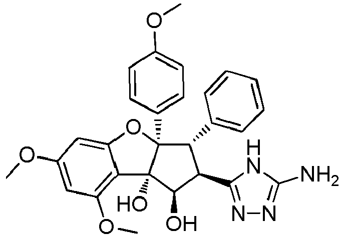

Solide blanc, 1 mg (2 %) ; obtenu à partir du composé rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2carbohydrazide comme suit : A une solution de rac-(1R,2R,3S,3aR,8bS)-1,8b-dihydroxy-6,8-dimethoxy-3a-(4-methoxyphenyl)-3-phenyl-2, 3,3a,8b-tetrahydro-1H-cyclopenta[b]benzofuran-2carbohydrazide (50 mg, 0,102 mmol) dans l'eau (0,6 mL) est ajouté 2-Methyl-isothiourea (9,15 mg, 0,102 mmol) puis sodium hydroxide (6,09 mg, 0,152 mmol) à 0°C. Le mélange est agité à 75°C pendant 5h. Purification du brut par HPLC préparative.

RMN ¹H (DMSO-D₆, 400MHz, δ, ppm) : 7.05 (m, 2H), 6.96 (m, 4H), 6.90 (m, 1H), 6.57 (m, 2H), 6.24 (m, 1H), 6.08 (m, 1H), 5.60 (bs, 2H), 4.95 (m, 3H), 4.40 (m, 1H), 4.34 (m, 1H), 4.11 (m, 1H), 3.76 (s, 3H), 3.70 (s, 3H), 3.58 (s, 3H) ; HRMS : C28H29N406 [M+H]⁺ cald 517,2082 found 517,2060.

### Composé 30

### rac-(1R,2S,3S,3aR,8bS)-6,8-dimethoxy-3a-(4-methoxyphenyl)-2-(5-methyl-4H-1,2,4-triazol-3-yl)-3-phenyl-2,3,3a,8b-tetrahydro-1H-cyclopenta[b]benzofuran-1,8b-diol

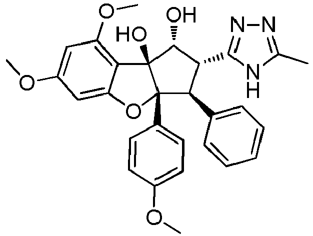

Solide blanc, 59 mg (37 %) ; obtenu à partir du composé rac-(1R,2R,3S,3aR,8bS)-1,8b-dihydroxy-6, 8-dimethoxy-3 a-(4-methoxyphenyl)-3-phenyl-2,3,3a,8b-tetrahydro-1H-cyclopenta[b]benzofuran-2carbohydrazide comme suit : A une solution de de sel d'acide chlorhydrique d'acétimidate d'éthyle (301 mg, 2,436 mmol) dans l'acétonitrile (16mL) est ajouté de la triéthylamine (849 µl, 6,09 mmol) puis rac-(1R,2R, 3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2carbohydrazide (600 mg, 1,218 mmol). Le mélange est agité à 90°C pendant 48 heures. Concentrer le milieu réactionnel et purifier le résidu sur gel de silice en utilisant comme éluant un mélange DCM/MeOH 98/2.

RMN ¹H (CDCl₃, 400MHz, δ, ppm) : 7,19 (d, 2H, J = 8,9 Hz), 7,00 (m, 5H), 6,65 (d, 2H, J = 8,9 Hz), 6,29 (d, 1H, J = 1,9 Hz), 6,13 (d, 1H, J = 1,9 Hz), 4,91 (d, 1H, J = 5,0 Hz), 4,51 (dd, 1H, J = 5,0 Hz, 14,1 Hz), 4,37 (d, 1H, J = 14,1 Hz), 3,86 (s, 3H), 3,84 (s, 3H), 3,70 (s, 3H), 3,07 (m, 1H), 2,32 (s, 3H) ; LCMS (ES+, m/z) : 516,0 [M+H]⁺ ; LCMS (ES-, m/z): 513,9 [M-H]⁻.

### Composé 31

### rac-(1R,2R,3S,3aR,8bS)-2-(3-isopropyl-1,2,4-oxadiazol-5-yl)-6,8-dimethoxy-3a-(4-methoxyphenyl)-3-phenyl-2,3,3a,8b-tetrahydro-1H-cyclopenta[b]benzofuran-1,8b-diol

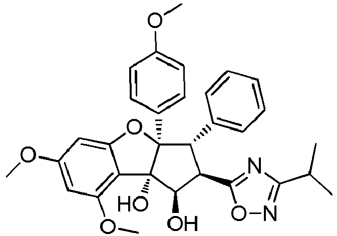

Solide blanc, 6,4 mg (11 %) ; obtenu à partir du composé rac-(1R,2R,3S,3aR, 8bS)-methyl1,8b-dihydroxy-6,8-dimethoxy-3a-(4-methoxyphenyl)-3-phenyl-2,3,3a,8b-tetrahydro-1Hcyclopenta[b]benzofuran-2-carboxylate comme suit : Dans un ballon et sous azote, introduire rac-(1R,2R,3S,3aR,8bS)-methyl 1,8b-dihydroxy-6,8-dimethoxy-3a-(4-methoxyphenyl)-3-phenyl-2,3,3a,8b-tetrahydro-1Hcyclopenta[b]benzofuran-2-carboxylate (50 mg, 0,102 mmol) dans 0,5 mL de toluène. Le milieu est limpide. Ajouter, à température ambiante, (Z)-N'-hydroxyisobutyrimidamide (11,41 mg, 0,112 mmol) puis K₂CO₃ (15,43 mg, 0,112 mmol). Agiter à reflux Après 4h, il y a très peu de produit qui se forme. Chauffer à reflux pendant 18 heures. Diluer le milieu avec de l'acétate d'éthyle puis laver avec de l'eau. Sécher la phase organique sur MgSO₄, filtrer puis concentrer. Purifier le résidu sur silice en utilisant comme éluant DCM/MeOH 95/5.

RMN ¹H (DMSO-D₆, 400MHz, δ, ppm) : 7,11 (d, 2H, J = 8,9 Hz), 7,01 (m, 3H), 6,86 (m, 2H), 6,61 (d, 2H, J = 8,9 Hz), 6,30 (d, 1H, J = 1,7 Hz), 6,11 (d, 1H, J = 1,7 Hz), 5,24 (s, 1H), 5,14 (m, 1H), 4,71 (m, 1H), 4,59 (dd, 1H, J = 5,1 Hz, 14,2 Hz), 4,41 (d, 1H, J = 14,2 Hz), 3,78 (s, 3H), 3,71 (s, 3H), 3,61 (s, 3H), 2,91 (hept., 1H, J = 6,9 Hz), 1,15 (d, 6H, J = 6,9 Hz) ; LCMS (ES+, m/z) : 545,0 [M+H]⁺ ; LCMS (ES-, m/z) : 588,9

[M+HCOO⁻]⁻.

### 1.4. Composés avec R₁ et R₂ formant un hétérocycle

Les composés selon l'invention peuvent être synthétisés selon le schéma réactionnel suivant : 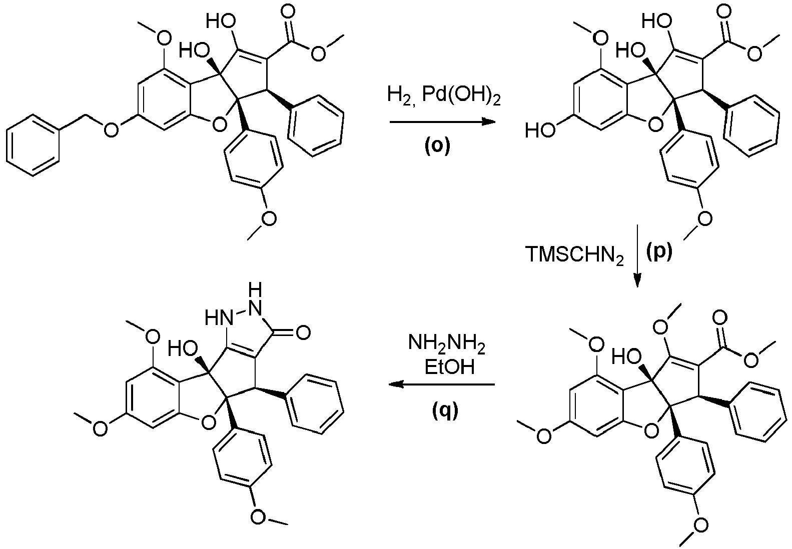
**(o) Procédure de déprotection du phénol:** Au rac-methyl (3R,3aR,8bS)-6-(benzyloxy)-1,8b-dihydroxy-8-methoxy-3a-(4-methoxyphenyl)-3-phenyl-3a,8b-dihydro-3H-cyclopenta[b]benzofuran-2-carboxylate (inter-1 décrit dans : Journal of the American Chemical Society, 2009, 131, 1607-1616) (1g, 1.765 mmol) dans l'acétate d'éthyle (10 mL) dégazé avec de l'azote est ajouté l'hydroxyide de palladium. Le milieu est ensuite agité sous pression d'hydrogène (1 bar) pendant 18h à température ambiante. Le palladium est filtré sur Célite^{®} et rincé avec de l'acétate d'éthyle puis un mélange CH₂Cl₂/MeOH : 80/20 à 10/90. Le filtrat est évaporé et le résidu obtenu (inter-2) est utilisé sans plus de purification.
**(p) Procédure de méthylation du phénol** : Dans un tube de Heck de 100 ml introduire le composé précédemment obtenu (0,869 g, 1,824 mmol), ajouter les solvants (MeOH 10 mL et toluène 5 mL). Additionner goutte à goutte une solution de triméthylsilyl diazométhane (2M dans l'hexane), boucher le flacon et agiter à température ambiante pendant 18 heures. Reprendre plusieurs fois par du dichlorométhane et de l'acétonitrile puis évaporer afin d'obtenir une mousse homogène. Purifier le résidu sur un gel de silice en utilisant comme éluant DCM/AcOEt 100/0 à 80/20 pour obtenir rac-methyl (3R,3aR,8bS)-8b-hydroxy-1,6,8-trimethoxy-3a-(4-methoxyphenyl)-3-phenyl-3a,8b-dihydro-3H-cyclopenta[b]benzofuran-2-carboxylate sous forme de solide blanc (inter-3, 540 mg, 58% sur 2 étapes).
   RMN ¹H (CDCl₃, 400MHz, δ, ppm) : 7,10 (m, 5H), 6,98 (m , 2H), 6,57 (d, 2h, J = 8,9 Hz), 6,22 (d, 1H, J = 1,9 Hz), 6,06 (d, 1H, J = 1,9 Hz), 4,51 (s, 1H), 4,19 (s, 3H), 3,83 (s, 3H), 3,82 (s, 3H), 3,68 (s, 3H), 3,52 (s, 3H) ; LCMS (ES+, m/z): 487.25 [M-OH]⁺ ; LCMS (ES-, m/z): 549.32 [M+HCOO⁻]⁻.
**(q) Procédure de formation du cycle pyrazolone** : Dans un flacon pour micro-onde muni d'un barreau aimanté, sont additionnés le composé obtenu précédemment (0.250 g, 0.496 mmol), l'éthanol (4 mL) puis l'hydrazine monohydrate (2.070 ml, 27.3 mmol). Le flacon bouché est placé dans un appareil micro-onde et le milieu réactionnel est soumis à deux cycles de chauffage de 1 minute à 180°C. Après évaporation à sec, reprendre par le solide avec un minimum de méthanol, filtrer le solide et sécher sous vide.

### Composé 32

### rac-(4R,4aR,9bS)-9b-hydroxy-7,9-dimethoxy-4a-(4-methoxyphenyl)-4-phenyl-1,2,4,4a-tetrahydrobenzofuro[2',3':4,5]cyclopenta[1,2-c]pyrazol-3(9bH)-one

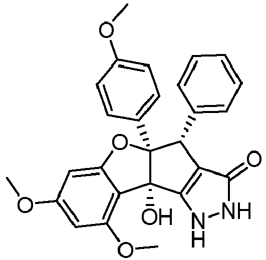

Solide beige, 136 mg (58 %) ; obtenu selon la procédure décrite ci-dessus.

RMN ¹H (DMSO-D₆, 400MHz, δ, ppm) : 11,73 (bs, 1H), 9,53 (bs, 1H), 7,06 (m, 4H), 6,98 (m, 3H), 6,37 (d, 1H, J = 1,9 Hz), 6,14 (d, 1H, J = 1,9 Hz), 5,41 (bs, 1H), 4,21 (s, 1H), 3,78 (s, 6H), 3,57 (s, 3H) ; LCMS (ES+, m/z) : 473,18 [M+H]⁺.

Les composés selon l'invention peuvent être synthétisés également selon le schéma réactionnel suivant : 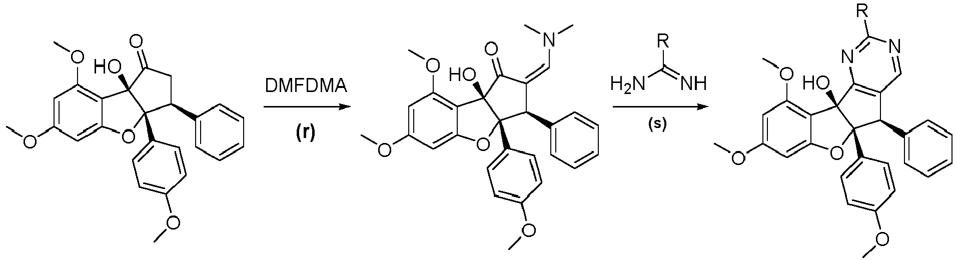
**(r) Procédure pour la formation de l'énamine :** Le rac-(3R,3aS,8bS)-8b-hydroxy-6,8-dimethoxy-3a-(4-methoxyphenyl)-3-phenyl-2,3,3a,8b-tetrahydro-1H-cyclopenta[b] benzofuran-1-one (Journal of the American Chemical Society, 2006, 128(24), 7754-7755) et le dimethylformamide dimethylacétal (DMFDMA, 5 équiv.) sont agités dans le DMF (0,5 M) à 60°C pendant 5 heures. Le DMF est évaporé sous vide puis le résidu est repris puis évaporé trois fois avec du dichlorométhane pour conduire à une mousse jaune (accompagné d'un sous-produit de masse M - H₂O).
**(s) Procédure pour l'addition de guanidines :** Dans un ballon, introduire rac-(3R,3aR,8bR,Z)-2-((dimethylamino)methylene)-8b-hydroxy-6,8-dimethoxy-3a-(4-methoxyphenyl)-3-phenyl-2,3,3a,8b-tetrahydro-1H-cyclopenta[b]benzofuran-1-one obtenu précédemment, le carbonate de potassium (3 équiv.) et le chlorhydrate de guanidine (2 équiv.) ainsi que de l'éthanol (0,2 M). Agiter magnétiquement à 60°C pendant 18h. Le précipité est filtré, puis rincé avec de l'eau puis de l'éther éthylique, il est enfin séché sous vide à 40°C. Lorsque le produit ne précipite pas, les solvants sont évaporés et le résidu est purifié sur gel de silice.

### Composé 33

### rac-(5aR,10bS)-2-amino-8,10-dimethoxy-5a-(4-methoxyphenyl)-5-phenyl-5a,10b-dihydro-5H-benzofuro[2',3':4,5]cyclopenta[1,2-d]pyrimidin-10b-ol

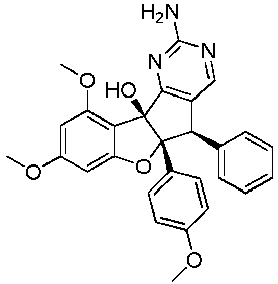

Solide blanc, 549 mg (63 %) ; obtenu à partir du composé rac-(3R,3aR,8bR,Z)-2-((dimethylamino)methylene)-8b-hydroxy-6,8-dimethoxy-3a-(4-methoxyphenyl)-3-phenyl-2,3,3a,8b-tetrahydro-1H-cyclopenta[b]benzofuran-1-one en suivant la procédure d'addition de guanidines en utilisant la guanidine.

RMN ¹H (DMSO-D₆, 400MHz, δ, ppm) : 7,80 (s, 1H), 7,08 (m, 3H), 6,83 (m, 4H), 6,74 (bs, 2H), 6.58 (m, 2H), 6,42 (s, 1H), 6,16 (s, 1H), 5,58 (s, 1H), 4,22 (s, 1H), 3,79 (s, 3H), 3,71 (s, 3H), 3,59 (s, 3H) ; LCMS (ES+, m/z) : 484,18 [M+H]⁺.

### Composé 34

### rac-(5aR,10bS)-8,10-dimethoxy-5a-(4-methoxyphenyl)-2-morpholino-5-phenyl-5a,10b-dihydro-5H-benzofuro[2',3':4,5]cyclopenta[1,2-d]pyrimidin-10b-ol

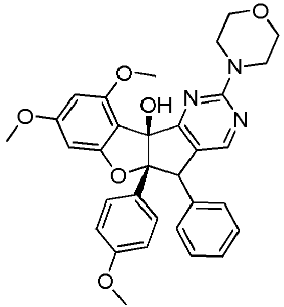

Solide beige, 9 mg (15 %) ; obtenu à partir du composé rac-(3R,3aR,8bR,Z)-2-((dimethylamino)methylene)-8b-hydroxy-6,8-dimethoxy-3a-(4-methoxyphenyl)-3-phenyl-2,3,3a,8b-tetrahydro-1H-cyclopenta[b]benzofuran-1-one en suivant la procédure d'addition de guanidines en utilisant la morpholine-4-carboximidamide.

RMN ¹H (DMSO-D₆, 400MHz, δ, ppm) : 7,97 (s, 1H), 7,08 (m, 3H), 6,88 (m, 2H), 6,82 (d, 2H, J = 8,5 Hz), 6.56 (d, 2H, J = 8,5 Hz), 6,41 (s, 1H), 6,14 (s, 1H), 5,70 (s, 1H), 4,27 (s, 1H), 3,85 (m, 4H), 3,79 (s, 3H), 3,73 (m, 4H), 3,71 (s, 3H), 3,58 (s, 3H) ; LCMS (ES+, m/z) : 554,07 [M+H]⁺

### Composé 35

### rac-N-((5R,5aR,10bS)-10b-hydroxy-8,10-dimethoxy-5a-(4-methoxyphenyl)-5-phenyl-5a,10b-dihydro-5H-benzofuro[2',3':4,5]cyclopenta[1,2-d]pyrimidin-2-yl)cyanamide

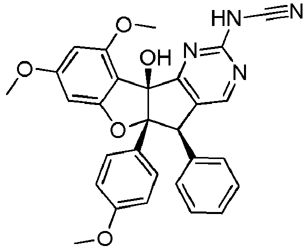

Solide blanc, 24 mg (46 %) ; obtenu à partir du composé rac-(3R,3aR,8bR,Z)-2-((dimethylamino)methylene)-8b-hydroxy-6,8-dimethoxy-3a-(4-methoxyphenyl)-3-phenyl-2,3,3a,8b-tetrahydro-1H-cyclopenta[b]benzofuran-1-one en suivant la procédure d'addition de guanidines en utilisant la cyano-guanidine.

RMN ¹H (DMSO-D₆, 400MHz, δ, ppm) : 7,70 (s, 1H), 7,06 (m, 3H), 6,83 (m, 4H), 6,55 (m, 2H), 6,38 (s, 1H), 6,14 (s, 1H), 5,60 (bs, 1H), 4,20 (s, 1H), 3,79 (s, 3H), 3,72 (s, 3H), 3,58 (s, 3H) ; LCMS (ES+, m/z) : 508,96 [M+H]⁺

### Composé 36

### rac-(5aR,10bS)-8,10-dimethoxy-5a-(4-methoxyphenyl)-2-(methylthio)-5-phenyl-5a,10b-dihydro-5H-benzofuro[2',3':4,5]cyclopenta[1,2-d]pyrimidin-10b-ol

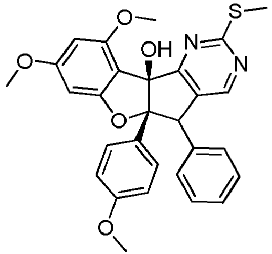

Solide beige, 18 mg (28 %) : obtenu à partir du composé rac-(3R,3aR,8bR,Z)-2-((dimethylamino)methylene)-8b-hydroxy-6,8-dimethoxy-3a-(4-methoxyphenyl)-3-phenyl-2,3,3a,8b-tetrahydro-1H-cyclopenta[b]benzofuran-1-one en suivant la procédure d'addition de guanidines en utilisant le methyl carbamimidothioate.

RMN ¹H (DMSO-D₆, 400MHz, δ, ppm) : 8,21 (s, 1H), 7,10 (m, 3H), 6,88 (m, 2H), 6,79 (d, 2H, J = 8,5 Hz), 6,58 (d, 2H, J = 8,5 Hz), 6,44 (s, 1H), 6,17 (s, 1H), 5,99 (s, 1H), 4,40 (s, 1H), 3,80 (s, 3H), 3,72 (s, 3H), 3,59 (s, 3H), 2,65 (s, 3H) ; LCMS (ES+, m/z) : 514,90 [M+H]⁺.

### Composé 37

### rac-(5R,5aR,10bS)-2-amino-4-ethoxy-8,10-dimethoxy-5a-(4-methoxyphenyl)-5-phenyt-5a,10b-dihydro-5H-benzofuro[2',3':4,5]cyctopenta[1,2-d]pyrimidin-10b-ot

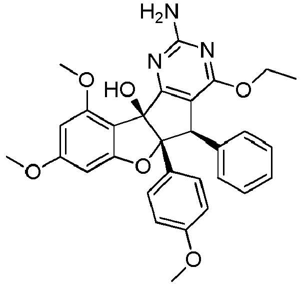

Solide jaune pâle, 6 mg (7 %) ; obtenu à partir de rac-(3R,3aR,8bR)-2-(bis(methylthio)methylene)-6,8-dimethoxy-3a-(4-methoxyphenyl)-3-phenyl-8b ((trimethylsilyl)oxy)-2,3,3a,8b-tetrahydro-1H-cyclopenta[b]benzofuran-1-one (décrit dans le Journal of Organic Chemistry, 1989, 54(1), 77-83) en suivant la procédure d'addition de guanidines en utilisant la guanidine.

RMN ¹H (CDCl₃, 400MHz, δ, ppm) : 7,02 (m, 5H), 6,82 (m, 2H), 6,55 (m, 2H), 6,26 (d, 1H, J = 1,9 Hz), 6,05 (d, 1H, J = 1,9 Hz), 5,42 (bs, 2H), 5,49 (s, 1H), 4,26 (qd, 1H, J = 7,0 Hz; 10,6 Hz), 4,14 (qd, 1H, J = 7,0 Hz; 10,6 Hz), 3,81 (s, 3H), 3,79 (s, 3H), 3,66 (s, 3H), 1,02 (t, 3H, J = 7,0 Hz) ; LCMS (ES+, m/z) : 528,24 [M+H]⁺.

### Composé 38

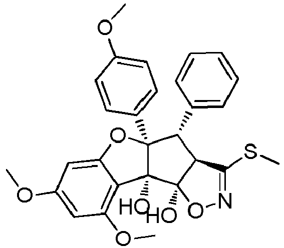

Solide jaune pâle, 15 mg (90 %) ; obtenu à partir de rac-(3R,3aR,8bR)-2-(bis(methylthio)methylene)-6,8-dimethoxy-3a-(4-methoxyphenyl)-3-phenyl-8b ((trimethylsilyl)oxy)-2,3,3a,8b-tetrahydro-1H-cyclopenta[b]benzofuran-1-one (décrit dans le Journal of Organic Chemistry, 1989, 54(1), 77-83) comme suit : Dans un ballon, mélanger l'hydroxylamine hydrochloride (11,41 mg, 0,164 mmol) et la triethylamine (22,83 µl, 0,164 mmol) dans l'éthanol (0,5 mL), laisser agiter à 50°C pendant 15 min puis ajouter le rac-(3R,3aR,8bR)-2-(bis(methylthio)methylene)-6,8-dimethoxy-3a-(4-methoxyphenyl)-3-phenyl-8b ((trimethylsilyl)oxy)-2,3,3a,8b-tetrahydro-1H-cyclopenta [b]benzofuran-1-one (20 mg, 0,033 mmol), agiter à 65°C pendant 24 heures. Ajouter de l'eau et de l'acétate d'éthyle, extraire 2 fois à l'acétate d'éthyle, laver une fois par H₂O/NaCl, sécher sur MgSO4, filtrer puis évaporer. Le résidu est purifié sur gel de silice en utilisant comme éluant CH₂Cl₂/AcOEt 90/10.

RMN ¹H (DMSO-D₆, 400MHz, δ, ppm) : 7,09 (d, 2H, J = 8,7 Hz), 7,05 (m, 3H), 6,80 (m, 2H), 6,66 (d, 2H, J = 8,7 Hz), 6,17 (d, 1H, J = 1,9 Hz), 6,08 (d, 1H, J = 1,9 Hz), 5,26 (s, 1H), 3,85 (s, 3H), 3,82 (d, 1H, J = 13,5 Hz), 3,76 (s, 3H), 3,66 (s, 3H), 3,37 (d, 1H, J = 13,5 Hz), 2,35 (s, 1H), 2,24 (s, 3H) ; LCMS (ES+, m/z): 504,0 [M-OH]⁺ ; LCMS (ES-, m/z) : 520,0 [M-H]⁻

### Composé 39

### rac-(4R,4aR,9bS)-7,9-dimethoxy-4a-(4-methoxyphenyl)-3-(methylthio)-4-phenyl-1,4,4a,9b-tetrahydrobenzofuro[2',3':4,5]cyclopenta[1,2-c]pyrazol-9b-ol

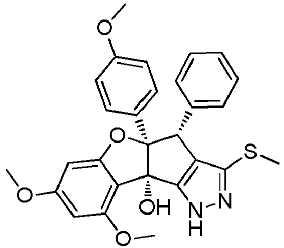

Solide jaune pâle, 8,8 mg (53 %); obtenu à partir de rac-(3R,3aR,8bR)-2-(bis(methylthio)methylene)-6,8-dimethoxy-3a-(4-methoxyphenyl)-3-phenyl-8b ((trimethylsilyl)oxy)-2,3,3a,8b-tetrahydro-1H-cyclopenta[b]benzofuran-1-one (décrit dans le Journal of Organic Chemistry, 1989, 54(1), 77-83) comme suit: Dans un ballon, mélanger rac-(3R,3aR,8bR)-2-(bis(methylthio)methylene)-6,8-dimethoxy-3a-(4-methoxyphenyl)-3-phenyl-8b ((trimethylsilyl)oxy)-2,3,3a,8b-tetrahydro-1H-cyclopenta [b]benzofuran-1-one (20 mg, 0,033 mmol) dans l'éthanol (0,5 mL) puis ajouter l'hydrazine monohydrate (17 µl, 0,164 mmol), chauffer à 50°C pendant 24h. Ajouter de l'eau et de l'acétate d'éthyle, extraire 2 fois à l'acétate d'éthyle, laver une fois par H₂O/NaCl, sécher sur MgSO4, filtrer puis évaporer.Le résidu est purifié sur gel de silice en utilisant comme éluant Cyclohexane/AcOEt 40/60 puis 20/80.

RMN ¹H (CDCl₃, 400MHz, δ, ppm) : 7,01 (m, 7H), 6,50 (d, 2H, J = 8,9 Hz), 6,22 (d, 1H, J = 1,9 Hz), 5,99 (d, 1H, J = 1,9 Hz), 4,49 (s, 1H), 3,75 (s, 3H), 3,69 (s, 3H), 3,58 (s, 3H), 2,27 (s, 3H) ; LCMS (ES+, m/z): 500,9 [M+H]⁺ ; LCMS (ES-, m/z) : 502,9 [M-H]⁻.

Les composés selon l'invention peuvent être synthétisés selon le schéma réactionnel suivant : 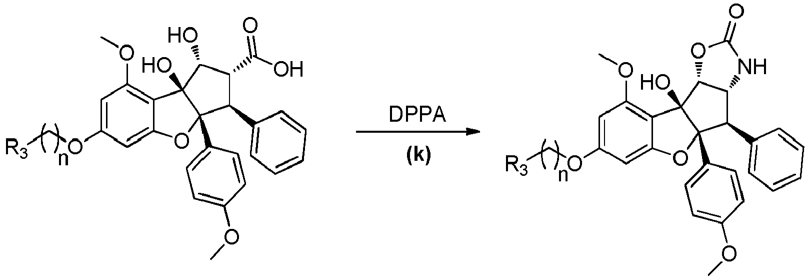 (k) Procédure pour le réarrangement de Curtius : Décrite précédemment pour l'Exemple avec R₃ = H et n = 1.

### Composé 40

### rac-(3aR,4R,4aR,9bS,9cR)-9b-hydroxy-7,9-dimethoxy-4a-(4-methoxyphenyl)-4-phenyl-3,3a,4,4a,9b,9c-hexahydro-2H-benzofuro[3',2':3,4]cyclopenta[1,2-d]oxazol-2-one

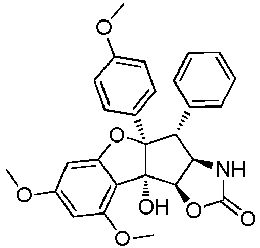

Solide blanc, 93 mg (47 %) ; obtenu à partir du composé rac-(1R,2R,3S,3aR,8bS)-1,8b-dihydroxy-6,8-dimethoxy-3a-(4-methoxyphenyl)-3-phenyl-2,3,3a, 8b-tetrahydro-1H-cyclopenta[b]benzofuran-2-carboxylic acid en suivant la procédure de réarrangement de Curtius.

RMN ¹H (DMSO-D₆, 400MHz, δ, ppm) : 8,15 (s, 1H), 7,11 (m, 3H), 7,01 (d, 2h J = 8,5 Hz), 6,9 (m, 2H), 6,70 (d, 2H, J = 8,5 Hz), 6,34 (d, 1H, J = 1,8 Hz), 6,22 (d, 1H, J = 1,8 Hz), 5,37 (s, 1H), 5,32 (d, 1H, J = 8,7 Hz), 4,79 (dd, 1H, J = 8,7 Hz; 10,5 Hz), 3,79 (s, 6H), 3,66 (s, 3H), 3,36 (d, 1H, J = 10,5 Hz) ; LCMS (ES+, m/z) : 457,88 [M-OH]⁺ ; LCMS (ES-, m/z) : 473,92 [M-H]⁻, 519,92 [M+HCOO-]⁻.

### Composé 41

### rac-(3aR,4R,4aR,9bS,9cR)-7-((4,5-dihydroxypentyl)oxy)-9b-hydroxy-9-methoxy-4a-(4-methoxyphenyl)-4-phenyl-3,3a,4,4a,9b,9c-hexahydro-2H-benzofuro[3',2':3,4]cyclopenta[1,2-d]oxazol-2-one

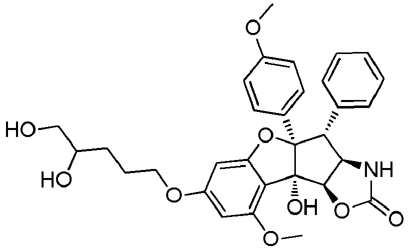

Solide blanc, 41 mg (77 %) : obtenu à partir de rac-methyl (1R,2R,3S,3aR,8bS)-1,6,8b-trihydroxy-8-methoxy-3a-(4-methoxyphenyl)-3-phenyl-2,3,3a,8b-tetrahydro-1H-cyclopenta[b]benzofuran-2-carboxylate en suivant les procédures de substitution du phénol avec le pent-4-èn-1-ol, de saponification, de réarrangement de Curtius, puis de dihydroxylation.

RMN ¹H (CDCl₃, 400MHz, δ, ppm) : 7,14 (m, 3H), 7,05 (m, 2H), 6,86 (m, 2H), 6,74 (m, 2H), 6,21 (d, 1H, J = 1,9 Hz), 6,115 (m, 1H, J = 1,9 Hz), 5,56 (d, 1H, J = 8,1 Hz), 5,04 (s, 1H), 4,66 (dd, 1H, J = 8,2 Hz, 10,8 Hz), 4,03 (m, 2H), 3,91 (s, 3H), 3,80 (m, 1H), 3,74 (s, 3H), 3,70 (dd, 1H, J = 3,1 Hz, 10,8 Hz), 3,63 (d, 1H, J = 10,8 Hz), 3,49 (dd, 1H, J = 7,5 Hz, 10,8 Hz), 1,92 (m, 2H), 1,66 (m, 2H) ; LCMS (ES+, m/z) : 563,99

[M+H]⁺.

### Composé 42

### rac-(3aR,4R,4aR,9bS,9cR)-9b-hydroxy-7-(4-hydroxybutoxy)-9-methoxy-4a-(4-methoxyphenyl)-4-phenyl-3,3a,4,4a,9b,9c-hexahydro-2H-benzofuro[3',2':3,4]cyclopenta[1,2-d]oxazol-2-one

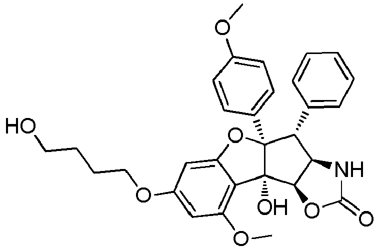

Solide blanc, 249 mg (98 %) : obtenu à partir du composé 41 en suivant la procédure de coupure oxydante suivie de la procédure de réduction de l'aldéhyde.

RMN ¹H (CDCl₃, 400MHz, δ, ppm) : 7,14 (m, 3H), 7,05 (m, 2H), 6,86 (m, 2H), 6,74 (m, 2H), 6,21 (d, 1H, J = 1,9 Hz), 6,15 (d, 1H, J = 1,9 Hz), 5,56 (d, 1H, J = 8,2 Hz), 5,05 (s, 1H), 4,65 (dd, 1H, J = 8,3 Hz, 10,8 Hz), 4,03 (t, 1H, J = 6,3 Hz), 3,91 (s, 3H), 3,74 (t, 1H, J = 6,3 Hz), 3,73 (s, 3H), 3,63 (d, 1H, J = 10,8 Hz), 1,91 (m, 2H), 1,77 (m, 2H); LCMS (ES+, m/z) : 534,00 [M+H]⁺.

### Composé 43

### rac-(3aR,4R,4aR,9bS,9cR)-7-(4-azidobutoxy)-9b-hydroxy-9-methoxy-4a-(4-methoxyphenyl)-4-phenyl-3,3a,4,4a,9b,9c-hexahydro-2H-benzofuro[3',2':3,4]cyclopenta[1,2-d]oxazol-2-one

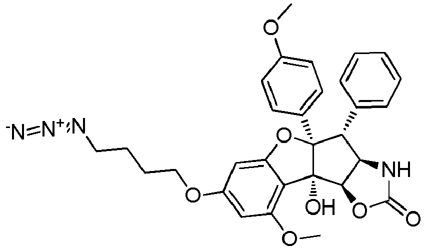

Solide beige, 104 mg (39 %) ; obtenu à partir du **composé 42** en suivant la procédure de synthèse de l'azoture à partir de l'alcool.

RMN ¹H (CDCl₃, 400MHz, δ, ppm) : 7,14 (m, 3H), 7,05 (m, 2H), 7,86 (m, 2H), 6,74 (m, 2H), 6, 20 (d, 1H, J = 1,9 Hz), 6,15 (d, 1H, J = 1,9 Hz), 5,56 (d, 1H, J = 8,3 Hz), 5,00 (s, 1H), 4,66 (dd, 1H, J = 8,3 Hz, 10,8 Hz), 4,02 (m, 2H), 3,91 (s, 3H), 3,74 (s, 3H), 3,65 (m, 2H), 3,40 (t, 1H, J = 6,7 Hz), 2,01 (m, 2H), 1,90 (m, 2H), 1,82 (m, 2H) ; LCMS (ES-, m/z) : 556.84 [M-H]⁻.

### Composé 44

### rac-(3aR,4R,4aR,9bS,9cR)-7-(4-aminobutoxy)-9b-hydroxy-9-methoxy-4a-(4-methoxyphenyl)-4-phenyl-3,3a,4,4a,9b,9c-hexahydro-2H-benzofuro[3',2':3,4] cyclopenta[1,2-d]oxazol-2-one

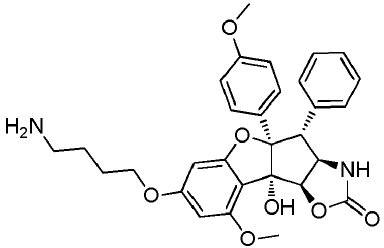

Solide beige, 26 mg (54 %) ; obtenu à partir du **composé 43** en suivant la procédure de réduction de l'azoture.

RMN ¹H (DMSO-D₆, 400MHz, δ, ppm) : 8,15 (s, 1H), 7,51 (bs, 1H), 7,11 (m, 3H), 7,00 (m, 2H), 6,94 (m, 2H), 6,69 (m, 2H), 6,33 (d, 1H, J = 1,9 Hz), 6,18 (d, 1H, J = 1,9 Hz), 5,36 (s, 1H), 5,32 (d, 1H, J = 8,4 Hz), 4,78 (dd, 1H, J = 8,4 Hz, 10,4 Hz), 4,03 (t, 1H, J = 6,4 Hz), 3,79 (s, 3H), 3,66 (s, 3H), 3,33 (m, 1H), 2,85 (t, 1H, J = 7,3 Hz), 1,78 (m, 2H), 1,71 (m, 2H); LCMS (ES+, m/z) : 533,01 [M+H]⁺.

### Composé 45

### rac-(3aR,4R,4aR,9bS,9cR)-7-(2-aminoethoxy)-9b-hydroxy-9-methoxy-4a-(4-methoxyphenyl)-4-phenyl-3,3a,4,4a,9b,9c-hexahydro-2H-benzofuro[3',2':3,4]cyclopenta[1,2-d]oxazol-2-one

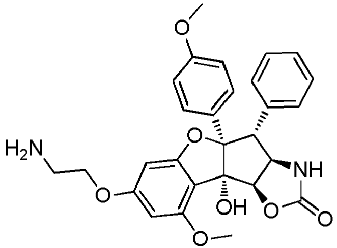

Solide blanc, 332 mg (63 %) ; obtenu à partir du composé rac-methyl (1R,2R,3S,3aR,8bS)-1,6,8b-trihydroxy-8-methoxy-3a-(4-methoxyphenyl)-3-phenyl-2,3, 3a,8b-tetrahydro-1H-cyclopenta[b]benzofuran-2-carboxylate en suivant les procédures de substitution du phénol, de saponification et de réarrangement de Curtius. Enfin, les procédures de dihydroxylation, coupure oxydante, réduction de l'alcool, transformation en azoture puis réduction de l'azoture ont conduit au produit désiré.

RMN ¹H (DMSO-D₆, 400MHz, δ, ppm) : 8,14 (s, 1H), 7,11 (m, 3H), 7,00 (m, 2H), 6,94 (m, 2H), 6,69 (m, 2H), 6,32 (d, 1H, J = 1,9 Hz), 6,21 (d, 1H, J = 1,9 Hz), 5,37 (bs, 1H), 5,32 (d, 1H, J = 8,6 Hz), 4,78 (dd, 1H, J = 8,6 Hz, 10,8 Hz), 3,96 (t, 2H, J = 5,6 Hz), 3,78 (s, 3H), 3,65 (s, 3H), 3,35 (d, 1H, J = 10,8 Hz), 2,88 (t, 2H, J = 5,6 Hz), 1,90 (bs, 2H); LCMS (ES+, m/z) : 504,94 [M+H]⁺.

### Composé 46

### rac-(3aR,4R,4aR,9bS,9cR)-9b-hydroxy-7,9-dimethoxy-4a-(4-methoxyphenyl)-4-phenyl-3,3a,4,4a,9b,9c-hexahydro-2H-benzofuro[3',2':3,4]cyclopenta[1,2-d]oxazole-2-thione

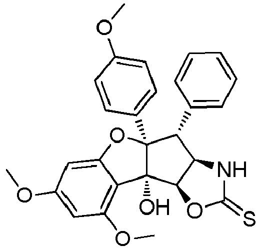

Solide jaune pâle, 54 mg (33 %); obtenu à partir du **composé 9** comme suit: Dans un flacon, introduire rac-(1R,2R,3R,3aR,8bS)-2-amino-6,8-dimethoxy-3a-(4-methoxy phenyl)-3-phenyl-2,3,3a,8b-tetrahydro-1H-cyclopenta[b]benzofuran-1,8b-diol (150 mg, 0,334 mmol) dans une solution 1M de carbonate de sodium (667 µl, 0,667 mmol), ajouter CS₂ (30,2 µl, 0,501 mmol) et chauffer à 110°C pendant 15 minutes. Laisser revenir à température ambiante. Un précipité se forme, filtrer et sécher. Le produit est obtenu sous forme d'un solide jaune pâle.

RMN ¹H (DMSO-D₆, 400MHz, δ, ppm) : 10,45 (bs, 1H), 7,12 (m, 3H), 7,99 (m, 4H), 6,69 (m, 2H), 6,35 (d, 1H, J = 1,9 Hz), 6,25 (d, 1H, J = 1,9 Hz), 5,64 (m, 1H), 5,50 (s, 1H), 5,04 (m, 1H), 3,83 (s, 3H), 3,79 (s, 3H), 3,65 (s, 3H), 3,39 (d, 1H, J = 10,9 Hz) ; LCMS (ES+, m/z) : 491,88 [M+H]⁺

### Composé 47

### rac-(3aR,4R,4aR,9bS,9cR)-2-amino-7,9-dimethoxy-4a-(4-methoxyphenyl)-4-phenyl-4,4a,9b,9c-tetrahydro-3aH-benzofuro[3',2':3,4]cyclopenta[1,2-d]oxazol-9b-ol

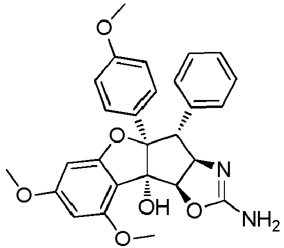

Solide blanc, 12 mg (35 %); obtenu à partir du **composé 9** comme suit: Dans un pilulier et sous azote, introduire rac-(1R,2R,3R,3aR,8bS)-2-amino-6,8-dimethoxy-3a-(4-methoxyphenyl)-3-phenyl-2,3,3a,8b-tetrahydro-1H-cyclopenta[b]benzofuran-1,8b-diol (34 mg, 0,076 mmol) dans 1,0 mL d'éthanol, ajouter, à température ambiante, le Cyanogen bromide (30,3 µl, 0,091 mmol) puis agiter pendant 18 heures. Concentrer le milieu réactionnel puis ajouter une solution de NaHCO₃ saturée. Un solide reste en suspension. Le filtrer et le rincer à l'eau puis sécher sous vide. Le produit est purifié sur silice en utilisant comme éluant un mélange DCM/MeOH 95/5.

RMN ¹H (DMSO-D₆, 400MHz, δ, ppm) : 7,05 (m, 7H), 6,69 (m, 2H), 6,29 (s, 1H), 6,18 (s, 1H), 5,72 (s, 1H), 5,13 (d, 2H, J = 8,4 Hz), 4,75 (t, 1H, J = 10,4 Hz), 3,80 (s, 3H), 3,77 (s, 3H), 3,65 (s, 3H); LCMS (ES+, m/z) : 475,01 [M+H]⁺.

### Composé 48

### rac-(4aR,5R,5aR,10bS,10cR)-10b-hydroxy-8,10-dimethoxy-5a-(4-methoxyphenyl)-5-phenyl-4,4a,5,5a,10b,10c-hexahydrobenzofuro[2',3':4,5]cyclopenta[1,2-b][1,4]oxazin-3(2H)-one

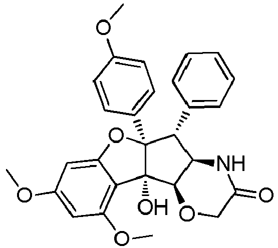

Solide blanc, 1,7 mg (2 %) ; obtenu à partir du **composé 9** comme suit: Dans un flacon, introduire rac-(1R,2R,3R,3aR,8bS)-2-amino-6,8-dimethoxy-3a-(4-methoxy phenyl)-3-phenyl-2,3,3a,8b-tetrahydro-1H-cyclopenta[b]benzofuran-1,8b-diol (69 mg, 0,154 mmol) en solution dans du THF (2,5 mL). Refroidir à 0°C et ajouter NaH à 60 % dans l'huile (6,45 mg, 0,161 mmol) puis methyl 2-chloroacetate (18,32 mg, 0,169 mmol). Laisser agiter à 0°C pendant 3 heures puis à 30°C pendant 3 heures. Hydrolyser par une solution saturée de NaHCO₃ et extraire 2 fois à l'acétate d'éthyle, rassembler les phases organiques, laver par une solution saturée de NH₄Cl puis par une solution saturée de NaCl. Sécher la phase organique sur Na₂SO₄ et concentrer. Le résidu est purifié sur gel de silice en utilisant comme éluant un mélange DCM/MeOH 99/1.

LCMS (ES-, m/z): 488,1 [M-H]⁻.

### Composé 49

### rac-(6R,6aS,11bR)-11b-hydroxy-9,11-dimethoxy-6a-(4-methoxyphenyl)-6-phenyl-3,4,6,6a,11b,11c-hexahydro-2H-benzofuro[3',2':3,4]cyclopenta[1,2-f][1,4]oxazepin-5(5aH)-one

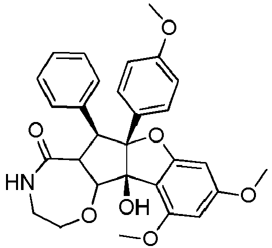

Mousse blanche, 6 mg (15 %) ; obtenue à partir du composé rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-carboxylic acid comme suit: Dans un flacon, introduire rac-(1R,2R,3S,3aR,8bS)-1,8b-dihydroxy-6,8-dimethoxy-3a-(4-methoxyphenyl)-3-phenyl-2,3,3a,8b-tetrahydro-1H-cyclopenta[b]benzofuran-2-carboxylic acid (300 mg, 0,627 mmol) en solution dans le DCM (7 mL), ajouter la di(1H-imidazol-1-yl) méthanone (122 mg, 0,752 mmol, CDI) et laisser agiter 18 heures à température ambiante. Ajouter de l'eau et laisser décanter. Récupérer et sécher la phase organique sur Na₂SO₄ et concentrer. Dans un flacon, introduire rac-(1R,2R,3S,3aR,8bS)-1,8b-dihydroxy-6,8-dimethoxy-3a-(4-methoxyphenyl)-3-phenyl-2,3,3a, 8b-tetrahydro-1H-cyclopenta[b]benzofuran-2-carboxylic 1H-imidazole-1-carboxylic anhydride (100 mg, 0,175 mmol) dans du THF (2 mL). Ajouter le sel d'acide chlorhydrique de la 2-chloroéthanamine (60,8 mg, 0,524 mmol) et la DIEA (183 µl, 1,048 mmol) et chauffer à 50°C pendant 6 heures. Filtrer le précipité puis concentrer le filtrat, le résidu est utilisé tel quel.

Dans un flacon, introduire rac-(1R,2R,3S,3aR,8bS)-N-(2-chloroethyl)-1,8b-dihydroxy-6,8-dimethoxy-3a-(4-methoxyphenyl)-3-phenyl-2,3,3a,8b-tetrahydro-1H-cyclopenta[b] benzofuran-2-carboxamide (45 mg, 0,083 mmol) dans du THF (1 mL). Refroidir à 0°C et ajouter NaH (4,00 mg, 0,100 mmol). Laisser agiter à température ambiante pendant 30 minutes puis chauffer à 50 °C pendant 2 heures. Ajouter de l'eau et extraire 2 fois à l'acétate d'éthyle, rassembler les phases organiques et les sécher sur Na₂SO₄, concentrer. Le résidu est purifié sur gel de silice en utilisant comme éluant un mélange DCM/MeOH 97.5/2.5.

RMN ¹H (DMSO-D₆, 400MHz, δ, ppm) : 6,99 (m, 5H), 6,88 (d, 2H, J = 7,4 Hz), 6,58 (d, 2H, J = 9,0 Hz), 6,27 (d, 1H, J = 1,9 Hz), 6,10 (d, 1H, J = 1,9 Hz), 5,76 (s, 1H), 5,05 (s, 1H), 4,81 (d, 1H, J = 4,0 Hz), 4,59 (m, 1H), 4,18 (d, 1H, J = 14,0 Hz), 4,06 (m, 2H), 3,80 (m, 1H), 3,78 (s, 3H), 3,73 (s, 3H), 3,59 (s, 3H), 3,55 (m, 2H); LCMS (ES+, m/z) : 504,30 [M+H]⁺.

### 2- Activité biologique des composés selon l'invention

### 2.1. Activité anti-proliférative des composés selon l'invention (IC₅₀ en M)

### Culture des lignées et mesure de la viabilité cellulaire :

La lignée HCT116 (ATCC, CCL-247) issue d'un cancer du côlon a été cultivée dans le milieu MEM (Minimum Essential Médium Eagle) supplémenté avec 2 mM de L-Glutamine (Sigma, G7513), 5 % de sérum de veau fœtal (Sigma, F7524) et des antibiotiques (Sigma, A59-55). Le protocole de détermination de l'activité cytotoxique consiste à ensemencer des cellules sur des plaques 96-puits (Perkin Elmer, 6005668) selon une densité de 1500 cellules par puits. Après 24h d'incubation, le composé à tester est appliqué dans chaque puits, en utilisant une série de dilutions en cascade dans le solvant diméthylsulfoxyde (DMSO) (Sigma, D8418), et ce, à partir de solutions stocks à 10 mM dans 100 % DMSO. Chacune des dilutions a été ajoutée sur les cellules 24 heures après leur ensemencement. Dans ces conditions la concentration finale en solvant est de 0,1 % DMSO. Une lecture de la prolifération cellulaire a été faite à 72 heures après ajout des produits avec le kit ATPLite^{™} (Perkin Elmer, 6016947) et selon les recommandations du fabricant. L'analyse des résultats de prolifération a été faite en se comparant avec des conditions où seul le solvant véhicule (milieu de culture 0,1 % DMSO) a été ajouté sur les cellules. Les courbes de dose-réponse obtenues ont été analysées à l'aide du logiciel Prism 4.03 (GraphPad Software Inc.), ou par une méthode analytique équivalente, afin de déterminer la concentration de chaque composé permettant d'inhiber 50% de la prolifération cellulaire (EC₅₀).

A titre d'exemple, les propriétés cytotoxiques de quelques composés de l'invention évalués sur la lignée HCT116 (lignée cellulaire humaine de cancer du côlon) sont reportées dans le **Tableau 1.**

**Tableau 1. Valeur d'EC₅₀ des composés selon l'invention et du silvestrol. Les valeurs de d'EC₅₀ sont exprimées en unités de concentration (mol/L).**

| **produit** | **HCT116 (EC₅₀)** |
|---|---|
| silvestrol | 2,46E-09 |
| 47 | 1,10E-09 |
| 50 | 1,22E-09 |
| 42 | 2,16E-09 |
| 40 | 2,52E-09 |
| 46 | 2,59E-09 |
| 8 | 2,61E-09 |
| 15 | 3,83E-09 |
| 2 | 4,03E-09 |
| 41 | 4,20E-09 |
| 52 | 4,90E-09 |

### 2.2. Activité anti-tumorale des composés selon l'invention

Les xénogreffes ont été établies à partir des cellules de cancer du sein MDA-MD-231 (ATCC: HTB-26) sur des souris SCID (Harlan, UK) par voie sous-cutanée. Les animaux ont été traités et soignés conformément au Guide pour les soins et l'utilisation des animaux de laboratoire (Conseil national de recherches, 1996) et de la Directive européenne CEE/ 86/609, sous la supervision d'un personnel expert et autorisé à réaliser des études expérimentales avec des animaux de laboratoire.

Toutes les expériences ont été menées dans le respect des réglementations françaises et locales (Direction des Services Vétérinaires, Haute-Garonne, Toulouse) suivant les directives d'un comité d'éthique, fondées sur les lignes directrices UKCCCR pour le bien-être des animaux à des fins expérimentales en cancérologie expérimentale, comme indiqué précédemment. L'implantation des fragments de tumeurs humaines MDA-MB-231 est effectuée par voie sous-cutanée au niveau du flan des souris SCID par trocart et pour l'étude de la croissance tumorale, on laisse les tumeurs atteindre un volume médian de 70-130 mm³ avant de démarrer les études.

Après randomisation dans les cages de traitement, les inhibiteurs ont été administrés par voie intraveineuse en suivant un schéma de traitement q1d5x3 (5 injections par semaine durant trois semaines). Les souris ont été surveillées et pesées quotidiennement. Les tumeurs ont été mesurées au pied à coulisse et les volumes des tumeurs (mm³) ont été estimés par la formule suivante : volume= 0,5 (longueur × largeur²). Chaque groupe expérimental contient 5 individus. L'efficacité du traitement a été évaluée par l'analyse de la mesure du volume médian des tumeurs traitées par rapport au volume médian des tumeurs traitées par le véhicule (contrôle). Le critère d'évaluation T/C correspond à : [(volume médian du groupe traité, T / volume médian du groupe contrôle, C) × 100]. Ce rapport T/C est exprimé en pourcentage. La valeur optimale de T/C correspond au ratio qui reflète l'inhibition de croissance maximale obtenue au cours de l'étude. Les gains ou pertes de poids maximum, exprimés en pourcentage du poids initial des animaux ainsi que le pourcentage de décès liés à la drogue (c'est-à-dire pourcentage des animaux traités qui sont morts avant le contrôle) ont été utilisés pour fournir une évaluation de la toxicité des composés. En accord avec le critère du NCI (National Cancer Institute), une dose est considérée comme toxique si elle induit une perte de poids supérieure à -20% par rapport au poids initial de la souris ou si elle induit plus de 20% de décès (Corbett et al. J Exp Ther Oncol 1996, 1:95-108). Les résultats obtenus sont reportés dans le tableau 2.

**Tableau 2. Activité anti-tumorale des composés selon l'invention et du silvestrol**

| **Composé** | **MDA-MB-231** | | | | |
|---|---|---|---|---|---|
| | **Schéma de traitement** | **Dose** (mg/kg) | **Mort** (%) | **T/C opt.** (%) | **Activité** |
| **Silvestrol** | q1d5 x3 | 1,4 | **100** | | Toxique |
| | | 1 | **100** | | Toxique |
| | | 0,7 | 40 | 63 | Toxique |
| | | 0,5 | 0 | 83 | Pas d'activité |
| | | 0,2 | 0 | 92 | Pas d'activité |
| **2** | q1d5 x3 | 0,2 | 0 | **37** | Actif |
| **24** | q1d5 x3 | 2,8 | 0 | **25** | Actif |
| **30** | q1d5 x3 | 0,7 | 0 | **15** | Actif |
| **46** | q1d5 x3 | 1,4 | 0 | **39** | Actif |
| **19** | q1d5 x3 | 1,3 | 0 | **26** | Actif |
| **18** | q1d5 x3 | 3,5 | 0 | **6** | Actif |
| | | 2,5 | 0 | **40** | Actif |
| **21** | q1d5 x3 | 1 | 0 | **22** | Actif |
| **22** | q1d5 x3 | 1,4 | 0 | **21** | Actif |

Le silvestrol est hautement toxique à partir d'une dose de 0,7mg.kg. En dessous de cette dose, le silvestrol ne présente plus aucune activité anti-tumorale. Les composés selon l'invention sont au contraire actifs à de faibles doses auxquelles ils induisent une diminution significative de la taille des tumeurs. Aucune toxicité n'a été constatée lors de l'administration des composés selon l'invention.

## Revendications

1. Composé de formule générale (I) suivante : 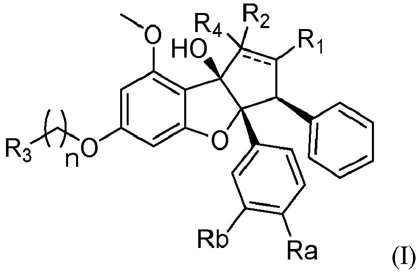
sous forme d'un de ses énantiomères ou d'un mélange de ses énantiomères tel qu'un mélange racémique,
ou un sel et/ou solvate pharmaceutiquement acceptable de celui-ci,
dans laquelle :
- 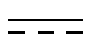 représente une liaison simple ou une liaison double,
- n représente un nombre entier compris entre 1 et 10,
- R₁ représente CO₂R₁₀, CONH₂, NR₁₁R₁₂, NR₁₃COR₁₄, NR₁₅CONR₁₆R₁₇, NR₁₈CSNR₁₉R₂₀, NR₂₁SO₂R₂₂, NR₂₃CO₂R₂₄ ou un hétéroaryle éventuellement substitué préférentiellement choisi parmi les triazoles et oxadiazoles éventuellement substitués,
- R₂ représente OH, ou
- R₁ et R₂ forment ensemble, avec les atomes de carbone qui les portent, un hétérocycle éventuellement substitué, préférentiellement choisi parmi les cycles pyrimidine, pyrazole, pyrazolone, oxazoline, isoxazoline, oxazalanone, oxazalanethione, morpholinone et oxazepane éventuellement substitués, l'hétérocycle éventuellement substitué ne pouvant être : 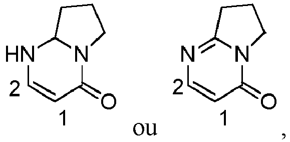 le carbone 1 désignant l'atome de carbone portant le groupe R₁ et le carbone 2 désignant l'atome de carbone portant le groupe R₂,
- R₃ représente H, OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NR₃₃(CH₂)ₓ)_{y}(CH₂)_{z}R₃₄, ONR₈₄R₈₅, aryle éventuellement substitué, ou hétéroaryle éventuellement substitué,
- R₄ est absent lorsque 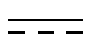 représente une liaison double et R₄ représente H ou OH lorsque 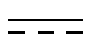 représente une liaison simple,
- R₁₀ à R₃₀, R₃₂, R₃₃, R₃₈ et R₃₉ représentent, indépendamment les uns des autres, H ou un groupe (C₁-C₆)alkyle, aryle, aryl-(C₁-C₆)alkyle ou (C₁-C₆)alkyl-aryle, ledit groupe étant éventuellement substitué par un ou plusieurs groupes choisis parmi (C₁-C₆)alkyle, OR₃₅, et NR₃₆R₃₇, ou
- R₁₁ et R₁₂, ou R₁₆ et R₁₇, ou R₁₉ et R₂₀, ou R₂₆ et R₂₇, ou R₂₉ et R₃₀, forment ensemble, avec l'atome d'azote qui les porte, un hétérocycle azoté éventuellement substitué,
- R₃₁ et R₃₄ représentent, indépendamment l'un de l'autre, H, OR₃₅, NR₃₆R₃₇ ou un groupe (C₁-C₆)alkyle, aryle, aryl-(C₁-C₆)alkyle, ONR₈₆R₈₇ ou (C₁-C₆)alkyl-aryle, ledit groupe étant éventuellement substitué par un ou plusieurs groupes choisis parmi (C₁-C₆)alkyle, OR₃₅, et NR₃₆R₃₇,
- R₃₅ à R₃₇ et R₈₄ à R₈₇ représentent, indépendamment les uns des autres, H ou un groupe (C₁-C₆)alkyle, aryle ou aryl-(C₁-C₆)alkyle,
- Ra représente un atome d'halogène (par ex. Br ou Cl), CN ou un groupe (C₁-C₆)alcoxy (tel que méthoxy),
- Rb représente H ou un groupe (C₁-C₆)alcoxy (tel que méthoxy), ou
- Ra et Rb forment ensemble une chaîne -OCH₂O-, et
- m, p, r, q, w, x, y, z représentent, indépendamment les uns des autres, un nombre entier compris entre 1 et 4,
à condition que lorsque R₁ représente CO₂R₁₀ ou CONH₂, R₃ représente OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, ou (NR₃₂(CH₂)_{w}NR₃₃(CH₂)ₓ)_{y}(CH₂)_{z}R₃₄ avec R₂₅ ≠ H.

2. Composé selon la revendication 1, **caractérisé en ce que** n est compris entre 1 et 4.

3. Composé selon la revendication 1 ou 2, **caractérisé en ce que** :
- 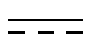 représente une liaison simple,
- R₁ représente CO₂R₁₀ ou CONH₂,
- R₃ représente OR₂₅, CHOHCH₂OH, CHO, N₃, NR₂₆R₂₇, CO₂R₂₈, CONR₂₉R₃₀, NR₃₈COR₃₉, (O(CH₂)ₘO(CH₂)ₚ)ᵣ(CH₂)_{q}R₃₁, (NR₃₂(CH₂)_{w}NP₃₃(CH₂)ₓ)_{y}(CH₂)_{z}P₃₄, ONR₈₄R₈₅, aryle éventuellement substitué, ou hétéroaryle éventuellement substitué,
- R₄ représente H, et
- R₁₀ représente H ou un groupe (C₁-C₆)alkyle.

4. Composé selon la revendication 1 ou 2, **caractérisé en ce que** :
- 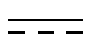 représente une liaison simple,
- R₁ représente NR₁₁R₁₂, NR₁₃COR₁₄, NR₁₅CONR₁₆R₁₇, NR₁₈CSNR₁₉R₂₀ ou NR₂₁SO₂R₂₂, et
- R₄ représente H,
et de préférence R₃ représente H.

5. Composé selon la revendication 1 ou 2, **caractérisé en ce que** :
- 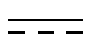 représente une liaison simple,
- R₁ représente 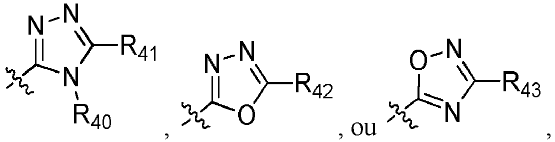
- R₄ représente H,
- R₄₀ représente H ou un groupe (C₁-C₆)alkyle, aryle ou aryl-(C₁-C₆)alkyle,
- R₄₁ à R₄₃ représentent, indépendamment les uns des autres, H ou un groupe (C₁-C₆)alkyle, aryle, aryl-(C₁-C₆)alkyle, OR₄₄, SR₄₅ ou NR₄₆R₄₇,
- R₄₄ à R₄₇ représentent, indépendamment les uns des autres, H ou un groupe (C₁-C₆)alkyle, aryle ou aryl-(C₁-C₆)alkyle, ledit groupe étant éventuellement substitué par un ou plusieurs groupes choisis parmi (C₁-C₆)alkyle, OR₄₈, NR₄₉R₅₀, et les polyamines, ou
- R₄₆ et R₄₇ forment ensemble, avec l'atome d'azote qui les porte, un hétérocycle azoté éventuellement subtitué par un groupe (C₁-C₆)alkyle, et
- R₄₈ à R₅₀ représentent, indépendamment les uns des autres, H ou un groupe (C₁-C₆)alkyle, aryle ou aryl-(C₁-C₆)alkyle.

6. Composé selon la revendication 1 ou 2, **caractérisé en ce que** le motif 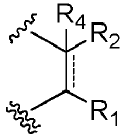 représente : 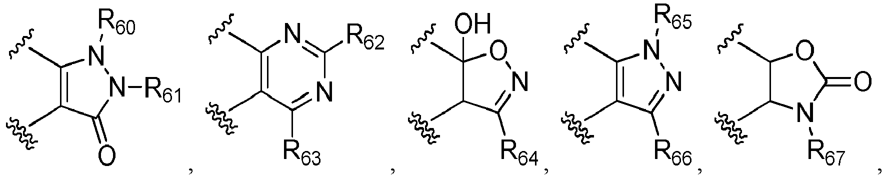 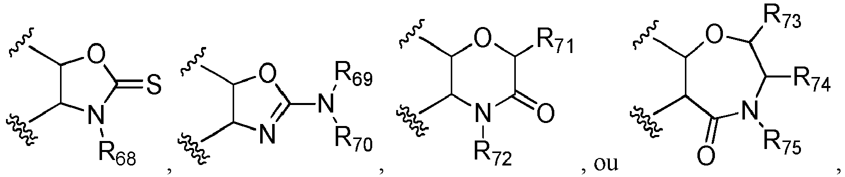 avec :
- R₆₀, R₆₁, R₆₅, R₆₇, R₆₈, R₇₂ et R₇₅ représentant, indépendamment les uns des autres, H ou un groupe (C₁-C₆)alkyle, aryle ou aryl-(C₁-C₆)alkyle,
- R₆₉ et R₇₀ représentant, indépendamment l'un de l'autre, H ou un groupe (C₁-C₆)alkyle, aryle ou aryl-(C₁-C₆)alkyle, ou formant ensemble, avec l'atome d'azote qui les porte, un hétérocycle azoté éventuellement substitué par un groupe (C₁-C₆)alkyle,
- R₆₂, R₆₃, R₆₄, R₆₆, R₇₁, R₇₃ et R₇₄ représentant, indépendamment les uns des autres, H ou un groupe (C₁-C₆)alkyle, aryle, aryl-(C₁-C₆)alkyle, OR₇₆, SR₇₇ ou NR₇₈R₇₉, et
- R₇₆ à R₇₉ représentant, indépendamment les uns des autres, H ou un groupe (C₁-C₆)alkyle, aryle, aryl-(C₁-C₆)alkyle, ou CN, ou
- R₇₈ et R₇₉ formant ensemble, avec l'atome d'azote qui les porte, un hétérocycle azoté éventuellement substitué par un groupe (C₁-C₆)alkyle.

7. Composé selon l'une quelconque des revendications 1 à 6, **caractérisé en ce que**, lorsque 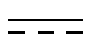 représente une liaison simple, R₁ et R₂ sont situés du même côté du noyau cyclopentane auquel ils sont liés, et de préférence du côté opposé aux groupes OH, phényle et *m*-Rb-*p*-Ra-phényle liés également à ce noyau cyclopentane.

8. Composé selon la revendication 1, **caractérisé en ce qu'**il est choisi parmi les composés suivants, sous forme d'un de leurs énantiomères ou d'un mélange de leurs énantiomères tel qu'un mélange racémique : 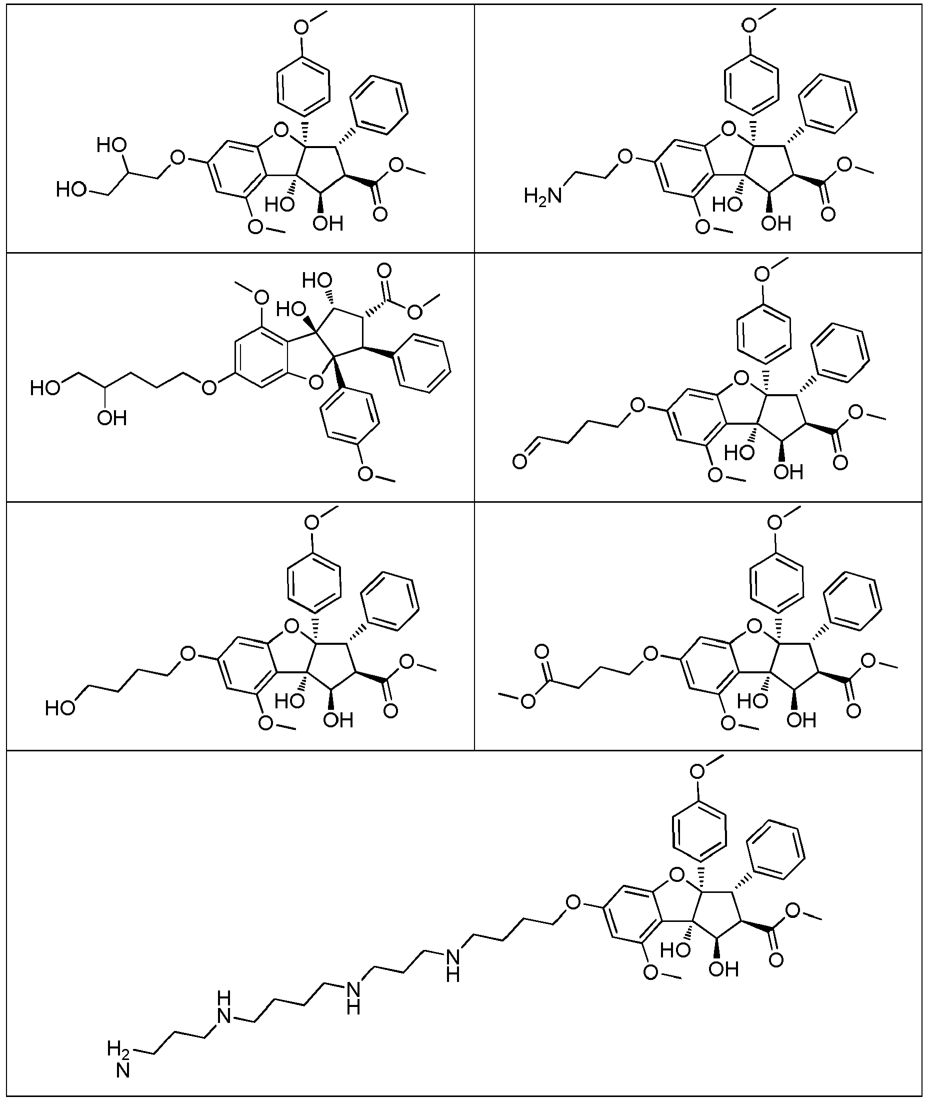 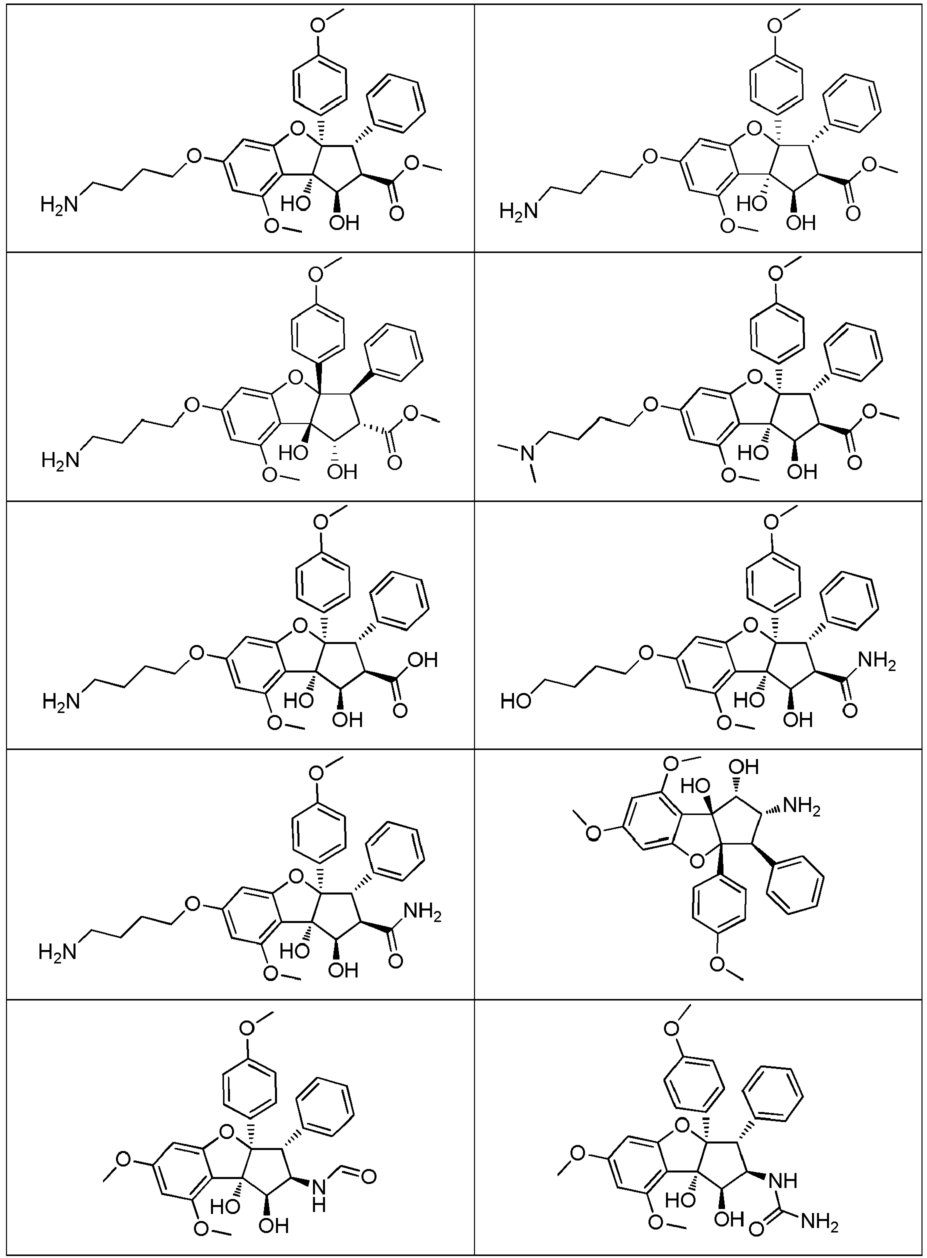 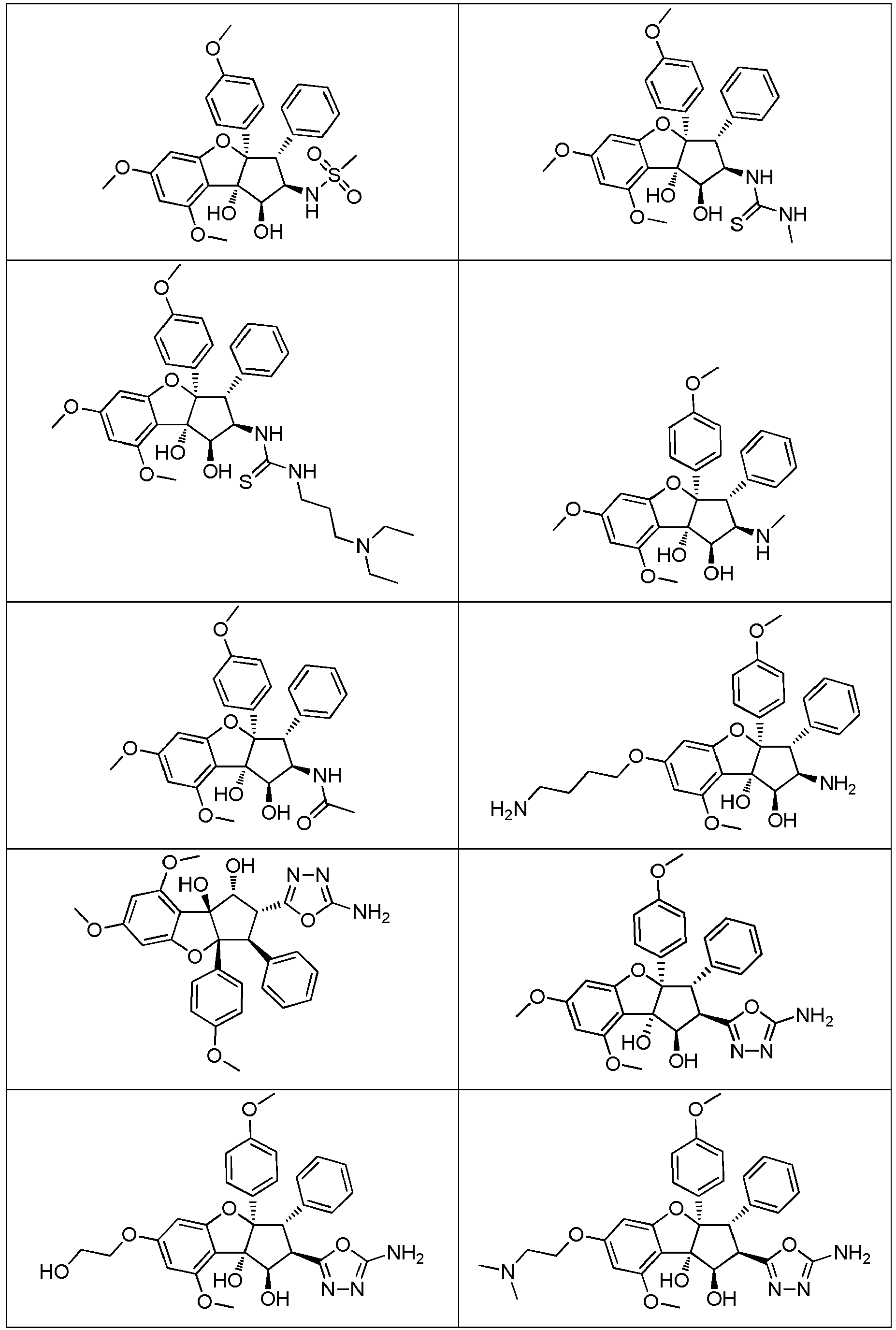 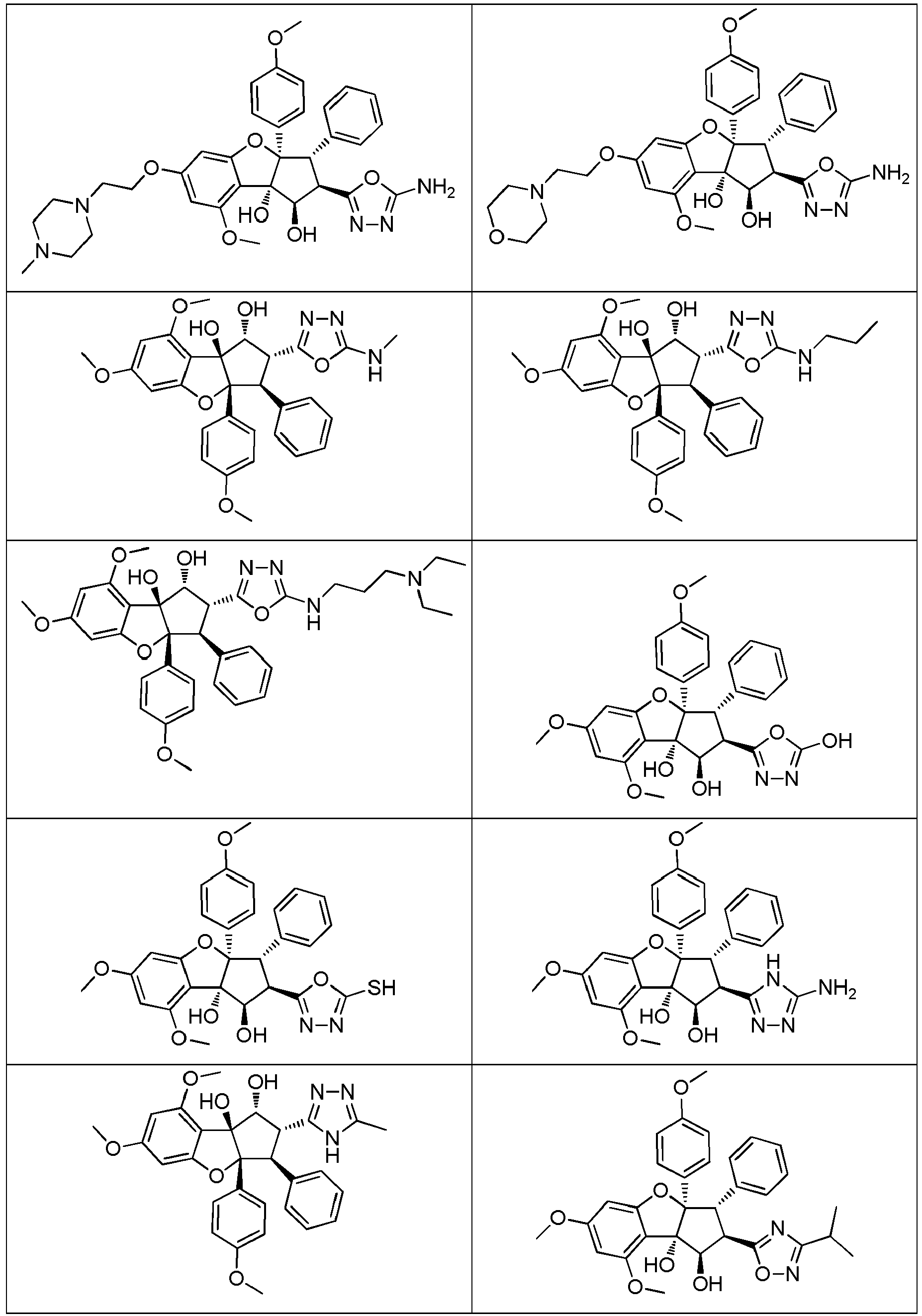 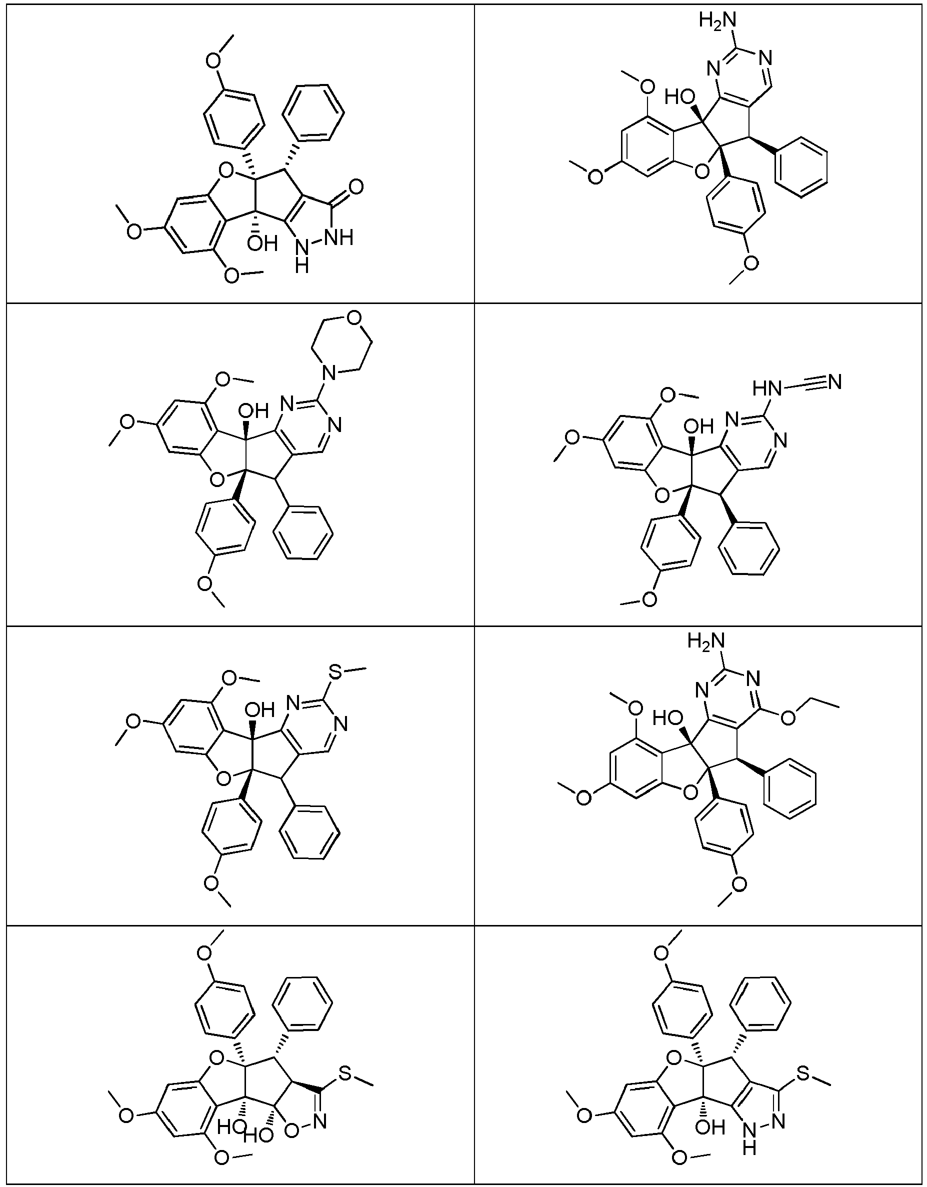 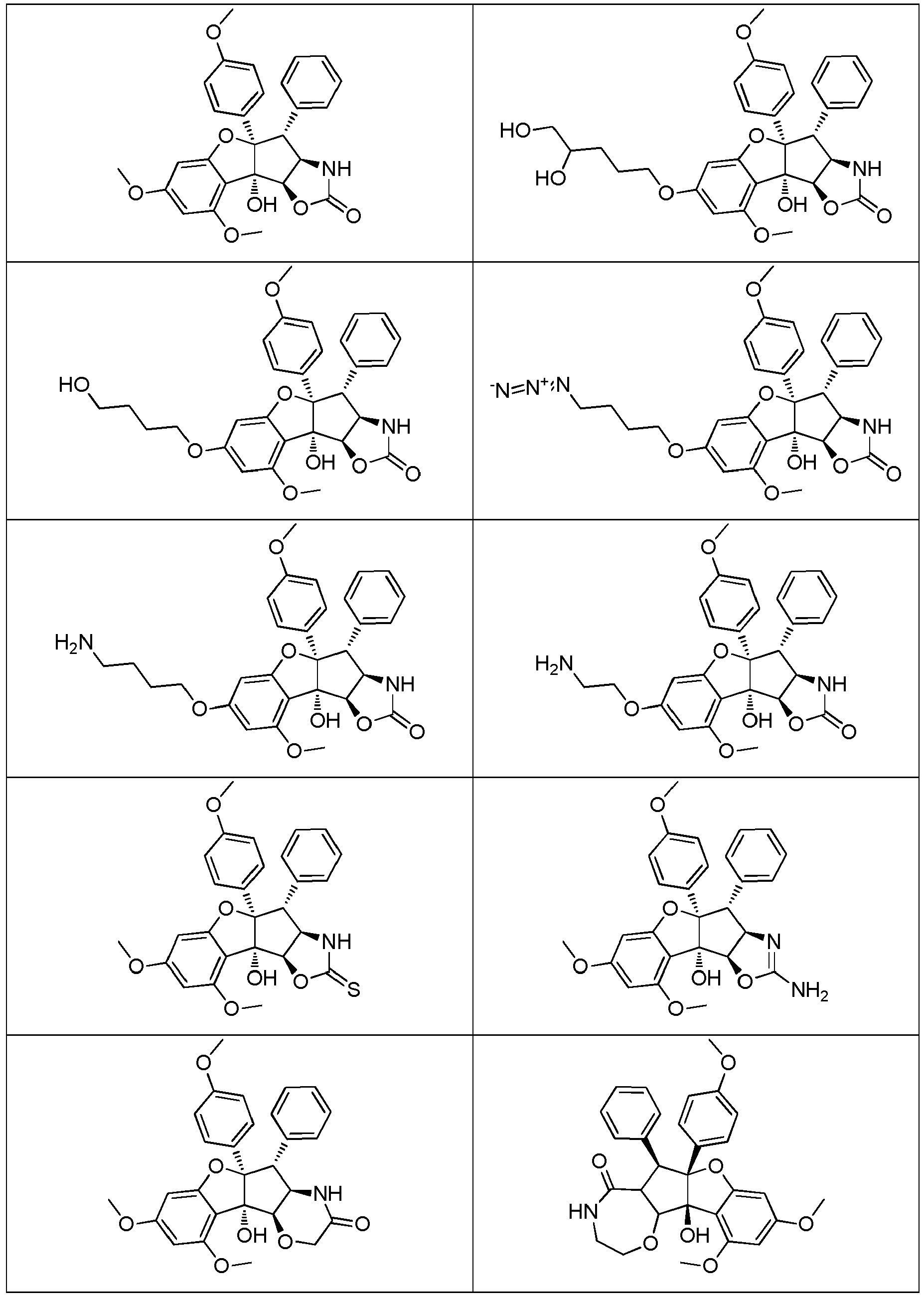 et leurs sels et/ou solvates pharmaceutiquement acceptables.

9. Composé selon l'une quelconque des revendications 1 à 8 pour son utilisation en tant que médicament.

10. Composé selon l'une quelconque des revendications 1 à 8 pour son utilisation dans le traitement du cancer.

11. Composition pharmaceutique comprenant au moins un composé selon l'une quelconque des revendications 1 à 8 et au moins un excipient pharmaceutiquement acceptable.

12. Procédé de préparation d'un composé de formule (I) selon la revendication 1 pour lequel R₃ = H comprenant la réaction d'un composé de formule (II) suivante : 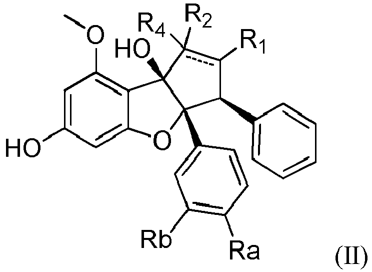 pour lequel Ra, Rb, R₁, R₂ et R₄ sont tels que définis à la revendication 1, avec un alcool de formule H-(CH₂)ₙOH, pour lequel n est tel que défini à la revendication 1, dans des conditions de Mitsunobu.

13. Procédé de préparation d'un composé de formule (I) selon la revendication 1 pour lequel R₃ = N₃, NR₃₈COR₃₉ ou NR₂₆R₂₇ comprenant :
(a1) pour obtenir un composé de formule (I) pour lequel R₃ = N₃, la réaction d'un composé de formule (III) suivante : 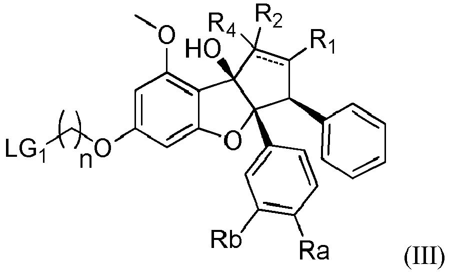 pour lequel Ra, Rb, R₁, R₂, R₄ et n sont tels que définis à la revendication 1 et LG₁ représente un groupe partant tel qu'un atome d'halogène ou une fonction hydroxyle activée,
avec un azoture de formule MN₃, M représentant un métal alcalin ou un groupe SiRR'R" avec R, R' et R" représentant chacun, indépendamment les uns des autres, un groupe (C₁-C₆)alkyle ou aryle,
(b1) pour obtenir un composé de formule (I) pour lequel R₃ = NH₂, la réduction de la fonction azoture d'un composé de formule (I) pour lequel R₃ = N₃ éventuellement obtenu selon l'étape (al),
(c1) pour obtenir un composé de formule (I) pour lequel R₃ = NR₃₈COR₃₉ ou NR₂₆R₂₇ et au moins l'un de R₂₆ et R₂₇ ne représente pas un atome d'hydrogène, la substitution d'un composé de formule (I) pour lequel R₃ = NH₂ éventuellement obtenu selon l'étape (b1).

14. Procédé de préparation d'un composé de formule (I) selon la revendication 1 pour lequel R₃ = CHOHCH₂OH, CHO, CO₂R₂₈, CONR₂₉R₃₀, OR₂₅, (O(CH₂)m O(CH₂)ₚ)ᵣ(CH₂)_{q}R₃₁, NR₂₆R₂₇ ou (NR₃₂(CH₂)_{w}NR₃₃(CH₂)ₓ)_{y}(CH₂)_{z}R₃₄, comprenant :
(a2) pour obtenir un composé de formule (I) pour lequel R₃ = CHOHCH₂OH, la réaction de dihydroxylation de la fonction vinyle d'un composé de formule (IV) suivante : 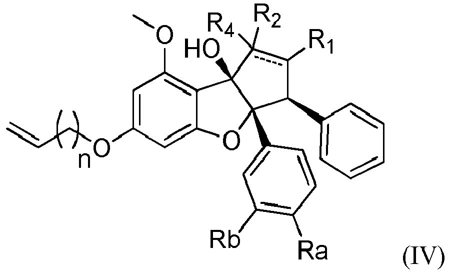 pour lequel Ra, Rb, R₁, R₂, R₄ et n sont tels que définis à la revendication 1,
(b2) pour obtenir un composé de formule (I) pour lequel R₃ = CHO, la coupure oxydante du groupe CHOHCH₂OH d'un composé de formule (I) pour lequel R₃ = CHOHCH₂OH éventuellement obtenu selon l'étape (a2),
(c2) pour obtenir un composé de formule (I) pour lequel R₃ = CO₂H, l'oxydation de la fonction aldéhyde d'un composé de formule (I) pour lequel R₃ = CHO éventuellement obtenu selon l'étape (b2),
(d2) pour obtenir un composé de formule (I) pour lequel R₃ = CO₂R₂₈ et R₂₈ ≠ H, la substitution de la fonction acide carboxylique d'un composé de formule (I) pour lequel R₃ = CO₂H éventuellement obtenu selon l'étape (c2),
(e2) pour obtenir un composé de formule (I) pour lequel R₃ = CONR₂₉R₃₀, la réaction d'un composé de formule (I) pour lequel R₃ = CO₂R₂₈, éventuellement obtenu selon l'étape (b2) ou (c2), avec une amine de formule HNR₂₉R₃₀,
(f2) pour obtenir un composé de formule (I) pour lequel R₃ = OH, la réduction de la fonction aldéhyde d'un composé de formule (I) pour lequel R₃ = CHO éventuellement obtenu selon l'étape (b2),
(g2) pour obtenir un composé de formule (I) pour lequel R₃ = OR₂₅ ou (O(CH₂)ₘO(CH₂)ₚ)ᵣ(CH₂)_{q}R₃₁ avec R₂₅ ≠ H, la substitution de la fonction hydroxyle d'un composé de formule (I) pour lequel R₃ = OH éventuellement obtenu selon l'étape (f2),
(h2) pour obtenir un composé de formule (I) pour lequel R₃ = NR₂₆R₂₇, l'amination réductrice de la fonction aldéhyde d'un composé de formule (I) pour lequel R₃ = CHO, éventuellement obtenu selon l'étape (b2), en présence d'une amine de formule HNR₂₆R₂₇.

15. Procédé de préparation d'un composé de formule (I) selon la revendication 5, pour lequel R₁ représente 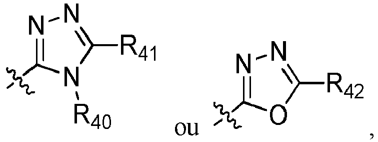 comprenant la réaction d'un composé de formule (V) suivante : 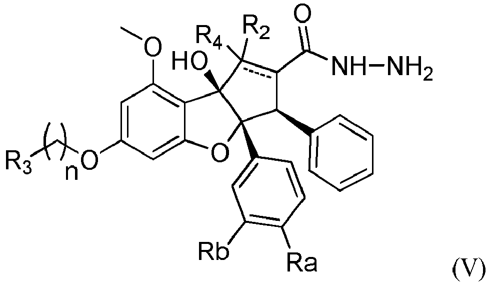 pour lequel Ra, Rb, R₂, R₃, R₄ et n sont tels que définis à la revendication 1, avec :
(1) un cyanogène de formule Hal-CN, pour lequel Hal représente un atome d'halogène, ou un isothiocyanate de formule 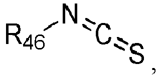 suivie éventuellement d'une ou plusieurs étapes de substitution pour donner un composé de formule (I) pour lequel R₁ représente 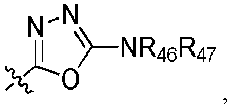 ou
(2) du sulfure de carbone (CS₂) en présence d'une base, suivie éventuellement d'une ou plusieurs étapes de substitution pour donner un composé de formule (I) pour lequel R₁ représente 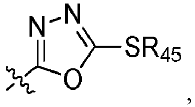 ou
(3) du carbonyldiimidazole en présence d'une base, suivie éventuellement d'une ou plusieurs étapes de substitution pour donner un composé de formule (I) pour lequel R₁ représente 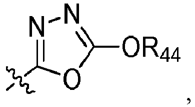 ou
(4) un acide carboxylique de formule R₄₂COOH, pour lequel R₄₂ représente un groupe (C₁-C₆)alkyle, aryle ou aryl-(C₁-C₆)alkyle, en présence de POCl₃ pour donner un composé de formule (I) pour lequel R₁ représente 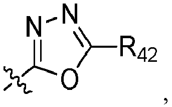 ou
(5) une isothiourée de formule 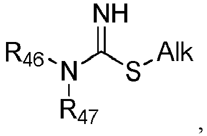 pour laquelle Alk représente un groupe (C₁-C₆)alkyle, en présence d'une base, suivie éventuellement d'une ou plusieurs étapes de substitution pour donner un composé de formule (I) pour lequel R₁ représente 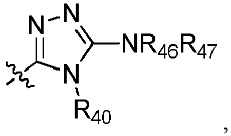 ou
(6) un imidate de formule 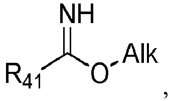 pour lequel R₄₁ représente un groupe (C₁-C₆)alkyle, aryle ou aryl-(C₁-C₆)alkyle et Alk représente un groupe (C₁-C₆)alkyle, en présence d'une base, suivie éventuellement d'une ou plusieurs étapes de substitution pour donner un composé de formule (I) pour lequel R₁ représente 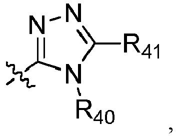 ou
(7) du triéthyl orthoformate suivie éventuellement d'une ou plusieurs étapes de substitution pour donner un composé de formule (I) pour lequel R₁ représente

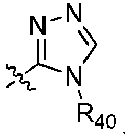

16. Procédé de préparation d'un composé de formule (I) selon la revendication 5, pour lequel R₁ représente 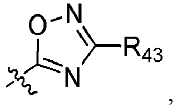 comprenant la réaction d'un composé de formule (I) avec R₁ = CO₂R₁₀ et R₁₀ représente un groupe (C₁-C₆)alkyle, avec un hydroxy-imidamide de formule 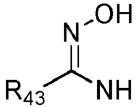 en présence d'une base.

17. Procédé de préparation d'un composé de formule (I) selon la revendication 6, pour lequel 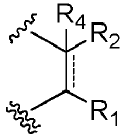 représente 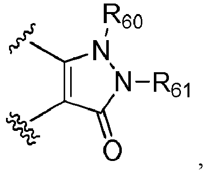 comprenant la réaction d'un composé de formule (I), pour lequel 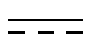 représente une liaison double, R₁ = CO₂R₁₀, R₂ = OH, R₄ = H et R₁₀ = (C₁-C₆)alkyle, avec une hydrazine de formule H₂N-NH₂, suivie éventuellement d'une ou plusieurs étapes de substitution.

18. Procédé de préparation d'un composé de formule (I) selon la revendication 6, pour lequel 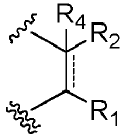 représente 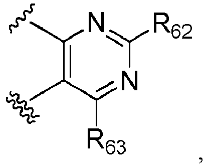 comprenant la réaction d'un composé de formule (VI) suivante : 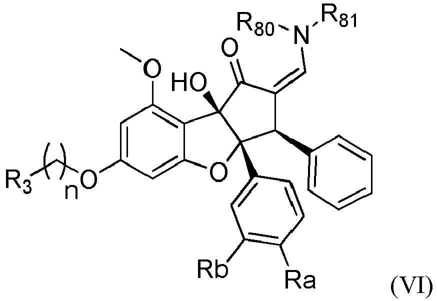 pour lequel Ra, Rb, R₃ et n sont tels que définis à la revendication 1, et R₈₀ et R₈₁ représentent, indépendamment l'un de l'autre, un groupe (C₁-C₆)alkyle, avec un dérivé de guanidine de formule

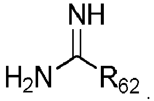

19. Procédé de préparation d'un composé de formule (I) selon la revendication 6, pour lequel 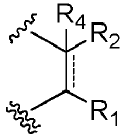 représente 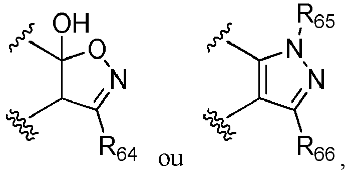 comprenant la réaction d'un composé de formule (VII) suivante : 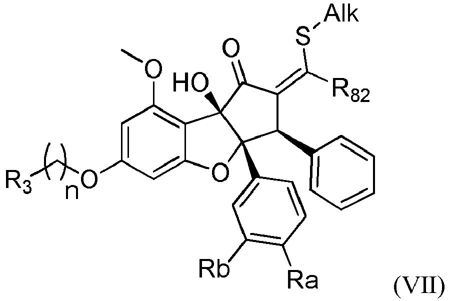 pour lequel Ra, Rb, R₃ et n sont tels que définis à la revendication 1, Alk représente un groupe (C₁-C₆)alkyle, et R₈₂ représente R₆₄ ou R₆₆, avec :
(1) l'hydroxylamine de formule HO-NH₂ en présence d'une base, pour donner un composé de formule (I) pour lequel 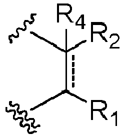 représente 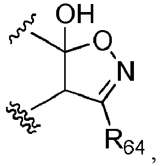 ou
(2) une hydrazine de formule H₂N-NH₂, suivie éventuellement d'une ou plusieurs étapes de substitution pour donner un composé de formule (I) pour lequel 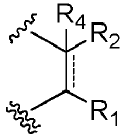 représente

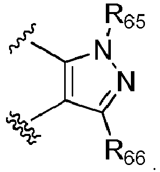

20. Procédé de préparation d'un composé de formule (I) selon la revendication 6, pour lequel 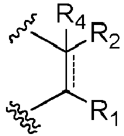 représente 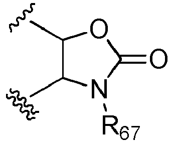 ou 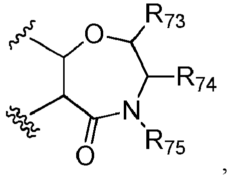 comprenant la réaction d'un composé de formule (VIII) suivante : 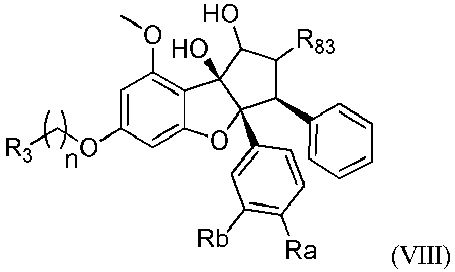 pour lequel Ra, Rb, R₃ et n sont tels que définis à la revendication 1, et R₈₃ représente un groupe CO₂H éventuellement sous une forme activée, avec :
(1) un azoture, dans des conditions de réarrangement de Curtius, suivie éventuellement d'une ou plusieurs étapes de substitution, pour donner un composé de formule (I) pour lequel 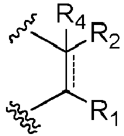 représente 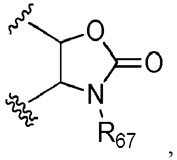 ou
(2) une amine de formule 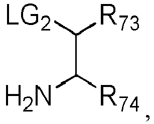 pour laquelle LG₂ représente un groupe partant tel qu'un halogène, en présence d'une base, suivie éventuellement d'une ou plusieurs étapes de substitution, pour donner un composé de formule (I) pour lequel 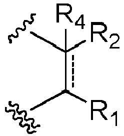 représente

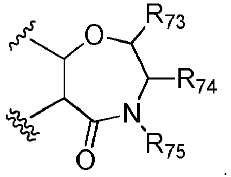

21. Procédé de préparation d'un composé de formule (I) selon la revendication 6, pour lequel 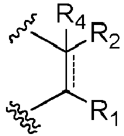 représente 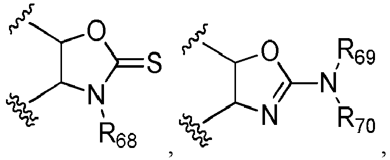 ou 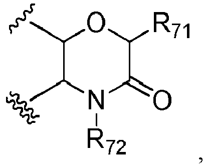 comprenant la réaction d'un composé de formule (I), pour lequel 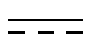 représente une liaison simple, R₁ = NH₂, R₂ = OH et R₄ = H, avec :
(1) du sulfure de carbone (CS₂) en présence d'une base, suivie éventuellement d'une ou plusieurs étapes de substitution, pour donner un composé de formule (I) pour lequel 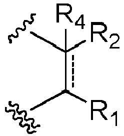 représente 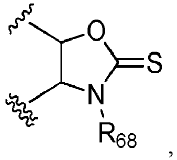 ou
(2) un cyanogène de formule Hal-CN, pour lequel Hal représente un atome d'halogène, en présence d'une base, suivie éventuellement d'une ou plusieurs étapes de substitution, pour donner un composé de formule (I) pour lequel 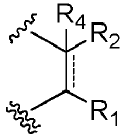 représente 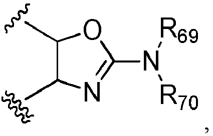 ou
(3) un ester de formule 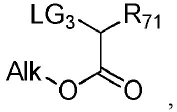 pour lequel Alk représente un groupe (C₁-C₆)alkyle et LG₃ représente un groupe partant tel qu'un atome d'halogène, en présence d'une base, suivie éventuellement d'une ou plusieurs étapes de substitution, pour donner un composé de formule (I) pour lequel 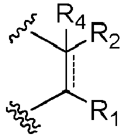 représente
